(19) Europäisches Patentamt
European Patent Office
Office européen des brevets

(11) **EP 4 183 802 A1**

(12) **EUROPEAN PATENT APPLICATION**
published in accordance with Art. 153(4) EPC

(43) Date of publication:
**24.05.2023 Bulletin 2023/21**

(21) Application number: **21827966.9**

(22) Date of filing: **21.06.2021**

(51) International Patent Classification (IPC):
*C07K 16/22* (2006.01)   *C07K 16/28* (2006.01)
*C12N 15/13* (2006.01)   *C12N 15/85* (2006.01)

(52) Cooperative Patent Classification (CPC):
**C07K 16/00; C07K 16/22; C07K 16/28; C12N 15/85**

(86) International application number:
**PCT/CN2021/101243**

(87) International publication number:
**WO 2021/259200 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 22.06.2020   CN 202010573625
09.12.2020   CN 202011428549

(71) Applicant: **Innovent Biologics (Suzhou) Co., Ltd.
Suzhou, Jiangsu 215123 (CN)**

(72) Inventors:
• **LI, Yiming
Suzhou, Jiangsu 215123 (CN)**

• **LI, Li
Suzhou, Jiangsu 215123 (CN)**
• **FU, Fenggen
Suzhou, Jiangsu 215123 (CN)**
• **JING, Hua
Suzhou, Jiangsu 215123 (CN)**
• **LIU, Junjian
Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **ANTI-ANG-2 ANTIBODY AND USE THEREOF**

(57)   The present invention relates to novel antibodies and antibody fragments specifically binding to ANG-2 and compositions comprising said antibodies or antibody fragments thereof. In addition, the present invention relates to nucleic acids encoding the antibody or antibody fragments thereof and host cells containing the nucleic acids, and their related uses. Furthermore, the present invention relates to the therapeutic and diagnostic use of these antibodies and antibody fragments.

EP 4 183 802 A1

**Description**

FIELD OF THE INVENTION

The present invention relates to novel antibodies and antibody fragments thereof, which specifically bind to ANG-2, and to compositions comprising the antibodies or antibody fragments thereof. In addition, the present invention relates to nucleic acids encoding the antibodies or antibody fragments thereof and host cells containing the nucleic acids, and their uses. Furthermore, the present invention relates to therapeutic and diagnostic uses of the antibodies and antibody fragments.

BACKGROUND OF THE INVENTION

Angiopoietin-2, also known as ANGPT2 or Ang2, is a member of the angiopoietin family. This family consists of four members, including Ang1, Ang2, and Ang3/4. Angiopoietins Ang1 and Ang2, as the ligands of Tie2 receptor (tyrosine kinase with Ig and EGF homology domains, also known as TEK), competitively bind to Tie2 expressed on the surface of vascular endothelial cells and are involved in a variety of blood vessel-related physiological and pathological processes.

Under physiological conditions, Ang1 is expressed by vascular pericytes and its binding to Tie2 causes Tie2 phosphorylation and signaling pathway activation. Thus, it plays a role in maintaining the structural and functional integrity of mature blood vessels. Ang-2, a natural antagonist of Ang-1, is believed to play a role in destabilizing blood vessels by antagonizing the ANG-1/Tie2 signaling pathway. Peters S. et al. showed that, the permeability of endothelial cells *in vitro* was increased when ang-2 or both ang-2 and VEGF were added to the culture medium containing endothelial cells (Peters S. et al., Angiopoietin-2 multiplies the permeability enhancing effect of vascular endothelial growth factor on retinal endothelial cells. Invest Ophthalmol Vis Sci. 2004, 45: 1901-1906). In addition, Laura Hakanpaa et al. (Endothelial destabilization by angiopoietin-2 via integrin β 1 activation, Nature Communications, 6: 5962 (2015)) proposed that Ang2 may also contribute to endothelial destabilization by binding to integrins in a Tie2-independent manner.

Several diseases have been known to be associated with vascular permeability and angiogenesis, including inflammation, pneumonia, mycoplasma pneumonia infection, sepsis, solid tumors, and ocular fundus diseases.

Age-related macular degeneration (AMD) and diabetic retinopathy (DR), which are caused by abnormal angiogenesis, may lead to irreversible blindness. Preclinical studies using an acute ocular inflammation model and a choroidal neovascularization model (CNV model) have shown that ang-2 and VEGF are synergistically involved in the pathological neovascularization and vascular permeability. Therefore, in addition to anti-VEGF therapy, the Tie2/angiopoietin signaling pathway has also been proposed as a therapeutic target for vascular-related retinopathies such as neovascular age-related macular degeneration (nAMD) and diabetic macular edema (DME). As an example, Nesvacumab (Regeneron, Tarrytown, NY, USA), an IgG1-type monoclonal antibody that selectively binds to ANG2 with high affinity and specifically blocks ANG2 binding to Tie2 receptor, was proposed for the treatment of nAMD and DME in combination with aflibercept (anti-VEGF-A). In addition, Faricimab, a bispecific antibody that inhibits both VEGF-A and ANG2 (developed by Roche, Basel, Switzerland and Genentech, South San Francisco, CA, USA), was proposed for the treatment of nAMD and DME. However, the currently available therapies still have limitations in terms of efficacy and treatment burden. See, Rehan M. Hussain et al., Tie-2/Angiopoietin pathway modulation as a therapeutic strategy for retinal disease, Expert Opinion on Investigational Drugs, 2019, https://doi.org/10.1080/13543784.2019.1667333.

Pulmonary inflammatory conditions, including acute bacterial or viral pneumonia and acute respiratory distress syndrome (ARDS) and acute lung injury, are characterized by leukocyte accumulation and increased microvascular permeability. ARDS has attracted much attention due to its association with severe lung injury and high mortality. ARDS manifests severe hypoxemia, decreased lung compliance, diffuse alveolar damage, and bilateral lung infiltration. ARDS can be caused by direct or indirect lung injury, in which the inflammation in the lung induces the damage to alveolar-capillary barrier, which is one of the significant and direct factors that contributes to ARDS. The current main treatment modality for ARDS patients is mechanical ventilation. In addition, a number of non-mechanical ventilation treatment modalities have been proposed, such as lung water removal and fluid management, surfactant replenishment, beta-receptor agonists, statins, and glucocorticoids, but for most of them, reliable efficacy has not been demonstrated. In addition, although the efficacy of the hormone drug dexamethasone in the treatment of inflammation-mediated lung injury (such as SARS and COVID-19 virus-induced pneumonia) has been clinically recognized (DOI: 10.1056/NEJMoa2021436), the drug has enormous side effects, and the profound impacts of the drug on the subsequent life of patients have been increasingly reported.

Given the role of ANG-2 in vascular permeability and angiogenesis, there is a need in the field for continuous development of new drug molecules that are capable of highly specifically and selectively targeting and regulating the biological activity of ANG-2, such as anti-ANG-2 antibodies and combination therapies thereof, for the treatment of vascular-related diseases.

SUMMARY OF THE INVENTION

The present invention provides anti-ANG-2 antibodies, their encoding genes, and uses thereof. Based on hybridoma screening, chimeric antibody construction, humanization and affinity maturation, the inventors have developed anti-human ANG-2 antibodies of the invention with high affinity and specificity. The antibodies of the invention can effectively block the ANG-2/receptor TIE2 signaling pathway and the ANG-2/integrin signaling pathway, and work synergistically with anti-VEGF agents to reduce vascular leakage, inhibit neovascularization and improve vascular integrity in an animal model of choroidal neovascularization. In addition, the antibodies of the invention exhibit low non-specific binding to a variety of retinal-derived cells, thereby facilitating its intravitreous administration at high concentrations for the treatment of ocular fundus diseases.

Meanwhile, the inventors have also found that the anti-ANG2 antibodies of the invention can improve vascular permeability, vascular leakage and vascular inflammatory conditions, and ameliorate various parameters in pulmonary inflammatory conditions, especially ARDS, such as pulmonary infiltration of inflammatory cells, blood oxygen saturation, pulmonary edema degree, lung weight index, and animal survival rate. Moreover, the treatment methods with the anti-ANG2 antibdoies of the invention are hormone-independent and can achieve an efficacy that is not inferior to, and in some parameters even superior to, that of the hormone dexamethasone.

Thus, in one aspect, the present invention provides novel anti-Ang-2 antibodies, and antigen-binding fragments thereof.

In some embodiments, the anti-ANG-2 antibodies of the invention exhibit one or more of the following properties:

(i) binding with high affinity to human ANG-2, but not to human ANG-1;

(ii) having immunological cross-reactivity with monkey ANG-2;

(iii) effectively binding to ANG-2 on a cell surface;

(iv) blocking ANG-2 binding to receptor TIE2;

(v) blocking ANG-2 binding to an integrin;

(vi) working synergistically with an anti-VEGF agent to reduce vascular leakage, inhibit neovascularization, and improve vascular integrity in a vascular-related disease, such as choroidal neovascularization;

(viii) having a low non-specific binding activity to cells not expressing Ang2.

In some embodiments, the present invention provides an antibody or antigen-binding fragment thereof that binds to ANG-2, comprising the HCDR1, HCDR2 and HCDR3 sequences of one of the heavy chain variable regions shown in SEQ ID NOs: 19, 21, 23, 25, 27, and 29, and/or the LCDR1, LCDR2 and LCDR3 sequences of one of the light chain variable regions shown in SEQ ID NOs: 20, 22, 24, 26, 28, and 30, or a variant of the CDR sequence combination.

In some embodiments, the present invention also provides an anti-ANG-2 antibody, or antigen-binding fragment thereof that binds to the epitope same as or overlapping with that bound by, and/or competes for binding to ANG-2 with, and/or inhibits (e.g., competitively inhibits) an exemplary antibody of the present invention, such as an antibody having a combination of VH and VL sequences as shown in Table B.

In some embodiments, the present invention provides a nucleic acid encoding an antibody or antigen-binding fragment of the invention, a vector comprising the nucleic acid and a host cell comprising the vector.

In some embodiments, the present invention provides a method of preparing an antibody or antigen-binding fragment of the invention.

In some embodiments, the present invention provides an immunoconjugate, a multispecific antibody, a pharmaceutical composition, and a combination product comprising an antibody of the invention.

In some embodiments, the present invention is also directed to a method of detecting a human and/or cynomolgus monkey ANG-2 in a sample.

In another aspect, the present invention also provides a method of blocking an ANG-2-mediated biological activity in a subject by using an antibody of the invention.

In some embodiments, the present invention provides a method of preventing or treating a vascular-related disease, such as ocular neovascularization diseases, pulmonary inflammation, and various solid tumors, by using an antibody of the invention.

In some embodiments, the present invention provides a use of an anti-ANG2 antibody of the invention in treatment of a disease or a disorder associated with vascular leakage and/or vascular inflammatory condition. In the use context, the diseases or disorders treatable by the methods of the invention include any diseases or disorders that may benefit from the blocking (i.e., ameliorating, inhibiting, relieving, or alleviating) of increased vascular endothelial permeability mediated by ANG2 and/or vascular inflammatory conditions associated with an ANG2 activity, such as any diseases or disorders accompanied with vascular leakage and/or vascular inflammation. Such diseases or disorders include, but are not limited to, diabetic macular edema, retinal vein occlusion, wet age-related macular degeneration (wet AMD), diabetic retinopathy, cancer, sepsis, capillary leakage syndrome, pulmonary inflammatory condition, angioedema and vascular leakage syndrome. In a preferable embodiment, the disease or disorder is a pulmonary inflammatory condition, such as pulmonary inflammation caused by infection with, for example, a virus or bacterial, e.g., viral pneumonia, and acute lung injury or acute respiratory distress syndrome caused, for example, by pulmonary inflammation. In yet another preferable embodiment, the disease or disorder is acute lung injury (ALI). In yet another preferable embodiment, the disease or condition is acute respiratory distress syndrome.

In one embodiment, the present invention provides a method for reducing vascular permeability, comprising administering to a subject in need thereof a therapeutically effective amount of an anti-ANG2 antibody or antigen-binding fragment thereof of the invention or a pharmaceutical composition comprising the same.

In one embodiment, the present invention provides a method of reducing vascular leakage and/or ameliorating a vascular inflammatory condition comprising administering to a subject in need thereof a therapeutically effective amount of an anti-ANG2 antibody or antigen-binding fragment thereof of the invention or a pharmaceutical composition comprising the same.

In another embodiment, the present invention provided a method for preventing and/or treating a disease or disorder accompanied with vascular leakage, comprising administering to a subject in need thereof a therapeutically effective amount of an anti-ANG2 antibody or antigen-binding fragment thereof of the invention or a pharmaceutical composition comprising the same.

In another embodiment, the present invention provides a method for preventing and/or treating a disease or disorder accompanied with vascular inflammation, comprising administering to a subject in need thereof a therapeutically effective amount of an anti-ANG2 antibody or antigen-binding fragment thereof of the invention or a pharmaceutical composition comprising the same..

In another embodiment, the present invention provides a method for preventing and/or treating a pulmonary inflammatory condition, especially acute respiratory distress syndrome and acute lung injury, comprising administering to a subject in need thereof a therapeutically effective amount of an anti-ANG2 antibody or antigen-binding fragment thereof of the invention or a pharmaceutical composition comprising the same.

In yet another embodiment, the present invention relates to a method of ameliorating one or more of the following disease parameters of acute respiratory distress syndrome: infiltration intensity of inflammatory cells in lung tissues, blood oxygen saturation, pulmonary edema degree, lung weight index, intensity of inflammatory lesions in lung tissues, lung CT images, and survival rate, comprising administering to a subject in need thereof a therapeutically effective amount of an anti-ANG2 antibody or antigen-binding fragment thereof of the invention or a pharmaceutical composition comprising the same.

In some embodiments, acute respiratory distress syndrome is caused by pulmonary inflammatory injury, e.g., bacterial or viral infection or an inflammatory agent such as bacterial endotoxin LPS. In a preferable embodiment, the method is a prophylactic for an individual at risk of developing ARDS. In yet another embodiment, the method is a therapeutic for an individual who has been diagnosed or suspected of having ARDS.

In the embodiments of the present invention, preferably, the anti-ANG2 antibody of the invention blocks the binding of human ANG-2 to human TIE2 expressed on the surface of vascular endothelial cells and/or blocks the binding of human ANG-2 to an integrin. Preferably, the anti-ANG2 antibody of the invention inhibits intracellular Tie2 phosphorylation induced by human ANG-2.

In the embodiments of the present invention, preferably, the anti-ANG2 antibody of the invention comprises an HCDR1 sequence of SEQ ID NO: 1, an HCDR2 sequence of SEQ ID NO: 2, an HCDR3 sequence of SEQ ID NO: 3, an LCDR1 sequence of SEQ ID NO: 4, an LCDR2 sequence of SEQ ID NO: 5, and an LCDR3 sequence of SEQ ID NO: 6. More preferably, the ANG2 antibody of the invention comprises a heavy chain variable region VH sequence of SEQ ID NO: 25 and a light chain variable region VL sequence of SEQ ID NO: 26.

In some embodiments, the anti-ANG2 antibody of the invention is administered as a monotherapy agent. In some embodiments, the anti-ANG2 antibody of the invention is administered in combination with a second therapeutic agent. In yet some other embodiments, the second therapeutic agent is an anti-VEGF inhibitor molecule. In yet other embodiments, the anti-ANG2 antibody of the invention is administered independently of (i.e., not in combination with) a VEGF pathway-blocking agent, such as an anti-VEGF inhibitor.

The present invention will be further illustrated with reference to the following figures and specific embodiments. However, these figures and specific embodiments shall not be construed as limiting the scope of the present invention, and the modifications that are readily conceivable by those skilled in the art will be included within the spirit of the present invention and the scope of protection of the appended claims.

BRIEF DESCRIPTION OF THE FIGURES

Figures 1A-1B show the binding activities of antibodies to hANG2 protein, as measured by ELISA.

Figures 2A-2F show the blocking effects of antibodies on Ang2/Tie2 binding, as measured by ELISA.

Figure 3 shows the blocking effects of antibodies on Ang2-Fc/Tie protein binding, as measured in a cell-based assay.

Figure 4 shows the inhibitory effects of antibodies on Ang2-Fc-induced Tie2 phosphorylation, as measured in a cell-based assay.

Figures 5A-5B show the inhibitory effects of antibodies on Ang2/integrin-mediated cell adhesion, as measured in a cell-based assay.

Figure 6 shows the results from detection of non-specific cell binding activities of anti-ANG2 antibody hz81H10.4. The binding activities of the antibody of the invention, which was expressed in CHO cells, were detected and compared on a variety of cells of different origins that express and do not express ANG2 via flow cytometry assay. An IgG isotype control antibody was used as a negative control, and Nesvacumab expressed in CHO cells as a positive control. The binding activities of anti-VEGF inhibitor molecule (IBI304) to different cells are also shown in this figure.

Figure 7 shows the effects of anti-ANG2 antibody hz81H10.4 on the expressions of inflammatory marker molecules on endothelial cells. Figure 7A shows that ANG2 sensitized TNF$\alpha$-induced CD54 and CD106 expressions on HUVEC-Tie2 cells in the presence of ANG1 (*indicates $p < 0.05$; **indicates $p < 0.01$). Figure 7B shows and compares the inhibitory effects of the antibody of the invention and the positive control antibody on the ANG2-sensitized expressions of CD54 and CD 106 on endothelial cells.

Figure 8 shows the inhibitory effect of anti-ANG2 antibody hz81H10.4 on vascular leakage in a HUVEC-Tie2 endothelial cell-based assay.

Figure 9 shows the results from white blood cell count and differential cell count in bronchoalveolar lavage fluid after administration of antibody hz81H10.4 in ARDS model animals induced by low dose of LPS.

Figure 10 shows the effect of administration of antibody hz81H10.4 on lung weight index, as compared to administration of sodium chloride solution, in ARDS model animals induced by different doses of LPS.

Figure 11 shows the effect of administration of antibody hz81H10.4 on animal survival rate, as compared to administration of sodium chloride solution, in ARDS model animals induced by different doses of LPS.

Figure 12 shows the ameliorative effect of anti-ANG2 antibody administration on lung pathology in LPS-induced ARDS model animals. Each column shows pathological sections of lungs from antibody-treated and control sodium chloride-treated animals with ARDS induced by low, medium, and high LPS doses, respectively.

Figure 13 shows the results from white blood cell count and differential cell count in bronchoalveolar lavage fluid after administration of different doses of antibody hz81H10.4 in ARDS model animals induced by LPS. *indicates $p < 0.05$; **indicates $p < 0.01$; *** indicates $p < 0.001$; and ns indicates no statistical significance, as compared to the control group.

Figure 14 shows and compares the degrees of improvement in blood oxygen saturation, which were induced by different concentrations of antibody hz81H10.4 and dexamethasone, in ARDS model animals. **indicates $p < 0.01$; ***indicates $p < 0.001$, as compared to the control group.

Figure 15 shows and compares the improvements in lung pathology in the ARDS model by administration of different concentrations of hz81H10.4 antibody and by administration of dexamethasone. Each panel shows the results of pathological sections of lungs from control ARDS animals treated with sodium chloride solution, ARDS animals treated with dexamethasone at 2 mg/kg, and ARDS animals treated with antibody hz81H10.4 at 5 mg/kg, 15 mg/kg, and 25 mg/kg, respectively, on post-treatment day D7.

Figure 16 shows the effects of different concentrations of hz81H10.4 antibody and dexamethasone on the intensity of inflammatory lesions in lung tissues of ARDS model animals.

Figure 17 shows the degrees of improvement in lung CT images of ARDS model animalstreated with different concentrations of hz81H10.4 antibody and dexamethasone.

Figure 18 shows the angiographic imaging results from a laser-induced choroidal neovascularization model after different treatments (control, anti-VEGF molecule IBI304 alone, anti-VEGF molecule IBI304 in combination with an antibody of the present invention).

Figure 19 shows the decrease in the area of vascular leakage caused by different treatments (control, anti-VEGF molecule IBI304 alone, anti-VEGF molecule IBI304 in combination with an antibody of the present invention) in a laser-induced choroidal neovascularization model.

Figure 20 shows the degrees of vascular leakage at the laser-injured sites, as scored at different post-dose times, in a laser-induced choroidal neovascularization model.

Figure 21 shows the morphological changes, and compares the sizes of injured and swollen areas, as observed in the tissues at the laser-injured sites after different treatments by conventional HE staining histopathological examination .

Figure 22 shows the results from CD31-immunohistochemical stained sections of retina and choroid.

Figure 23 shows the proportion of CD31-positive cells, as counted by CD31-staining of retinal and choroidal sections.

Figure 24 shows the ratios of Desmin-positive cells/CD31-positive cells , as counted by double-fluorescence staining of retinal and choroidal sections for CD31 and Desmin.

Figure 25 shows the CDR sequences of the exemplary antibodies of the present invention.

Figure 26 shows the VH and VL sequences of the exemplary antibodies of the present invention.

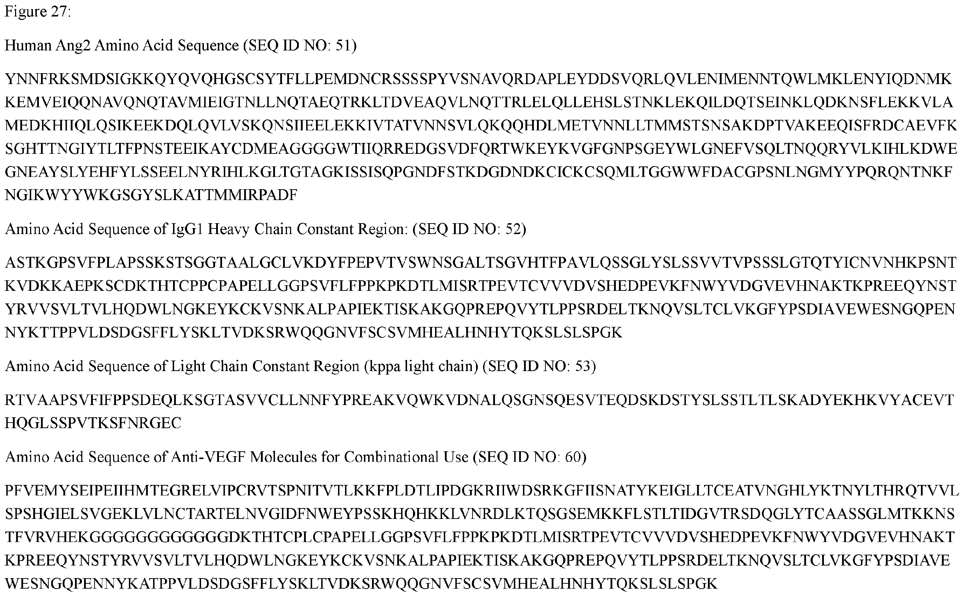

Figure 27 shows an exemplary amino acid sequence of human ANG2, as well as the amino acid sequences of the heavy chain constant region and the light chain constant region used to construct the chimeric and humanized antibodies of the present invention, as well as the amino acid sequence of an exemplary anti-VEGF molecule for use in combination with the antibodies of the present invention.

DETAILED DESCRIPTION OF THE INVENTION

Definition

Unless otherwise defined, all technical and scientific terms used herein have the same meaning as is generally understood by a person of ordinary skill in the art. For the purposes of the present invention, the following terms are defined as follows.

The term "about", when used in conjunction with a numeric value, is intended to encompass the numeric values falling within the range having a lower limit of 5% less than the specified numeric value and an upper limit of 5% greater than the specified numeric value.

The term "and/or", used in conjunction with options, shall be construed to mean any one of the options or a combination of any two or more of the options.

As used herein, the term "comprise" or "include" means the inclusion of a stated element(s), integer(s), or step(s), but not the exclusion of any other element(s), integer(s), or step(s). In the present disclosure, the term "comprise" or "include" encompasses the situations consisting of the stated element(s), integer(s) or step(s) unless otherwise indicated. For example, when reference is made to a variable region of an antibody that "comprises" a particular sequence, it is also intended to encompass a variable region of an antibody that consists of that particular sequence.

As used herein, the term "antibody" refers to a polypeptide comprising at least a light or heavy chain immunoglobulin variable region that specifically recognizes and binds to an antigen. This term covers a variety of antibody structures, including, but not limited to, monoclonal antibodies, polyclonal antibodies, single- or multiple-chain antibodies, monospecific or multispecific antibodies (e.g., bispecific antibodies), chimeric or humanized antibodies, full-length antibodies, and antibody fragments, as long as they exhibit the desired antigen-binding activities.

It will be understood by those skilled in the art that a "whole antibody" (used interchangeably herein with "full-length antibody", "complete antibody" and "intact antibody") comprise at least two heavy chains (H) and two light chains (L). Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain, CL. Variable regions are domains in the heavy or light chains of antibodies that are involved in the binding of the antibodies to their antigen. Constant regions are not directly involved in antibody-antigen binding, but they display a variety of effector functions. The light chains of antibodies can be assigned to one of two types, known as kappa ($\kappa$) and lambda ($\kappa$), based on the amino acid sequences of light chain constant domains. The heavy chains of antibodies can be divided into five major types, IgA, IgD, IgE, IgG, and IgM, depending on the amino acid sequences of heavy chain constant regions, , and several of these types can be further divided into subtypes, such as IgG1, IgG2, IgG3, and IgG4, IgA1, and IgA2. The heavy chain constant regions corresponding to different antibody types are called $\alpha$, $\delta$, $\epsilon$, $\gamma$, and $\mu$, respectively. The term "isotype" refers to the type of an antibody that is determined by the heavy chain constant region of the antibody. See generally, Fundamental Immunology, Ch.7 (Paul, W. Ed., 2nd ed., Raven Press, N.Y. (1989)) (which is incorporated herein by reference in its entirety and for all purposes). In a preferable embodiment, a full-length antibody comprising two heavy chains and two light chains, especially an antibody of IgG type, is used in the methods of the present invention.

The term "antigen-binding portion" of an antibody (used interchangeably herein with "antibody fragment" and "antigen-binding fragment") refers to a molecule other than an intact antibody that comprises a portion of an intact antibody that binds to the antigen to which the intact antibody binds. As understood by those skilled in the art, the antigen-binding portion of an antibody typically comprises amino acid residues from a "complementary determining region" or a "CDR". An antigen-binding fragment can be prepared using recombinant DNA technology, or by enzymatic or chemical cleavage of an intact antibody. Antigen-binding fragments include, but are not limited to, Fab, scFab, Fab', F (ab')$_2$, Fab'-SH, Fv, single-chain Fv, diabody, triabody, tetrabody, minibody, and single-domain antibody (sdAb). More detailed description of antibody fragments can be found in Fundamental Immunology, edited by W. E. Paul, Raven Press, N.Y. (1993); Shao Rongguang, et al. (Editor), Research and Application of Antibody-based Drugs. People's Medical Publishing House (2013); Hollinger et al., PNAS USA 90:6444-6448(1993); and Hudson et al., Nat. Med. 9: 129-134 (2003).

In some embodiments, antibodies or antigen-binding fragments of the present invention may comprise the following configurations of variable and constant regions: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL.

The term "chimeric antibody" refers to an antibody in which the variable region sequence is derived from one species and the constant region sequence is derived from another species, e.g., an antibody in which the variable region sequence is derived from a mouse antibody and the constant region sequence from a human antibody.

The term "humanized antibody" refers to an antibody in which CDR sequences derived from another mammalian species (such as mice) are grafted into the framework region sequences of human. Additional modifications to framework regions can be made within the human framework region sequences.

An "isolated" antibody is one that has been separated from a component of its natural environment. In some embodiments, the antibody is purified to greater than 95% or 99% purity, as determined by, for example, electrophoresis (e.g., SDS-PAGE, isoelectric focusing (IEF), capillary electrophoresis) or chromatography (e.g., ion exchange or reverse phase HPLC). For a review of methods for assessing antibody purity see, e.g., Flatman, S. et al., J. Chrom. B 848 (2007) 79-87.

The term "epitope" refers to a region in an antigen to which an antibody binds. Epitopes can be formed by contiguous amino acids or non-contiguous amino acids juxtaposed by tertiary folding of a protein.

The terms "ANG-2" and "ANG2" are used interchangeably herein and refer to angiopoietin 2. The expressions include variants, isoforms, homologs, and species homologs of ANG-2. The term "human ANG-2" refers to ANG-2 of a human sequence. A specific human ANG-2 sequence is shown in SEQ ID NO: 51. In some embodiments, a human ANG-2 sequence has at least 95%, or even 96%, 97%, 98%, or 99% amino acid sequence identity to the human ANG-2 amino acid sequence as shown in SEQ ID NO: 51. Amino acid sequences of ANG-2 proteins of other species, such as mouse and monkey ANG-2 sequences, are available from the NCBI protein sequence database, e.g. under accession numbers NP_031452 and BAE89705.1. In the present disclosure, "ANG-2" encompasses fragments of ANG-2, such as fragments capable of exerting a biological function of a full-length ANG-2 molecule, e.g., fragments comprising a C-terminal fibrinogen-like domain (FLD). In the present disclosure, the term ANG2, if not explicitly indicated as from a non-human species (e.g., "murine ANG2" or "monkey ANG2"), refers to ANG2 from human, or a biologically active fragment thereof.

As used herein, the term "ANG1" or "ANG-1" refers to angiopoietin 1. Previous studies have shown that Ang1 has beneficial effects in maintaining vascular homeostasis and preventing adult vascular leakage. In the present disclosure, the antibodies of the present invention bind to ANG2, but not to ANG1, although ANG2 and ANG1 share a considerable amino acid sequence homology and competitively bind to the same site of the TIE2 receptor. An amino acid sequence of human ANG1 is available from the NCBI Protein Sequence Database under accession number AAB50557. In the present disclosure, the term ANG1, if not explicitly indicated as from a non-human species (e.g., "murine ANG1" or "monkey ANG1"), refers to ANG1 from human, or a biologically active fragment thereof.

As used herein, the term "Tie2" (also known as "TEK") refers to tyrosine kinase 2 with Ig and EGF homology domains. The term TIE2, if not explicitly indicated herein as from a non-human species (e.g., "murine TIE2" or "monkey TIE2"), refers to TIE2 from human or a biologically active fragment thereof. An amino acid sequence of human TIE2 is available from the NCBI Protein Sequence Database under accession number AAA61130.

The term "specifically bind" means that an antibody selectively or preferentially binds to an antigen. If an antibody binds to human ANG-2 with a $K_D$ of about $5\times 10^{-7}$ M or less, about $1\times 10^{-7}$ M or less, about $5\times 10^{-8}$ M or less, about $1\times 10^{-8}$ M or less, or about $5\times 10^{-9}$ M or less in a biological optical interferometry assay, the antibody is considered as an antibody that "specifically binds to human ANG-2". However, antibodies that specifically bind to human ANG-2 can be cross-reactive with ANG-2 proteins from other species. For example, in some embodiments, an antibody specific for human ANG-2 is cross-reactive with ANG-2 protein from cynomolgus monkeys, but not with murine ANG-2. Methods of determining cross-reactivity include the methods described herein in the Examples and the standard assays known in the art, for example, by using biological optical interferometry or flow cytometry.

The term "affinity" or "binding affinity" refers to the intrinsic binding affinity that reflects the interactions between members of a binding pair. The affinity of a molecule X for its partner Y can generally be represented by an equilibrium dissociation constant ($K_D$), which is the ratio of a dissociation rate constant to an association rate constant ($k_{dis}$ and $k_{on}$, respectively). Affinity can be measured by common methods known in the art. A specific method for measuring affinity is the ForteBio kinetic binding assay described herein.

The term "high affinity" for an IgG antibody means that the antibody binds to a target antigen with a $K_D$ of $1\times 10^{-7}$ M or less, preferably $5\times 10^{-8}$ M or less, more preferably about $1\times 10^{-8}$ M or less, and even more preferably about $5\times 10^{-9}$ M or less. However a "high affinity" for binding may vary with different antibody isotypes. For example, for an antibody of IgM isotype, "high affinity" means that the antibody has a $K_D$ of $1\times 10^{-6}$ M or less, preferably $1\times 10^{-7}$ M or less, more preferably about $1\times 10^{-8}$ M or less.

In some embodiments, antibodies of the present invention are ANG2 neutralizing antibodies or blocking antibodies that, upon binding to ANG2, block the interaction of ANG2 with its receptor (TIE2) and/or with an integrin and/or result in inhibition of at least one biological activity of ANG2. Detection of the activity of the neutralizing antibodies and the blocking antibodies can be performed using the assays known in the art or described in this disclosure, such as those exemplified in the Examples.

An " antibody that competes for binding" with a reference antibody that binds to an antigen (e.g., ANG-2), refers to an antibody that blocks the binding of the reference antibody to the antigen (e.g., ANG-2) by 50% or more in a competition assay, and conversely, the reference antibody blocks the binding of the antibody to the antigen (e.g., ANG-2) by 50% or more in a competition assay. Exemplary competition assays are described in: "Antibodies", Harlow and Lane (Cold Spring Harbor Press, Cold Spring Harbor, NY). The antibodies that compete for binding may bind to the same epitope region as the reference antibody, e.g., the same epitopes, adjacent epitopes, or overlapping epitopes.

An antibody that inhibits (e.g., competitively inhibits) the binding of a reference antibody to its antigen refers to an antibody that inhibits the binding of the reference antibody to its antigen by more than 50%, 60%, 70%, 80%, 90% or 95%. Conversely, the reference antibody also inhibits the binding of the antibody to its antigen by more than 50%, 60%, 70%, 80%, 90%, or 95%. The binding of an antibody to its antigen can be measured by affinity, such as an equilibrium dissociation constant. Methods for affinity determination are known in the art.

An antibody that exhibits the same or similar binding affinity and/or specificity as a reference antibody refers to an antibody that is capable of exhibiting at least 50%, 60%, 70%, 80%, 90%, or more than 95% of the binding affinity and/or specificity of the reference antibody. This can be determined by any method known in the art for determining the binding affinity and/or specificity.

The term "Fc region" is used herein to define a C-terminal region of an immunoglobulin heavy chain that contains at least a portion of the constant region. The term includes native sequence Fc-regions and variant Fc-regions. In an embodiment, a human IgG heavy chain Fc region extends from Cys226 or from Pro230 to the carboxy terminus of the heavy chain. However, the C-terminal lysine (Lys447) of the Fc-region may or may not be present. Unless otherwise indicated herein, the numbering of amino acid residues in the Fc-region or constant region is according to the EU numbering system, also known as the EU Index, as described in Kabat, E. A. et al., Sequences of Proteins of Immunological Interest, 5th edition, Public Health Service, National Institutes of Health, Bethesda, MD (1991), NIH Publication 91-3242.

The term "variant", with reference to an antibody, is used herein to refer to an antibody comprising an antibody region of interest that has been modified in amino acids by substitution, deletion and/or insertion of at least one amino acids, e.g., 1-30 amino acids, or 1-20 amino acids or 1-10 amino acids (e.g., 1, 2, 3, 4 or 5 amino acids), as compared to a reference antibody, wherein the variant substantially retains at least one biological property (e.g., antigen-binding capacity) of the original antibody molecule without the modification. The antibody region of interest may be the full length of the antibody, or the heavy chain variable region, the light chain variable region, or the combination thereof, or the heavy chain CDR region(s) or the light chain CDR region(s) or the combination thereof. In the present disclosure, as compared to a reference antibody region, an antibody region that has amino acid modifications is also referred to as a "variant" of that antibody region.

In the present disclosure, "sequence identity" refers to the degree to which a sequence is identical to another on a nucleotide-by-nucleotide or amino acid-by-amino acid basis in a comparison window. The "percentage of sequence identity" can be calculated using the following method: comparing two optimally aligned sequences in a comparison window to determine the number of the positions where the same nucleic acid base (e.g., A, T, C, G, I) or the same amino acid residue (e.g., Ala, Pro, Ser, Thr, Gly, Val, Leu, Ile, Phe, Tyr, Trp, Lys, Arg, His, Asp, Glu, Asn, Gln, Cys, and Met) is present in the two sequences so as to obtain the number of matching positions, then dividing the number of matching positions by the total number of the positions in the comparison window (i.e., window size), and multiplying the result by 100 to obtain the percentage of sequence identity. The optimal alignment for determining the percent sequence identity can be achieved in a variety of ways known in the art, for example, using publicly available computer software such as BLAST, BLAST-2, ALIGN or Megalign (DASTAR) software. One skilled in the art can determine suitable parameters for aligning sequences, including any algorithm required to achieve maximum alignment over the full length of the sequence being compared or within a sequence region of interest.

In the present disclosure, for antibody sequences, the percentage of amino acid sequence identity is determined by optimally aligning a candidate antibody sequence with a reference antibody sequence, and in a preferred embodiment by optimally aligning according to the Kabat numberings. In the present disclosure, if not specifying a comparison window (i.e., the antibody region of interest to be compared), the alignment is made over the full length of the reference antibody sequence . In some embodiments, sequence identity may be present along the entire heavy chain variable regions and/or the entire light chain variable regions of antibodies, or sequence percentage identity may be limited to the framework regions only, with the sequences in corresponding CDR regions remaining 100% identical.

Similarly, for antibody sequences, a candidate antibody with an amino acid modification in an antibody region of interest, as relative to a reference antibody, can be identified based on sequence alignment.

In the present disclosure, a "conservative substitution" means an amino acid modification by which an amino acid is replaced with a chemically similar amino acid. Amino acid modifications such as substitutions can be introduced into the antibodies of the present invention using standard methods known in the art, such as site-directed mutagenesis and PCR-mediated mutagenesis. A table of conservative substitutions providing functionally similar amino acids is well-known in the art. In a preferable aspect, residues for conservative substitution are from the following conservative substitution table, Table A, and preferably are the residues under preferred conservative substitutions in Table A.

Table A

| Original residue | Exemplary substitutions | Preferred conservative substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |

(continued)

| Original residue | Exemplary substitutions | Preferred conservative substitutions |
|---|---|---|
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr(T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

In some embodiments, the antibodies of the present invention used in the methods of the present invention are antibody sequences having conservative substitutions, for example, antibody sequences that are derived from an exemplary antibody sequence of the invention (e.g., any one or combination of SEQ ID Nos: 1 to 10) by introducing one or more conservative substitutions, especially preferred conservative substitutions in Table A.

The terms "individual" and "subject" are used herein interchangeably to refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the term "subject" refers to a human.

In the present disclosure, "treatment" includes 1) a therapeutic measure that cures, postpones, alleviates the symptoms of and/or halts the progression of a diagnosed pathological condition or a disorder; and 2) a prophylactic or preventive measure that prevents and/or postpones the development of a pathological condition or a disorder. Thus, subjects in need of treatment include individuals having already developed a disorder, individuals susceptible to a disorder, and individuals to be prevented from a disorder. The individuals will benefit from the therapeutic or prophylactic measure and exhibit a reduction or improvement in the occurrence, recurrence, or progression of a disease, disorder, condition, and/or symptom as compared to an individual not receiving the said treatment. In some embodiments, the present invention involves the treatment of a disease or a disorder. In other embodiments, the present invention involves the prevention of a disease or a disorder.

In some embodiments of the invention, "treatment" of a disease or a disorder means amelioration of the disease or the disorder (i.e., slowing or preventing or reducing the progression or at least one clinical symptom). In some other embodiments, "treatment" means relief or improvement of at least one physical parameter, including those physiological parameters that may be indiscernible by patients. In some other embodiments, "treatment" means modulating a disease or a disorder physically (e.g., stabilizing a discernible symptom), physiologically (e.g., stabilizing a physical parameter), or both. Unless explicitly otherwise indicated herein, methods for assessing the therapeutic and/or prophylactic efficacy of a disease or disorder are generally known in the art.

In some embodiments of the present invention, "treatment" of a disease or a disorder means "prevention" of a disease or a disorder, including inhibition of the occurrence or progression of the disease or the disorder or a particular symptom of the disease or the disorder. In some embodiments, the subject is a candidate for a prevention regimen. Typically, the term "prevention" refers to administration of a drug prior to the appearance of any signs or symptoms of a disease or a disorder, particularly in a subject at risk of developing a disease.

The terms "therapeutically effective amount", "prophylactically effective amount" or "effective amount" herein refer to an amount of an anti-ANG2 antibody or antigen-binding fragment thereof of the present invention, which, when administered to a cell, tissue or subject alone or in combination with additional therapeutic agent, is effective in preventing or ameliorating one or more symptoms of or the progression of a disease or a disorder. A therapeutically effective amount also refers to an amount of an antibody or antigen-binding fragment thereof that is sufficient to result in the improvement of a symptom, such as an amount that results in treatment, remedy, prevention, or amelioration of a relevant medical condition or an increase in the rate of treatment, remedy, prevention, or amelioration of such a condition. When an active agent is administered to an individual alone, the therapeutically effective amount refers to the amount of the agent alone. When more than one active agents are administered in combination, the therapeutically effective amount refers to the combined amount of the active agents that cause a therapeutic effect, whether the active agents are administered concomitantly, sequentially, or simultaneously. An effective amount of a therapeutic agent will result in an improvement in a diagnostic criteria or parameter of at least 10%, typically at least 20%, preferably at least about 30%, more preferably at least 40%, and most preferably at least 50%.

In the present disclosure, a VEGF blocker refers to an anti-VEGF agent that blocks a function/activity of VEGF or VEGF receptor. Anti-VEGF agents include, for example, monoclonal antibodies specifically binding to VEGF-A, which agents bind to the VEGF to block the binding of the VEGF to its receptor; and VEGFR blocking agents consisting of an extracellular fragment of a VEGF receptor and an Fc region of an immunoglobulin, which agents have an inherently high specificity for VEGF. Other examples of anti-VEGF agents include VEGF-Trap (see, e.g., US7,087,411), anti-VEGF antibodies (e.g., bevacizumab), small molecule kinase inhibitors for VEGF receptors (e.g., sunitinib). In one embodiment of the present invention, a second therapeutic agent for use in combination with an antibody of the present invention is a VEGF blocker. In yet another embodiment of the invention, an antibody of the invention is administered independently of (i.e., not in combination with) a VEGF blocker.

The following subsections will further detail various aspects of the invention.

## I. Anti-ANG-2 Antibodies of the Present Invention

In one aspect, the present invention provides antibodies or antigen-binding fragments thereof that specifically bind to ANG-2, preferably human ANG-2 protein (e.g., a human ANG-2 sequence of SEQ ID NO: 51). In some embodiments, the antigen-binding fragments of the antibodies of the present invention are an antibody fragment selected from the following antibody fragments: Fab, Fab', Fab'-SH, Fv, a single-chain antibody such as scFv, (Fab')$_2$ fragment, a single-domain antibody, a diabody (dAb), and a linear antibody.

Advantageous biological properties of antibodies

In some embodiments, preferably, the antibodies of the invention have one or more advantageous properties selected from the group consisting of:

(i) binding with high affinity to human ANG-2 but not to human ANG-1, wherein, in one embodiment, the antibodies bind to human ANG2 with a $K_D$ value of about 0.1-10 $\times$ 10$^{-9}$ M, but not to human ANG1 (no tendency to associate and dissociate), as measured by ForteBio affinity assay, e.g., as described in Example 3.1, and wherein in another embodiment, the antibodies bind to human ANG2 with a $K_D$ value of about 1-10 $\times$ 10$^{-11}$ M, as measured by surface plasmon resonance-based affinity assay, e.g., as described in Example 3.2;

(ii) having immunological cross-reactivity with monkey ANG-2 and rabbit ANG-2 but not with murine ANG-2, wherein, in one embodiment, the antibodies bind to the C-terminal FLD domain of cynomolgus monkey ANG2, with a $K_D$ value of about 1-10 $\times$ 10$^{-8}$ M as measured by ForteBio affinity assay, e.g., as described in Example 3.1, and wherein in another embodiment, the antibodies bind to rhesus monkey ANG2 and/or rabbit ANG2, with a $K_D$ value of about 1-10 $\times$ 10$^{-10}$ M , as measured by surface plasmon resonance-based affinity assay, e.g., as described in Example 3.2;

(iii) blocking the binding of human ANG-2 to human Tie2 protein, wherein in some embodiments, IC50 values of the antibodies are detected in an ELISA assay based on human ANG2 or SA-biotin-ANG2, e.g., as described in Example 5, and wherein in some embodiments, the antibodies of the invention have an IC50 value of about 0.1-5 nM;

(iv) blocking the binding of human ANG-2 to human Tie2 expressed on a cell surface, wherein in some embodiments, IC50 values of the antibodies are detected in a FACS assay, e.g., as described in Example 6, using, for example, 293 cells expressing human TIE2, and wherein, in some embodiments, the antibodies of the invention have an IC50 value of about 1-5 nM, and in some embodiments, the antibodies of the invention completely block the binding of ANG2 to Tie2 expressing cells;

(v) inhibiting of human ANG-2-induced Tie2 phosphorylation in endothelial cells, wherein in some embodiments, IC50 values of the antibodies are determined by detecting the changes in the amount of phosphorylated TIE2 in the cells induced by the antibodies upon incubation of the antibodies with human ANG-2 and TIE2-expressing cells (such as 293 cells), e.g., as described in Example 7, and wherein, in some embodiments, the IC50 values are about 1-5 nM;

(vi) blocking the binding of human ANG-2 to an integrin and inhibiting cell adhesion mediated by ANG2/integrin, wherein, in some embodiments, the inhibitory effect of the antibodies on hANG2/integrin-mediated cell adhesion is detected on integrin-expressing cells such as U87 cells using CellTiter-Glo® luminescence assay, e.g., as described in Example 8;

(vii) exhibiting high binding specificity and selectivity for ANG2-expressing cells, relative to cells not expressing ANG2.

Preferably, the antibodies or antigen-binding fragments thereof of the present invention exhibit at least one, more preferably at least two, more preferably at least three, four, or five of the above properties, and even more preferably all of the above properties. Preferably, the antibodies of the invention are a humanized antibody. Still preferably, the antibodies of the invention are antibodies produced and isolated from mammalian producer cells, such as CHO cells.

CDR Region of Antibody

"Complementarity determining region" or "CDR region" or "CDR" (used interchangeably herein with hypervariable region "HVR") is an amino acid region in the variable region of an antibody that is predominantly responsible for binding to an epitope of an antigen. The CDRs of heavy and light chains are commonly referred to as CDR1, CDR2, and CDR3, numbered sequentially from the N-terminus. The CDRs located within the variable domain of an antibody heavy chain are referred to as HCDR1, HCDR2, and HCDR3, while the CDRs located within the variable domain of an antibody light chain are referred to as LCDR1, LCDR2, and LCDR3.

The VH and VL sequence combinations of some exemplary antibodies of the invention are given in Table B below:

| Antibody | VH comprising or consisting of an amino acid sequence shown in the following SEQ ID NO | VL comprising or consisting of an amino acid sequence shown in the following SEQ ID NO |
|---|---|---|
| 1 | SEQ ID NO:19 | SEQ ID NO:20 |
| 2 | SEQ ID NO:21 | SEQ ID NO:22 |
| 3 | SEQ ID NO:23 | SEQ ID NO:24 |
| 4 | SEQ ID NO:25 | SEQ ID NO:26 |
| 5 | SEQ ID NO:27 | SEQ ID NO:28 |
| 6 | SEQ ID NO:29 | SEQ ID NO:30 |

In some preferable embodiments, an antibody of the invention comprises: (i) the HCDR1, HCDR2 and HCDR3 sequences contained in the VH of SEQ ID NO: 25; and (ii) the LCDR1, LCDR2 and LCDR3 sequences contained in the VL of SEQ ID NO: 26.

Various schemes for determining the CDR sequences of a given VH or VL amino acid sequence are known in the art. For example, Kabat complementarity determining regions (CDRs) are determined based on sequence variability and are the most commonly used ones (Kabat et al., Sequences of Proteins of Immunological Interest, 5th Ed. Public Health Service, National Institutes of Health, Bethesda, Md. (1991)). Chothia scheme refers to the positions of the structural loops (Chothia and Lesk, J. Mol. Biol. 196: 901-917 (1987)). AbM HVRs are a compromise between Kabat HVRs and Chothia structural loops and are used by Oxford Molecular's AbM antibody modeling software. "Contact" HVRs are based on analysis of available complex crystal structures. Residues of each HVR/CDR of these HVRs according to the different CDR determination shemes are described below.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

HVRs can also be HVR sequences located according to the Kabat numbering system at the following Kabat residue positions:

Positions 24-36 or 24-34 (LCDR1), positions 46-56 or 50-56 (LCDR2), and positions 89-97 or 89-96 (LCDR3) in VL; and positions 26-35 or 27-35B (HCDR1), positions 50-65 or 49-65 (HCDR2), and positions 93-102, 94-102, or 95-102 (HCDR3) in VH.

In one embodiment, the HVRs of an antibody of the present invention are HVR sequences located according to the Kabat numbering system at the following Kabat residue positions:

Positions 24-34 (LCDR1), positions 50-56 (LCDR2), and positions 89-97 (LCDR3) in VL, and

positions 26-35 (HCDR1), positions 50-65 (HCDR2), and positions 95-102 (HCDR3) in VH.

HVRs can also be determined based on the same positions according to Kabat numbering as a reference CDR sequence (e.g., any one of the exemplary CDRs of the invention, for example, the sequences of SEQ ID NOs: 1-6).

Unless otherwise specified, the term "CDR" or "CDR sequence" or "HVR" or "HVR sequence" used herein includes HVR or CDR sequences determined in any of the ways described above.

Unless otherwise specified, residue positions in an antibody variable region (including heavy chain variable region residues and light chain variable region residues) referred to herein are numbered according to the Kabat numbering system (Kabat et al., Sequences of Proteins of Immunological Interest, 5th Ed. Public Health Service, National Institutes of Health, Bethesda, Md. (1991)).

Table C below gives some exemplary CDR sequence combinations of the invention:

| Combination | HCDR1, comprising or consisting of the amino acid sequence shown in the following SEQ ID NO | HCDR2, comprising or consisting of the amino acid sequence shown in the following SEQ ID NO | HCDR3, comprising or consisting of the amino acid sequence shown in the following SEQ ID NO | LCDR1, comprising or consisting of the amino acid sequence shown in the following SEQ ID NO | LCDR2, comprising or consisting of the amino acid sequence shown in the following SEQ ID NO | LCDR3, comprising or consisting of the amino acid sequence shown in the following SEQ ID NO |
|---|---|---|---|---|---|---|
| 1 | SEQ ID NO:1 | SEQ ID NO:2 | SEQ ID NOT | SEQ ID NO:4 | SEQ ID NO:5 | SEQ ID NO:6 |
| 2 | SEQ ID NO:7 | SEQ ID NO:8 | SEQ ID NO:9 | SEQ ID NO:10 | SEQ ID NO:11 | SEQ ID NO:12 |
| 3 | SEQ ID NO:13 | SEQ ID NO:14 | SEQ ID NO:15 | SEQ ID NO:16 | SEQ ID NO:17 | SEQ ID NO:18 |

In one embodiment, an antibody or antigen-binding fragment thereof of the invention comprises a heavy chain variable region and a light chain variable region, wherein the antibody comprises:

(i) six CDR sequences contained in the VH and VL sequences of any of the antibodies listed in Table B; or

(ii) six CDR sequences contained in any of the combinations listed in Table C; or

(iii) six CDR sequences, which, relative to the sequences of (i) or (ii), collectively include at least one but not more than 10 or 5, 4, 3, 2, 1 amino acid modifications (preferably amino acid substitutions, preferably conservative substitutions).

In a preferable embodiment, an antibody or antigen-binding fragment thereof of the invention comprises:

(i) the HCDR1, HCDR2 and HCDR3 sequences of the heavy chain variable region as shown in SEQ ID NO: 19, 21, or 23, and the LCDR1, LCDR2 and LCDR3 sequences of the light chain variable region as shown in SEQ ID NO: 20, 22, or 24,

(ii) the HCDR1, HCDR2 and HCDR3 sequences of the heavy chain variable region as shown in SEQ ID NO: 25, and the LCDR1, LCDR2 and LCDR3 sequences of the light chain variable region as shown in SEQ ID NO: 26,

(iii) the HCDR1, HCDR2 and HCDR3 sequences of the heavy chain variable region as shown in SEQ ID NO: 27, and the LCDR1, LCDR2 and LCDR3 sequences of the light chain variable region as shown in SEQ ID NO: 28, or

(iv) the HCDR1, HCDR2 and HCDR3 sequences of the heavy chain variable region as shown in SEQ ID NO: 29 and the LCDR1, LCDR2 and LCDR3 sequences of the light chain variable region as shown in SEQ ID NO: 30.

In a preferable embodiment, an antibody or antigen-binding fragment thereof of the invention comprises three complementarity-determining regions of a heavy chain variable region (HCDRs), and three complementarity-determining regions of a light chain variable region (LCDRs), wherein,

- HCDR1 comprises or consists of an amino acid sequence selected from the group consisting of SEQ ID NOs: 1, 7, 13 and 54,

- HCDR2 comprises or consists of an amino acid sequence selected from the group consisting of SEQ ID NOs: 2, 8, 14 and 55,

- HCDR3 comprises or consists of an amino acid sequence selected from the group consisting of SEQ ID NOs: 3, 9, 25 and 56,

- LCDR1 comprises or consists of an amino acid sequence selected from the group consisting of SEQ ID NOs: 4, 10, 16 and 57,

- LCDR2 comprises or consists of an amino acid sequence selected from the group consisting of SEQ ID NOs: 5, 11, 17 and 58, and

- LCDR3 comprises or consists of an amino acid sequence selected from the group consisting of SEQ ID NOs: 6, 12, 18 and 59.

In a preferable embodiment, an antibody or antigen-binding fragment thereof of the invention comprises three complementarity-determining regions of a heavy chain variable region (HCDRs), and three complementarity-determining regions of a light chain variable region (LCDRs), wherein,

(a) HCDR1 comprises the amino acid sequence shown in SEQ ID NO: 1; HCDR2 comprises the amino acid sequence shown in SEQ ID NO: 2; HCDR3 comprises the amino acid sequence shown in SEQ ID NO: 3; LCDR1 comprises the amino acid sequence shown in SEQ ID NO: 4; LCDR2 comprises the amino acid sequence shown in SEQ ID NO: 5, and LCDR3 comprises the amino acid sequence shown in SEQ ID NO: 6; or

(b) HCDR1 comprises the amino acid sequence shown in SEQ ID NO: 7, HCDR2 comprises the amino acid sequence shown in SEQ ID NO: 8; HCDR3 comprises the amino acid sequence shown in SEQ ID NO: 9; LCDR1 comprises the amino acid sequence shown in SEQ ID NO: 10; LCDR2 comprises the amino acid sequence shown in SEQ ID NO: 11, and LCDR3 comprises the amino acid sequence shown in SEQ ID NO: 12; or

(c) HCDR1 comprises the amino acid sequence shown in SEQ ID NO: 13; HCDR2 comprises the amino acid sequence shown in SEQ ID NO: 14; HCDR3 comprises the amino acid sequence shown in SEQ ID NO: 15; LCDR1 comprises the amino acid sequence shown in SEQ ID NO: 16; LCDR2 comprises the amino acid sequence shown in SEQ ID NO: 17, and LCDR3 comprises the amino acid sequence shown in SEQ ID NO: 18; or

(d) HCDR1 comprises the amino acid sequence shown in SEQ ID NO: 54; HCDR2 comprises the amino acid sequence shown in SEQ ID NO: 55; HCDR3 comprises the amino acid sequence shown in SEQ ID NO: 56; LCDR1 comprises the amino acid sequence shown in SEQ ID NO: 57; LCDR2 comprises the amino acid sequence shown in SEQ ID NO: 58, and LCDR3 comprises the amino acid sequence shown in SEQ ID NO: 59.

In a preferable embodiment, an antibody or antigen-binding fragment thereof of the invention comprises three complementarity-determining regions of a heavy chain variable region (HCDRs), and three complementarity-determining regions of a light chain variable region (LCDRs), wherein,

- HCDR1 comprises or consists of the amino acid sequence of SEQ ID NO: 1,

- HCDR2 comprises or consists of the amino acid sequence of SEQ ID NO: 2,

- HCDR3 comprises or consists of the amino acid sequence of SEQ ID NO: 3,

- LCDR1 comprises or consists of the amino acid sequence of SEQ ID NO: 4,

- LCDR2 comprises or consists of the amino acid sequence of SEQ ID NO: 5, and

- LCDR3 comprises or consists of the amino acid sequence of SEQ ID NO: 6.

In some embodiments, the present invention also contemplates antibodies comprising variants of the CDR sequences disclosed herein. For example, using at least two of the Kabat, Chothia, AbM, and Contactschemes, the smallest overlapping region can be determined to provide a "minimum binding unit" for antigen binding. As will be understood by those skilled in the art, the residues in the remaining portions of the CDR sequences outside the minimum binding unit can be determined by the structure and protein folding of the antibody. Thus, as an example of a variant of a CDR, the amino acid residues of the minimal binding unit may remain unchanged, while one or more of the remaining CDR residues according to the Kabat or Chothia scheme are conservatively substituted, such as by those conservative substitution residues presented in Table A. The activities of the antibodies, including the binding activity to human ANG-2, the activity of blocking ANG-2 binding to Tie2 and/or integrins, and/or the activity of modulating vascular permeability and/or vascular inflammatory condition, can be characterized using the methods described herein, such as those described in the Examples. As will be understood, each CDR may be modified individually or in combination. Preferably, the amino acid modification is an amino acid substitution, especially a conservative amino acid substitution, such as a preferred conservative amino acid substitution set out in Table A. In some embodiments, preferably, the amino acid substitution occurs at an amino acid position corresponding to an X residue of a consensus CDR sequence provided herein (e.g., SEQ ID NO: 54, 55, 56, 57, 58, or 59).

Antibody Variable Region

A "variable region" or "variable domain" is a domain in the heavy or light chain of an antibody that is involved in binding of the antibody to antigen thereof. A heavy chain variable region (VH) and a light chain variable (VL) region can be further subdivided into hypervariable regions (HVRs, also known as complementarity-determining regions (CDRs)), interspersed with more conservative regions (i.e., framework regions (FRs)). Each VH or VL consists of three CDRs and four FRs, arranged in the following order from amino-terminus to carboxy-terminus: FR1, CDR1, FR2, CDR2, FR3, CDR3, FR4.

As known in the art, one or more residues in one or both of the two variable regions (i.e., VH and/or VL) of an antibody can be modified, for example, a residue modification, especially a conservative residue substitution, can be made in one or more CDR regions and/or in one or more framework regions, while the resulted modified antibody still substantially retains at least one biological property (e.g., ability to bind to antigen) as compared to before the modification. The binding properties or other functional properties of a mutated antibody can be assessed by *in vitro* or *in vivo* assays. For example, a framework region residue can be mutated, e.g., to improve a property of an antibody. For example, one or more framework residues can be " back-mutated" to the residue(s) of a corresponding germline sequence.

CDR grafting is another type for antibody variable region modification known in the art. Since CDR sequences are responsible for most antibody-antigen interactions, a recombinant antibody variant can be constructed that mimic the properties of a known antibody. In such an antibody variant, CDR sequences from a known antibody are grafted onto framework regions from a different antibody with a different property. Thus, in one embodiment, the present invention relates to an anti-ANG-2 antibody, or antigen-binding fragment thereof, comprising CDR sequences from the heavy and light chain variable regions of one of the antibodies presented in Table B, but with different framework region sequences. The framework region sequences for such substitution can be obtained from public DNA databases, including germline antibody gene sequences, or from published anti-ANG-2 antibody sequences. For example, germline DNAs for human heavy and light chain variable region genes are available from the GenBank database. The sequence of the antibody protein can be compared with the protein sequences in databases using sequence similarity search tools, such as Gapped BLAST. Preferably, as compared to the framework sequence of the antibody of the invention selected for modification, the framework sequence for substitution has a structural similarity, e.g., a sequence identity of at least 80%, 85% or 90%, or more than 95%, 96%, 97%, 98% or 99%.

Thus, in one embodiment, an antibody of the invention comprises, or consists of, the heavy chain variable region VH sequence of any of the antibodies listed in Table B. In yet another embodiment, an antibody of the invention comprises a variant of said VH sequence.

In another embodiment, an antibody of the invention comprises, or consists of, the light chain variable region VL sequence of any of the antibodies listed in Table B. In yet another embodiment, an antibody of the invention comprises a variant of said VL sequence.

In yet another embodiment, an antibody of the invention comprises:

(i) a VH sequence comprising the amino acid sequence shown in SEQ ID NO: 19 or 21 or 23, or a variant thereof, and/or a VL sequence comprising the amino acid sequence shown in SEQ ID NO: 20 or 22 or 24, or a variant thereof, or

(ii) a VH sequence comprising the amino acid sequence shown in SEQ ID NO: 25 or 27 or 29, or a variant thereof, and/or a VL sequence comprising the amino acid sequence shown in SEQ ID NO: 26 or 28 or 30, or a variant thereof.

In one embodiment, the variant of the VH sequence has at least 80%, 85%, 90%, 92%, 95%, 97%, 98%, 99% or greater identity in amino acid sequence compared to the reference VH sequence, preferably over the full length or within the three regions of CDR1, 2 and 3. In one embodiment, the variant of the VH sequence comprises at least one and no more than 30, 10, or 5, 4, 3, 2, 1, 0 amino acid modifications (preferably amino acid substitutions, preferably conservative substitutions) in amino acid sequence compared to the reference VH sequence, preferably, over the full length or within the three regions of CDR1, 2, and 3. Preferably any of the sequence differences do not occur in the CDR regions.

In one preferable embodiment, the variant of the VL sequence has at least 80%, 85%, 90%, 92%, 95%, 97%, 98%, 99% or greater identity in amino acid sequence compared to the reference VL sequence, preferably over the full length or within the three regions of CDR1, 2 and 3. In one preferable embodiment, the variant of the VL sequence comprises at least one and no more than 30, 10, or 5, 4, 3, 2, 1, 0 amino acid modifications (preferably amino acid substitutions, preferably conservative substitutions) in amino acid sequence compared to the reference VL sequence, preferably over the full length or within the three regions of CDR1, 2, and 3. Preferably any of the sequence differences do not occur in the CDR regions.

In one preferable embodiment, an antibody of the invention comprises or consists of the VH/VL sequence pairs of the heavy chain variable region and the light chain variable region of any of the antibodies presented in Table B. The present invention also provides variants of the antibody, such as variants having at least 95% to 99% identity or comprising no more than 10 amino acid modifications in the VH, the VL, or both the VH and the VL. Preferably any of the sequence differences do not occur in the CDR regions.

In some embodiments, the present invention provides an antibody, or antigen-binding fragment thereof, comprising:

(i) a heavy chain variable region comprising or consisting of an amino acid sequence having at least 80%, 85% or 90% sequence identity to the amino acid sequence shown in SEQ ID NO: 19, 21, or 23, and/or a light chain variable region comprising or consisting of an amino acid sequence having at least 80%, 85% or 90% sequence identity to the amino acid sequence shown in SEQ ID NO: 20, 22, or 24; or

(ii) a heavy chain variable region comprising or consisting of an amino acid sequence having at least 80%, 85% or 90% sequence identity to the amino acid sequence shown in SEQ ID NO: 25, 27, or 29, and/or a light chain variable region comprising or consisting of an amino acid sequence having at least 80%, 85% or 90% sequence identity to the amino acid sequence shown in SEQ ID NO: 26, 28, or 30.

In some embodiments, the present invention provides an antibody, or antigen-binding fragment thereof, comprising a heavy chain variable region and a light chain variable region selected from the group consisting of:

(i) a heavy chain variable region comprising or consisting of the amino acid sequence shown in SEQ ID NO: 19, 21, or 23, and a light chain variable region comprising or consisting of the amino acid sequence shown in SEQ ID NO: 20, 22, or 24; or

(ii) a heavy chain variable region comprising or consisting of the amino acid sequence shown in SEQ ID NO: 25, 27, or 29, and a light chain variable region comprising or consisting of the amino acid sequence shown in SEQ ID NO: 26, 28, or 30.

In a preferred embodiment of the invention, an antibody of the invention comprises a heavy chain variable region, wherein the heavy chain variable region comprises or consists of the sequence of SEQ ID NO: 25. In yet another preferable embodiment of the invention, an antibody of the invention comprises a light chain variable region, wherein the light chain variable region comprises or consists of the sequence of SEQ ID NO: 26. In a yet preferable embodiment, an antibody of the present invention comprisesa heavy chain variable region with VH sequence of SEQ ID NO: 25 and a light chain variable region with VL sequence of SEQ ID NO: 26.

In yet another embodiment of the invention, an antibody of the invention comprises:

(i) a VH sequence comprising the amino acid sequence shown in SEQ ID NO: 25 or a sequence having at least 80%, 85%, or 90%, or more than 95%, 96%, 97%, 98%, or 99% sequence identity thereto, and/or

(ii) a VL sequence comprising the amino acid sequence shown in SEQ ID NO: 26 or a sequence having at least 80%, 85%, or 90%, or more than 95%, 96%, 97%, 98%, or 99% sequence identity. Preferably any of the sequence differences do not occur in the CDR regions.

In yet more preferable embodiments, the present invention relates to anti-ANG-2 antibodies, or antigen-binding fragments thereof, and their uses, wherein the antibodies comprise the CDR sequences contained in the heavy chain variable region of SEQ ID NO: 25 and the light chain variable region of SEQ ID NO: 26, but having different framework region sequences. Preferably, as compared to the framework sequences of the antibody of the invention selected for modification, the framework sequences for substitution have structural similarity, e.g., a sequence identity of at least 80%, 85% or 90%, or more than 95%, 96%, 97%, 98% or 99%.

Antibody heavy chains and light chains

In some embodiments, an antibody of the invention comprises a heavy chain Fc region, such as an Fc region of an IgG1, IgG2 or IgG4 isotype. In one embodiment, an antibody of the invention comprises an IgG1 Fc region. In some embodiments, an antibody of the invention comprises a kappa light chain constant region, such as a human kappa light chain constant region.

In yet another preferable embodiment, the Fc region comprises the amino acid sequence of SEQ ID NO: 52, or an amino acid sequence comprising at least one, two or three, but not more than 20, 10 or 5 amino acid modifications relative to the amino acid sequence of SEQ ID NO: 52, or a sequence having at least 95% to 99% identity to the amino acid sequence of SEQ ID NO: 52.

In a preferable embodiment of the invention, an antibody of the invention comprises a light chain constant region. In a preferable embodiment, the light chain constant region is a human kappa light chain constant region. In yet another preferable embodiment, the light chain constant region comprises the amino acid sequence of SEQ ID NO: 53, or an amino acid sequence comprising at least one, two or three, but not more than 20, 10 or 5 amino acid modifications relative to the amino acid sequence of SEQ ID NO: 53, or a sequence having at least 95% to 99% identity to the amino acid sequence of SEQ ID NO: 53.

In some preferable embodiments, an antibody of the invention comprises a heavy chain, which comprises an amino acid sequence selected from the group consisting of SEQ ID NOs: 31, 33, 35, 37, 39, and 41, or an amino acid sequence comprising at least 1, 2 or 3, but no more than 20, 10 or 5 amino acid modifications relative thereto, or an amino acid sequence having at least 80%, 85%, 90%, 92%, 95%, 97%, 98%, 99% or greater identity thereto. Preferably, the amino acid modifications do not occur in the CDR regions and, more preferably, do not occur in the variable regions.

In some preferable embodiments, an antibody of the invention comprises a light chain, which comprises an amino acid sequence selected from the group consisting of SEQ ID NOs: 32, 34, 36, 38, 40, and 42, or an amino acid sequence comprising at least 1, 2 or 3, but not more than 20, 10 or 5 amino acid modifications relative thereto, or an amino acid sequence having at least 80%, 85%, 90%, 92%, 95%, 97%, 98%, 99% or greater identity thereto. Preferably, the amino acid modifications do not occur in the CDR regions and, more preferably, do not occur in the variable regions.

In a preferable embodiment, an antibody of the invention comprises a heavy chain sequence and/or a light chain sequence selected from:

(a) a heavy chain sequence comprising the amino acid sequence of SEQ ID NO: 31, or a variant thereof, and/or a light chain sequence comprising the amino acid sequence of SEQ ID NO: 32, or a variant thereof;

(b) a heavy chain sequence comprising the amino acid sequence of SEQ ID NO: 33, or a variant thereof, and/or a light chain sequence comprising the amino acid sequence of SEQ ID NO: 34, or a variant thereof;

(c) a heavy chain sequence comprising the amino acid sequence of SEQ ID NO: 35, or a variant thereof, and/or a light chain sequence comprising the amino acid sequence of SEQ ID NO: 36, or a variant thereof;

(d) a heavy chain sequence comprising the amino acid sequence of SEQ ID NO: 37, or a variant thereof, and/or a light chain sequence comprising the amino acid sequence of SEQ ID NO: 38, or a variant thereof;

(e) a heavy chain sequence comprising the amino acid sequence of SEQ ID NO: 39, or a variant thereof, and/or a light chain sequence comprising the amino acid sequence of SEQ ID NO: 40, or a variant thereof;

(d) a heavy chain sequence comprising the amino acid sequence of SEQ ID NO: 41, or a variant thereof, and/or a light chain sequence comprising the amino acid sequence of SEQ ID NO: 42, or a variant thereof;
wherein, relative to the respective reference sequence, the variant comprises an amino acid sequence with at least 1, 2 or 3, but not more than 20, 10 or 5 amino acid modifications, or an amino acid sequence having at least 80%, 85%, 90%, 92%, 95%, 97%, 98%, 99% or greater identity . Preferably, the amino acid modifications do not occur in the CDR regions and, more preferably, do not occur in the variable regions.

In one embodiment, a residue modification is introduced into a constant region of the antibody, for example, to alter a property of the antibody, such as an effector function.

In a preferable embodiment, an antibody of the present invention comprises a heavy chain sequence as shown in SEQ ID NO: 37 and a light chain sequence as shown in SEQ ID NO: 38.

Exemplary antibody sequences

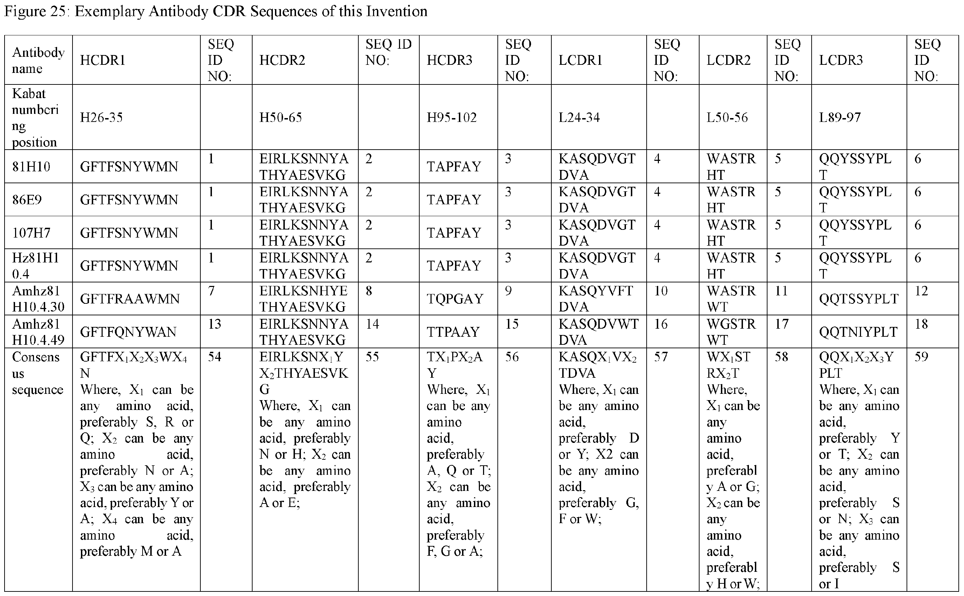

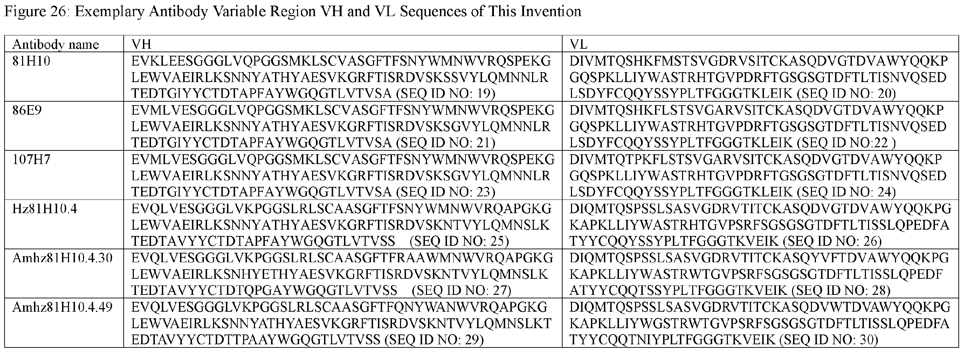

The present invention provides antibodies that specifically bind to ANG-2 (e.g., human ANG-2), which were isolated and characterized as described in the Examples. Figure 26 shows the VH and VL sequences in the variable regions of the exemplary antibodies of the present invention. Figure 25 shows the exemplary CDR sequences of the antibodies. The heavy and light chain amino acid sequences and the nucleotide sequences encoding the variable regions VH and VL of the exemplary antibodies of the invention are shown in the Sequence Listing.

Antibody variants

In one aspect, the present invention provides variants of any of the antibodies described herein, especially the exemplary antibodies listed in Table B. In one embodiment, the antibody variant retains at least 60%, 70%, 80%, 90%, or 100% of a biological activity (e.g., antigen-binding activity) of the unmodified antibody. In some embodiments, the modification in the antibody variant do not cause loss of the antigen-binding activity, but optionally conferring a property such as an improved antigen-binding affinity and a different effector function.

As will be understood, the heavy chain variable region, the light chain variable region, or each CDR region, of an antibody can be modified individually or in combination. In some embodiments, an antibody of the invention has at least 80%, 85%, 90%, 91%, 92%, 93%, 94%, 95%, 96%, 97%, 98%, or 99% or greater sequence identity in heavy chain variable region, as compared to a reference antibody (e.g., one of the antibodies listed in Table D). In yet another embodiment, an antibody of the invention has at least 80%, 85%, 90%, 91%, 92%, 93%, 94%, 95%, 96%, 97%, 98%, or 99% or greater sequence identity in light chain variable region, as compared to a reference antibody (e.g., one of the antibodies listed in Table D). In yet another embodiment, an antibody of the invention has at least 80%, 85%, 90%, 91%, 92%, 93%, 94%, 95%, 96%, 97%, 98%, or 99% or greater sequence identity in heavy chain variable region and/or light chain variable region, as compared to a reference antibody (e.g., one of the antibodies listed in Table D).

In addition, the Fc region of an antibody can be altered. The modification in the Fc region can be made alone or in combination with the modification to the framework and/or CDR regions described above. The Fc region may be modified, for example, so as to alter one or more of the functions of the antibody, such as serum half-life, complement fixation, Fc receptor binding, and/or antigen-dependent cytotoxicity. In addition, an antibody of the invention can also be chemically modified (e.g., conjugated with PEG), or have altered glycosylation pattern.

In some embodiments, the Fc region may comprise an Fc region having one or more amino acid substitutions that increase ADCC activity, e.g., substitutions at Fc region positions 298, 333, and/or 334 (EU numbering scheme for residues). In some embodiments, the Fc region can also be modified to result in altered (i.e., increased or decreased) C1q binding and/or complement-dependent cytotoxicity (CDC) (see, for example, US6,194,551, WO99/51642 and Idusogie, E. E. et al., J. Immunol. 164 (2000): 4178-4184).

In some other embodiments, the Fc region can be modified to increase or decrease its glycosylation degree and/or to alter its glycosylation pattern. Addition or deletion of glycosylation sites of the Fc region can be conveniently achieved by altering the amino acid sequence so as to create or remove one or more glycosylation sites. For example, one or more amino acid substitutions can be made to eliminate one or more glycosylation sites, thereby eliminating glycosylation at the sites. Antibodies with altered glycosylation patterns can be prepared, such as low- or non-fucosylated antibodies with reduced contents of fucosyl residues or antibodies with increased bisecting GlcNac structures. Such altered glycosylation patterns have been shown to increase ADCC activities of antibodies. The present invention also contemplates antibody variants having at least one galactose residue in an oligosaccharide attached to Fc region. These antibody variants may have an improved CDC function.

In certain embodiments, the present invention also contemplates antibody variants that have some but not all effector functions, which makes them ideal candidates for certain applications where the *in vivo* half-life of an antibody is important, but some effector functions, such as complement and ADCC, are unnecessary or deleterious. For example, Fc region may comprise a mutation that eliminates or attenuates an effector function, such as a human IgG1 Fc region harboring the mutations P329G and/or L234A and L235A, or a human IgG4 Fc region harboring the mutations P329G and/or S228P and L235E.

In some embodiments, mutations that may facilitate antibody production in the cross-mAb format may be introduced into the Fc region, such as knob-into-hole mutations. See , e.g., WO2016075035.

In certain embodiments, it may be desirable to produce cysteine-engineered antibodies, such as " thioMabs", in which one or more antibody residues are substituted with a cysteine residue. For example, the number of cysteine residues in the hinge region of an antibody can be altered, e.g., to facilitate the assembly of light and heavy chains or increase or decrease the stability of the antibody. See, e.g., U.S. Patent No. 5,677,425.

In certain embodiments, the antibodies provided herein can be further modified to contain a non-protein moiety. The moieties suitable for antibody derivatization include, but are not limited to, water soluble polymers. Non-limiting examples of water-soluble polymers include, but not limited to, polyethylene glycol (PEG), which can be used, for example, to extend the half-life (e.g., in serum) of an antibody. Methods for PEGylation of proteins are known in the art and can be applied to the antibodies of the invention. See, e.g., EP 0154 316 and EP 0401384.

## II. Polynucleotides, Vectors and Hosts

The present invention provides a nucleic acid that encodes any of the above anti-ANG-2 antibodies or fragments thereof. A vector comprising the nucleic acid is also provided. In one embodiment, the vector is an expression vector. A host cell comprising the nucleic acid or the vector is also provided. In one embodiment, the host cell is eukaryotic. In another embodiment, the host cell is selected from a yeast cell and a mammalian cell (e.g., a CHO cell or a 293 cell). In another embodiment, the host cell is prokaryotic.

In one aspect, the present invention provides a nucleic acid that encodes any of the above anti-ANG-2 antibodies or fragments thereof. The nucleic acid may comprise a nucleic acid that encodes the amino acid sequence of the light chain variable region and/or the heavy chain variable region of the antibody, or comprise a nucleic acid that encodes the amino acid sequence of the light chain and/or the heavy chain of the antibody. The polypeptides encoded by the polynucleotides exhibit ANG-2 antigen-binding ability when expressed in suitable expression vectors.

Also provided in the present invention is a polynucleotide encoding at least one CDR region, and generally all three CDR regions, of the heavy chain VH or light chain VL of an ANG-2-binding antibody described herein above. In some further embodiments, the polynucleotide encodes all or substantially all of the variable region sequence of the heavy chain and/or the light chain of the ANG2-binding antibody described herein above .

As will be understood by those skilled in the art, each antibody or polypeptide amino acid sequence may be encoded by a number of nucleic acid sequences due to codon degeneracy.

The polynucleotide sequences can be generated by de novo solid-phase DNA synthesis or by PCR mutagenesis of the sequences encoding antibodies or antigen-binding fragments thereof that bind ANG-2 using methods well known in the art.

In one embodiment, a vector(s) comprising a nucleic acid of the invention is provided. In one embodiment, the vector is an expression vector, such as an eukaryotic expression vector. Vectors include, but are not limited to, viruses, plasmids, cosmids, lambda phages, or yeast artificial chromosomes (YAC). In preferable embodiments, the expression vector of the invention is a pTT5 expression vector.

In one embodiment, a host cell comprising the vector is provided. Host cells suitable for cloning or expressing vectors encoding antibodies include prokaryotic or eukaryotic cells described herein. For example, antibodies can be produced in bacteria, particularly when glycosylation and Fc effector function are not required. For example, antibodies or antibody fragments can be expressed in E. coli. After expression, the antibodies can be isolated from bacterial cell pastes or soluble fractions, and further purified.

In one embodiment, the host cell is eukaryotic. In another embodiment, the host cell is selected from yeast cells, mammalian cells, or other cells suitable for producing an antibody or antigen-binding fragment thereof. For example, eukaryotic microorganisms such as filamentous fungi or yeasts are suitable hosts for cloning or expressing vectors that encode antibodies. For example, fungal and yeast strains in which the glycosylation pathway has been "humanized" can generate antibodies with partially or fully humanized glycosylation patterns. Host cells suitable for expressing glycosylated antibodies can also be derived from multicellular organisms (invertebrates and vertebrates). Vertebrate cells may be used as host cells. For example, mammalian cell lines that have been adapted for growth in suspension may be used. Further examples of useful mammalian host cell lines are SV40 transformed monkey kidney CV1 line (COS-7) and human embryonic kidney line (293HEK or 293 cells), etc. Other useful mammalian host cell lines include Chinese hamster ovary (CHO) cells, including DHFR⁻ CHO cells and myeloma cell lines such as Y0, NS0 and Sp2/0.

## III. Preparation of Antibodies

In one embodiment, a method of preparing an anti-ANG-2 antibody is provided, wherein the method comprises culturing a host cell comprising a nucleic acid that encodes the antibody, as provided hereinbefore, under conditions suitable for expressing the antibody, and optionally recovering the antibody from the host cell (or the culture medium of the host cell). To recombinantly produce anti-ANG-2 antibodies, nucleic acids encoding antibodies, such as those described hereinbefore, are isolated and inserted into one or more vectors for further cloning and/or expression in host cells. Such nucleic acids can be readily isolated and sequenced using conventional procedures (e.g., by using oligonucleotide probes capable of specifically binding to genes encoding the heavy and light chains of the antibodies).

### IV. Assays

The anti-ANG-2 antibodies provided herein can be identified, screened, or characterized for physical/chemical properties and/or biological activities by a variety of assays known in the art.

In one aspect, an antibody of the invention is tested for antigen-binding activity. The binding of the antibody to human ANG-2 can be determined e.g., by methods known in the art, such as ELISA, Western blotting, or by exemplary methods disclosed herein in the Examples, e.g., as described in Example 3 or 4. For example, the binding profile of the antibody, including binding kinetics (e.g., $K_D$ value), can be determined using a recombinant ANG-2 protein or a C-terminal FLD fragment thereof in a biological optical interferometry assay. In some embodiments, Fortebio affinity assay is employed to determine the binding affinity of the antibody to ANG2 and/or ANG1 derived from human or other species, such as cynomolgus monkeys.

In yet another aspect, the blocking effect of an antibody on ANG2/TIE2 binding or ANG2/integrin binding is assayed. The determination can be performed using e.g., a flow cytometry assay in which the antibody reacts with ANG2 and TIE2 or integrin expressing cell lines , such as 293 cells transfected to express human TIE2 on the cell surface or U87 cells that express a number of integrins.

In another aspect, competition assays can be used to identify antibodies that compete with any of the anti-ANG-2 antibodies disclosed herein for binding to ANG-2. In certain embodiments, such competitive antibodies bind to epitopes (e.g., linear or conformational epitopes) that are identical to or overlapping with the epitope bound by any anti-Ang-2 antibody disclosed herein.

The present invention also provides assays for determining the biological activities of anti-ANG-2 antibodies. Biological activities may include, for example, binding to ANG-2 (e.g., binding to human ANG-2), blocking the binding of ANG-2 (e.g., binding of human ANG-2) to Tie2 molecules or integrins, and when in combination with an anti-VEGF agent, inhibiting vascular permeability and/or neovascularization in for example a choroidal neovascularization model, and improving the integrity of blood vessels. Various exemplary assays are described in the Examples of the present invention.

As will be understood, any of the above assays can be performed using the immunoconjugates or multispecific antibodies of the present invention, in lieu of or in addition to the anti-ANG-2 antibodies.

### V. Multispecific Antibody

In yet another aspect, the present invention provides multispecific (including bispecific) antibody molecules that specifically bind to ANG-2, preferably human ANG-2. In one embodiment, the multiple-specific antibody comprises an antibody (or antigen-binding fragment thereof) of the present invention that provides a first binding specificity for ANG-2. In yet another embodiment, the multispecific antibody further comprises a second specificity for VEGF-A.

In one embodiment, binding specificities are provided by the "binding site" or "antigen-binding site" of an antibody (i.e., the region in the antibody molecule that actually binds to the antigen). In a preferable embodiment, the antigen-binding site is composed of a VH/VL pair consisting of an antibody light chain variable domain (VL) and an antibody heavy chain variable domain (VH). Thus, in one embodiment, "multispecific" antibodies have at least two antigen-binding sites, each of which binds to a different epitope on the same antigen or a different epitope on a different antigen.

### VI. Immunoconjugates

In yet another aspect, the present invention provides immunoconjugates formed by conjugating an antibody of the invention to a heterologous molecule. In one embodiment, , the antibody (or antigen-binding fragment thereof) of the invention in the immunoconjugate is conjutated to a therapeutic or diagnostic agent. In some embodiments, the antibody of the invention that is conjugated to the heterologous molecule may be a full-length antibody or an antibody fragment. For example, the antibody may be conjugated in the form of a fragment, such as Fab fragment, Fab' fragment, $F(ab)'_2$ fragment, single-chain scFab antibody and single-chain scFv.

Linkers can be used to covalently link different entities of the conjugates. Suitable linkers include chemical linkers or peptide linkers. Advantageously, the linker is a "cleavable linker" that facilitates the polypeptide release upon delivery to a target site. For example, acid-labile linkers, peptidase-sensitive linkers, photolabile linkers, dimethyl linkers, or disulfide-containing linkers can be used.

In some embodiments, an antibody of the invention can be conjugated to a diagnostic or detectable agent. Such conjugates can be used as part of a clinical test method (e.g., to determine the efficacy of a particular therapy), or used for monitoring or predicting the onset, development, progression, and/or severity of a disease or a disorder. To achieve the diagnosis and detection, the antibody can be conjugated to a detectable agent, including, but not limited to, a variety of enzymes, e.g., but not limited to, horseradish peroxidase; prosthetic groups, e.g., but not limited to, streptavidin/biotin and avidin/biotin; fluorescent substances; luminescent substances; radioactive materials; as well as positron-emitting metals and non-radioactive paramagnetic metal ions used in various positron emission tomography imaging procedures.

## VII. Pharmaceutical Compositions, Pharmaceutical formulations and Combination Products

The present invention also encompasses compositions (including pharmaceutical compositions or pharmaceutical formulations) comprising anti-ANG-2 antibodies or immunoconjugates or multispecific antibodies thereof and compositions comprising polynucleotides encoding anti-ANG-2 antibodies or immunoconjugates or multispecific antibodies thereof. These compositions can optionally further comprise suitable pharmaceutical auxiliary materials, such as pharmaceutical carriers and pharmaceutical excipients (including buffers) known in the art. Antibodies of the invention can be formulated in any suitable dosage form. In some embodiments, antibodies of the invention are formulated into a formulation suitable for intraocular administration, such as a formlation suitable for intravitreal injection administration. In other embodiments, antibodies of the invention are formulated into a formulation suitable for intravenous injection administration.

Pharmaceutical carriers suitable for formulating the formulations of the invention may include, but not limited to, buffers, antioxidants, preservatives and stabilizers.

A pharmaceutical formulation of the invention can be formulated, preferably in the form of a lyophilized formulation or an aqueous solution, by mixing an anti-ANG-2 antibody, an immunoconjugate or a multispecific antibody of the invention having a desired purity with one or more optional pharmaceutical excipients (Remington's Pharmaceutical Sciences, 16th ed., Osol, A. Ed. (1980)).

Sustained release formulations can also be prepared. Relevant examples of sustained-release formulations include semi-permeable matrices of solid hydrophobic polymers containing antibodies, in the form of shaped articles, such as films or microcapsules.

A pharmaceutical composition or formulation of the invention can also be combined with additional active agent(s) which is required for the specific indication being treated, preferably those active agents having complementary activities that do not adversely affect each other. For example, it would be desirable to further provide other anti-cancer active agents, such as chemotherapeutic agents, PD-1 axis binding antagonists (e.g., anti-PD-1 antibody or anti-PD-L1 antibody or anti-PD-L2 antibody), or anti-angiogenic agents (e.g., bevacizumab). The active agents are suitably present in combination in amounts effective for the intended use.

Thus, in one aspect, the present invention also provides pharmaceutical combination products. In one embodiment, the combination product comprises an antibody, an immunoconjugate, or a multispecific antibody of the invention and a second therapeutic agent, which are formulated in the same pharmaceutical composition or formulation. In another embodiment, the combination product comprises an antibody, an immunoconjugate or a multispecific antibody of the invention and a second therapeutic agent, which are separately contained in different pharmaceutical compositions or formulations. The second therapeutic agent can be administered prior to, simultaneously with (e.g., in the same formulation or in a different formulation) or after administration of the antibody of the invention.

In some embodiments, the second therapeutic agent suitable for use in the present invention may be any therapeutic agent advantageously used in combination with an ANG2 inhibitor, such as a drug that is benefical to a vascular-related disease to be treated.

Given the importance of the VEGF signaling pathway in angiogenesis, anti-VEGF agents aiming at blocking VEGF or VEGF receptor have been proposed to inhibit angiogenesis and have already been used in the treatment of diseases involving neovascularization, such as cancer and retinopathy. Anti-VEGF agents for angiogenesis include, for example, monoclonal antibodies that specifically bind to VEGF-A, which mechanism of action is to block the binding of VEGF to its receptor by binding to VEGF; VEGFR blocking agents consisting of an extracellular fragment of VEGF receptor and an Fc region of an immunoglobulin, which have an inherently high specificity for VEGF. In CN200510115530.1, an effective anti-VEGF agent molecule is disclosed, which is composed of the second immunoglobulin-like domain of VEGFR-1, the third immunoglobulin-like domain of VEGFR-2, and a human immunoglobulin Fc, wherein a peptide linker that increases the structural flexibility is inserted before the Fc, thereby improving the stability and the biological activity of the fusion protein, so that the fusion protein can bind and neutralize VEGF, block angiogenesis, and inhibit tumor progression more effectively, both *in vitro* and in animals. Thus, in a preferable embodiment of the present invention, the second therapeutic agent for use in combination with the antibody of the present invention is an anti-VEGF agent. In yet another preferable embodiment, the anti-VEGF agent is a fusion protein formed from the second immunoglobulin-like domain of VEGFR-1, the third immunoglobulin-like domain of VEGFR-2, and a human immunoglobulin Fc, wherein a peptide linker that increases the structural flexibity is inserted before the Fc, such as the molecule shown in SEQ ID NO: 60, or a molecule having at least 90%, 91%, 92%,93%, 94%, 95%, 96%, 97%, 98%, or 99% identity thereto. Other anti-VEGF agents such as VEGF-Traps (see, e.g., US7,087,411), anti-VEGF antibodies (e.g., bevacizumab), small molecule kinase inhibitors for VEGF receptors (e.g., sunitinib) may also be used in the present invention.

Second therapeutic agents that can be used in the present invention also include, but not limited to, other drugs that inhibit or reduce angiogenesis, anti-inflammatory drugs, immune checkpoint drugs such as anti-PD1 and anti-CD40 drugs, and chemotherapeutic drugs.

In some embodiments, pharmaceutical compositions or pharmaceutical formulations and combination products of the invention are suitable for the prevention or treatment of vascular-related diseases, including diseases or disorders caused by or associated with angiogenesis and/or vascular permeability.

Pharmaceutical compositions, pharmaceutical formulations, and combination products of the invention may be provided as articles of manufacture for the treatment, prevention, and/or diagnosis of the diseases and/or disorders described herein. Such articles of manufacture may comprise a container and a label or a package insert. Suitable containers include bottles, syringes, IV infusion bags, to name a few. Such containers can be made of a variety of materials such as glass or plastic. In one embodiment, the articles of manufacture may comprise (a) a first container containing an antibody or the fragment thereof, an immunoconjugate, or a multispecific antibody of the invention, and optionally (b) a second container containing a second therapeutic agent. In addition, the articles of manufacture may contain other desirable materials from a commercial and user's view, including buffers, pharmaceutically acceptable diluents such as sterile water for injection, needles, syringes, and syringe pumps.

## VIII. Treatment Methods and Uses

The terms "individual" and "subject" are used herein interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the subject is a human.

The term "treatment" herein refers to a clinical intervention intended to alter the natural course of a disease in an individual being treated. The desired therapeutic effects include, but are not limited to, preventing disease onset or recurrence, alleviating symptom, minimizing any direct or indirect pathological consequences of disease, preventing metastasis, postponing disease progression, improving or relieving disease status, as well as alleviating or improving prognosis.

In one aspect, the present invention provides a method of inhibiting a biological activity mediated by ANG-2, including administering a therapeutic effective amount of an antibody of the invention. Accordingly, in one embodiment, the present invention relates to a method of preventing or treating a disease or disorder associated with an ANG2 activity by using an antibody of the invention. The ANG2 activity-associated diseases or disorders includes any diseases or disorders that can be ameliorated, alleviated, prevented, or treated by eliminating, inhibiting, or reducing ANG2 activity. Preferably, the antibody of the invention is used to treat vascular-related diseases, including diseases or disorders caused by, or associated with, angiogenesis. Examples of such diseases include, but are not limited to, cancers, inflammatory conditions, sepsis, and ocular vascular diseases.

In some embodiments, an antibody of the invention is used to treat solid tumors. In the treatment context, the antibody of the invention can be used alone or in combination with additional antitumor agent(s).

In some other embodiments, an antibody of the invention is used to treat ocular vascular diseases. Such ocular diseases may be caused by abnormal angiogenesis or vascular obstruction or occlusion. Examples of ocular diseases that can be treated with the antibody of the invention, particularly in combination with anti-VEGF agents, include, but are not limited to, ocular neovascularization diseases, and ocular diseases associated with choroidal neovascularization, retinal edema and inflammation. In some embodiments, when used in combination with an anti-VEGF agent for a vascular-related disease, such as an ocular disease, e.g., choroidal neovascularization (CNV), an antibody of the invention (e.g., an antibody molecule having the same six CDR sequences or the same VH and VL sequences as the antibody molecule of the invention hz81H10.4) results in one or more of reduction of vascular leakage, inhibition of neovascularization, and improvement of vascular integrity. As revealed by assessment of vascular leakage, retinal morphology, neovascularization and vascular integrity e.g., via fundus fluorescein angiography, or pathological examination based on histochemical staining (e.g., conventional HE staining can be used to examine retinal morphology such as the size of injured and swollen area, and double-fluorescence staining for CD31 and Desmin can be used to evaluate vascular endothelial cell number and pericyte/smooth muscle cell number), preferably, as compared to the anti-VEGF agent alone, the antibody of the present invention in combination with the anti-VEGF agent results in a further improved therapeutic effect, for example, improved vascular leakage inhibition, improved retinal morphology, reduced neovascularization and improved vascular integrity.

Furthermore, it has been shown that ANG2 expression is associated with a variety of inflammatory and/or infectious diseases. Racheal G. Akwii et al., Role of Angiopoietin-2 in Vascular Physiology and Pathology. Cells, 2019, 8, 471; DOI: 10.3390/cells 8050471. Therefore, in some embodiments, an antibody of the invention or antibody fragment thereof is used to treat infectious diseases. Exemplary infectious diseases include, but are not limited to, sepsis and pneumonia.

In the treatment methods of the invention, the antibodies of the invention can be administered alone or in combination with a second therapeutic agent, such as a second therapeutic agent described hereinbefore. The combined administration includes concomitant administration (wherein two or more therapeutic agents are contained in the same or different formulations) and separate administration (wherein the antibodies of the invention are administered before, simultaneously with, or after the administration of additional therapeutic agent(s)).

The antibodies of the invention (and pharmaceutical compositions or immunoconjugates comprising the same, and any additional therapeutic agent) can be administered via any suitable administration mode, including parenteral administration, intrapulmonary administration, and intranasal administration as well as intralesional administration if local treatment is required. Parenteral infusions include intramuscular, intravenous, intra-arterial, intraperitoneal or subcutaneous administration. Any suitable route, such as injection, e.g., intravenous or subcutaneous injection, can be adopted, partially depending on whether the administration is a short-time one or a long-term one. The present disclosure encompasses a variety of dosing courses, including, but not limited to, single dosing or multiple dosing at multiple time points, bolus dosing, and pulsed infusion.

For the purpose of disease prevention or treatment, the appropriate dosage of an antibody of the invention, when used alone or in combination with one or more additional therapeutic agents, will be selected depending on the type of disease to be treated, the type of antibody, the severity and course of the disease, whether the antibody is administered for prophylactic or therapeutic purpose, previous treatment, the patient's clinical history and response to the antibody treatment, as well as the judgment of the attending physician. The antibody is suitably administered to the patient in a single treatment or over a series of treatments.

In the foregoing methods of the invention, a composition, a multispecific antibody or an immunoconjugate of the invention may be administered in lieu of an antibody or antigen-binding portion of the invention. Alternatively, in these methods, in addition to administering the antibody or antigen-binding portin of the invention, the composition, multispecific antibody or immunoconjugate of the invention can be further administered.

In yet another aspect, the present invention provides the uses of anti-ANG-2 antibodies, compositions, immunoconjugates, and multispecific antibodies of the invention in the manufacture of a medicament for use in the foregoing methods (e.g., for treatment).

## IX. Methods and uses of antibodies of the invention in diseases accompanied with vascular leakage and/or vascular inflammation

In one aspect, the present invention provides methods of regulating, especially reducing, ANG-2-induced vascular permeability by using the anti-ANG2 antibodies or antigen-binding fragments thereof of the invention. The methods can be an *in vivo* or *in vitro* method.

In one embodiment, the said methods are an in vitro method. The methods can be used to screen for molecules that regulate (e.g., decrease, e.g., synergistically or additively decrease) vascular endothelial permeability when in combination with the anti-ANG2 antibodies of the invention. The said candidate molecules can be peptides or small molecule compounds, such as ANG1 analogues, VEGF inhibitors, and the like.

Preferably, in one embodiment, the said methods are an in vivo method comprising administering to an individual in need thereof a therapeutically effective amount of an antibody or antigen-binding fragment thereof of the invention. In one embodiment, the said methods are used to prevent, alleviate, ameliorate, and/or treat a disease or a disorder associated with abnormal increase of vascular permeability (e.g., acute pneumonia, acute lung injury, and acute respiratory distress syndrome). In one embodiment, the abnormal increase of vascular endothelial permeability is associated with VEGF and can be sensitized by ANG-2. In one embodiment, the abnormal increase of vascular endothelial permeability is caused by an inflammatory agent such as LPS or a bacterial or viral infection. In yet another embodiment, the said methods are used to alleviate the abnormal increase of vascular endothelial permeability in the lung. In one embodiment, the said methods further comprise the step of identifying a subject in need of reducing vascular permeability prior to administration of the anti-ANG2 antibody of the invention.

In one embodiment of the said methods, the expression of an inflammatory marker molecule on a vascular endothelial cell is reduced by the administration of an anti-ANG2 antibody of the invention. Preferably, the said inflammatory marker molecule is a molecular marker of acute inflammatory response. In some cases, increased expression of the inflammatory marker on vascular endothelial cells is induced in response to an inflammatory cytokine such as TNF-$\alpha$ or a inflammatory agent such as the bacterial endotoxin LPS. Preferably, the inflammatory marker molecule is ICAM-1 and/or VCAM-1. In one embodiment, the said methods further comprise the step of detecting the level of an inflammatory marker molecule on endothelial cell prior to the administration of the anti-ANG2 antibody of the invention. In one embodiment, the subject to be administered with the antibody of the invention has an elevated level of an inflammatory marker molecule on endothelial cell, such as ICAM-1 and/or VCAM-1. In yet another embodiment, the subject to be administered with the antibody of the invention is an individual at high risk for ARDS or acute lung injury and having an elevated level of an inflammatory marker molecule on endothelial cell, such as ICAM-1 and/or VCAM-1.

In yet another aspect, the present invention relates to a method of preventing or treating vascular leakage, comprising administering to a subject in need thereof a therapeutically effective amount of an antibody of the invention or an antigen-binding fragment thereof. In one embodiment, an ANG2 activity is blocked, inhibited, or reduced by administration of the anti-ANG2 antibody of the invention, thereby ameliorating, alleviating, preventing, or treating vascular leakage or one or more conditions associated therewith. In one embodiment, the said method further comprises the step of identifying a subject in need of blocking vascular leakage prior to the administration of the anti-ANG2 antibody of the invention.

In yet another aspect, the present invention relates to a method of preventing or treating a vascular inflammatory condition, comprising administering to a subject in need thereof a therapeutically effective amount of an antibody of the invention or an antigen-binding fragment thereof. In one embodiment, an ANG2 activity is blocked, inhibited, or reduced by administration of the anti-ANG2 antibody of the invention , thereby ameliorating, alleviating, preventing, or treating the vascular inflammatory condition. In one embodiment, the said method further comprises the step of identifying a subject in need of alleviating a vasulcar inflammatory condition prior to the administration of the anti-ANG2 antibody of the invention.

In yet another aspect, the invention also relates to a method of preventing or treating a disease or a disorder accompanied with vascular leakage and/or vascular inflammatory condition, the said method comprising administering to a subject in need thereof a therapeutically effective amount of an antibody of the invention or an antigen-binding fragment thereof. In one embodiment, the disease or disorder is a disease or disorder accompanied with vascular leakage. In one embodiment, the disease or disorder is a disease or disorder accompanied with vascular inflammation. Examples of such diseases or conditions include, but are not limited to, pulmonary inflammatory conditions such as pneumonia, acute lung injury (ALI), and acute respiratory distress syndrome (ARDS) and sepsis.

Pulmonary inflammation or pneumonia is defined as a disease characterized by inflammation of the lung. Most commonly, the disease is caused by a lung infection. In a preferable embodiment of the methods and uses of the invention, the pneumonia is acute pneumonia, especially acute pneumonia at risk of developing ARDS or acute lung injury (ALI). Preferably, in embodiments of the methods and uses of the present invention, the administration of an anti-ANG2 antibody blocks, prevents, alleviates or reduces the inflammatory process in the lung. The improvement of the inflammatory process in the lung can be observed by lung biopsy or lung CT images.

Acute lung injury (ALI) is an acute hypoxic respiratory insufficiency caused by diffuse pulmonary interstitial and alveolar edema due to the damage of alveolar epithelial cells and capillary endothelial cells induced by various direct and indirect injury factors. The pathophysiological features of ALI include reduced lung volume, reduced lung compliance, and ventilation/blood flow imbalance. Clinically, it is characterized by progressive hypoxemia. Acute respiratory distress syndrome (ARDS) may occur when the injury reaches a certain degree.

Acute respiratory distress syndrome is also called ARDS. The disease is a type of acute lung injury, characterized by extensive lung inflammation. Patients with ARDS usually manifest with bilateral lung infiltration and severe hypoxemia. The diagnostic criteria of ARDS includes time of onset; chest imaging (such as X-ray or CT scanning of the chest); pulmonary edema; and the degree of hypoxia. Hypoxemia for the diagnosis of ARDS can be defined, for example, by the oxygenation index of the patient's arterial blood ($PaO_2/FiO_2$; i.e., the ratio of the partial pressure of arterial oxygen to the fraction of inspired oxygen). In the 2012 Berlin definition of ARDS, based on the degree of hypoxemia determined by PaO2/FiO2, ARDS is classified into three categories: mild ARDS ($PaO_2/FiO_2$ 200-300mm Hg), moderate ARDS ($PaO_2/FiO_2$ 100-200 mm Hg), and severe ARDS ($PaO_2/FiO_2 < 100$ mm Hg) (see, e.g., The ARDS Definition Task Force, JAMA 307 (23): 2526-2533, 2012).) As will be understood, in the present disclosure, ARDS covers any appropriate ARDS clinical definitions known in the art, including the 2012 Berlin definition. In the present disclosure, individuals at risk for ARDS refer to those who are susceptible to ARDS. Trauma, major bleeding, severe pneumonia, influenza or SARS/COVID-19 infection, and/or sepsis are the risk factors for ARDS. Individuals with these risk factors may be individuals at risk for ARDS.

In some embodiments, a pulmonary inflammatory condition, such as ARDS, ALI or pneumonia, is caused by pulmonary infection. The infection can be a bacterial infection, a viral infection, a fungal infection, a parasitic infection, or an infection by other types of microorganisms. In some embodiments, the bacterial infection is caused by Enterobacteriaceae species, Streptococcus pneumoniae, Staphylococcus aureus, Bacillus anthracis, Haemophilus influenzae, Klebsiella pneumoniae, Escherichia coli, Pseudomonas aeruginosa, Bordetella pertussis, Chlamydia pneumoniae, Legionella spp. or Mycoplasma pneumoniae. In some embodiments, the viral infection is caused by influenza virus, parainfluenza virus, respiratory syncytial virus, human parainfluenza virus, adenovirus, metapneumovirus, severe acute respiratory syndrome (SARS) virus, herpes simplex virus (HSV), varicella zoster virus (VZV), measles virus, rubella virus, cytomegalovirus (CMV), variola virus, dengue virus, rhinovirus, bocavirus, Middle East respiratory syndrome virus, or coronavirus. One of the major host responses to infection is inflammatory response, which leads to pulmonary microvascular leakage and lung injury, sometimes may further lead to respiratory failure. Inhibition of microvascular leakage and/or vascular inflammatory conditions may ameliorate the symptoms of infectious diseases such as viral infections, bacterial infections, etc. In some embodiments, an anti-ANG2 antibody or pharmaceutical composition thereof of the present invention is administered prior to appearance of a clinical symptom of an infectious disease. For example, the anti-ANG2 antibody of the present invention, or pharmaceutical composition thereof, can be administered after an infectious agent (e.g., bacteria or viruses) is detected present in a sample from a patient or after a patient is exposed to an infectious agent (e.g., bacteria or viruses, including in close contact with an asymptomatic and/or symptomatic infected individual). In yet some other embodiments, the anti-ANG2 antibody of the present invention or a pharmaceutical composition thereof is administered after appearance of the first symptom of an infectious disease.

In yet another aspect, the invention relates to a method of preventing or treating acute respiratory distress syndrome by using an antibody of the invention; or a method of ameliorating one or more parameters of acute respiratory distress syndrome by using an antibody of the invention, wherein the method comprises administration to a subject in need thereof a therapeutically effective amount of an antibody or antigen-binding fragment thereof of the invention. In one embodiment, the said parameter is selected from the group consisting of: infiltration intensity of inflammatory cells in lung tissues (including, but not limited to, leukocyte count and differential inflammatory cell count in lung lavage fluid, such as neutrophil count, lymphocyte count and monocyte count), intensity of inflammatory lesions in lung tissues (including but not limited to, lesion intensity grades), arterial oxygen saturation (including, but not limited to, $PO_2$, $PCO_2$, pH, $sO_2$% value), lung images (X-ray or CT images), degree of pulmonary edema, lung weight index, and/or survival rate, or any combination thereof. Preferably, the said parameter comprises the infiltration intensity of inflammatory cells in the lung. Again preferably, the said parameter comprises arterial oxygen saturation. In one embodiment, the said method further comprises, prior to the administration of the anti-ANG2 antibody of the invention, identifying an individual eligible for prevention or treatment of acute respiratory distress syndrome or for ameliorating one or more of the aforementioned parameters of ARDS. In one embodiment, the subject has mild, moderate, or severe hypoxemia. In another embodiment, the subject is at risk of developing hypoxemia, and the anti-ANG2 antibody of the invention is administered prophylactically. In yet another embodiment, the subject has suffered from lung inflammation and presented with at least a slight, mild, moderate or severe inflammatory lesions in lung tissues. In yet another embodiment, the subject is at risk of developing an inflammatory lesion in lung tissues, and the anti-ANG2 antibody of the invention is administered prophylactically.

The intensity of inflammatory lesions in lung tissues can be evaluated in a manner known in the art. For example, histopathological grades can be evaluated on lung tissue sections based on an increasing extent and/or complexity of pathological change, using a 4-point scale (1= minimal, 2= mild; 3= moderate; and 4= severe). The intensity of inflammatory lesions in the lung tissues can be evaluated, for example, by examining the infiltration intensity of inflammatory cells in alveoli/interstitium/bronchi/pleura, the degree of pulmonary edema/fibrin-like material exudation, the degree of thrombus, the degree of degeneration/necrosis in blood vessels/bronchi/alveoli/interstitium, the degree of abscess, the degree of alveolar wall thickening, and the degree of alveolar/pleural hemorrhage.

Oxygen saturation can be evaluated in a manner known in the art. For example, $PO_2$, $PCO_2$, pH, and $sO_2$% values can be measured using collected arterial blood with a blood gas analyzer to detect (e.g., before and after administration of the antibody of the invention) the hypoxemic condition of the subject.

In some embodiments, the methods of the invention include treating one or more symptoms of ARDS, such as mild, moderate, or severe hypoxemia. In some embodiments, a subject with hypoxemia may exhibit alterations in one or more parameters, such as partial pressure of oxygen, oxygenation index ($PaO_2/FiO_2$, i.e., the ratio of arterial partial pressure of oxygen to the fraction of inspired oxygen), CT images of the lung (e.g., localized lung inflammation, increased markings in both lungs, decreased transmittance of both lungs), and levels of one or more inflammatory markers, relative to a healthy subject.

In yet another aspect, the present invention also relates to a use of an anti-ANG2 antibody of the invention as a medicament or in the manufacture of a medicament, wherein the medicament may be used in any one or more of the above methods of the invention. In one embodiment, the anti-ANG2 antibody is used to modulate, especially reduce, the ANG-2-induced vascular endothelial permeability. In one embodiment, the anti-ANG2 antibody is used to prevent or treat vascular leakage and/or vascular inflammatory condition associated with ANG2 activity. In yet another embodiment, the anti-ANG2 antibody is used to prevent or treat a disease or disorder accompanied with vascular leakage and/or vascular inflammatory condition. In a preferable embodiment, the said disease or condition is selected from the group consisting of: pulmonary inflammatory condition (particularly acute inflammatory condition), sepsis, acute lung injury, and acute respiratory distress syndrome.

In any one embodiment of the treatment methods of the invention and the uses of the invention, the anti-ANG2 antibodies of the invention may bind to ANG2 on endothelial cells of a subject and block the binding of ANG2 to its receptor (e.g., TIE2 or an integrin). In one embodiment, the anti-ANG2 antibodies of the invention block the interaction of ANG2 with TIE2. In another embodiment, the anti-ANG2 antibodies of the invention block the interaction of ANG2 with an integrin. In yet some embodiments, the administration of the anti-ANG2 antibodies inhibits the expression of an inflammatory marker on an endothelial cell. In other embodiments, the administration of the anti-ANG2 antibodies inhibits an increase in vascular permeability (caused by e.g., an inflammatory agent such as a cytokine or LPS).

In the treatment methods and the uses of the invention, the antibodies of the invention can be administered alone or in combination with a second therapeutic agent. In a preferable embodiment of the invention, the antibodies of the invention are administered as the sole active agent. In another embodiment, the antibodies of the invention are administered in combination with additional therapeutic agent(s), wherein the said combined administration comprises concomitant administration (in which the two or more therapeutic agents may be contained in the same or different formulations) and separate administration, wherein the antibodies of the invention can be administered before, simultaneously with, or after the administration of the additional therapeutic agent (s). The second therapeutic agent may be, for example, an anti-inflammatory agent, such as a glucocorticoid (e.g., dexamethasone); an anti-infective agent (e.g., antibiotics, antivirals, antifungals, antiparasitics); a bronchodilator (such as β2 agonists); a vasopressor (e.g., isoproterenol, phenylephrine); or a sedative (e.g., propofol, escitalopram, morphine).

In some embodiments, the subject was a mammal. In preferable embodiments, the subject was human. In some embodiments, the subject is identified as an individual in need of treatment by detection of one or more of the following: inflammatory marker level in vascular endothelium, arterial blood gas level, partial pressure of oxygen ($PaO_2$) level, partial pressure of carbon dioxide ($PaCO_2$) level, the intensity of pulmonary inflammatory infiltration and chest imaging.

In any of the methods and uses of the invention described above, the composition of the invention may be administered in lieu of the antibody or antigen-binding portion of the invention. Alternatively, in these methods, in addition to administering the antibody or antigen-binding portion of the invention, the composition of the invention can be further administered.

In some embodiments of the methods or uses of the invention, the antibodies of the invention are administered in the form of pharmaceutical compositions. The pharmaceutical compositions of the present invention can be formulated using suitable carriers, excipients, and other substances that provide suitable transfer, delivery, tolerance, and the like. A multitude of suitable formulations can be found in a pharmacopoeia known to all medicinal chemists: Remington's Pharmaceutical Sciences, Mack Publishing Company, Easton, PA. These formulations include, for example, powders, pastes, ointments, gels, waxes, oils, lipids, lipid (cationic or anionic) containing vesicles (such as LIPOFECTIN™), DNA conjugates, anhydrous absorbent pastes, oil-in-water and water-in-oil emulsions, carbowaxes (polyethylene glycols of various molecular weights) emulsions, semi-solid gels, and semi-solid mixtures containing carbowaxes.

The dosage of antibody administered to a patient according to the methods of the invention may vary depending on the patient's age and body size, symptoms, conditions, route of administration, and the like. Usually, the dosage is calculated based on body weight or body surface area. The frequency and duration of treatment can be adjusted depending on the severity of the disease. The effective dosage and dosing regimen for administering a pharmaceutical composition comprising an anti-ANG2 antibody can be determined empirically. For example, disease progression can be monitored through periodic assessment, and the dosage adjusted accordingly. In addition, inter-species scaling of dosage can be performed using methods well known in the art.

A variety of delivery systems are known in the art and can be used to administer pharmaceutical compositions comprising the anti-ANG2 antibodies of the present invention, such as syringes, pre-filled syringes, auto-injectors, microinfusors, glass vials, encapsulation in liposomes, microparticles, microcapsules, recombinant cells capable of expressing mutant viruses and receptor-mediated endocytosis. Adminstration methods include, but are not limited to, intradermal, intramuscular, intraperitoneal, intravenous, subcutaneous, intranasal, epidural, and oral routes. The composition can be administered by any convenient route of administration, such as by infusion or bolus injection, by absorption through epithelial or mucocutaneous linings (e.g., oral mucosa, rectal and intestinal mucosa, and the like), and can be co-administered with other biologically active agents.

The pharmaceutical compositions of the present invention can be delivered subcutaneously or intravenously using standard needles and syringes. In one embodiment, the syringe is a pre-filled syringe. Such a pre-filled syringe can be a single-dose syringe. In one embodiment, the pharmaceutical composition of the present invention is delivered subcutaneously or intravenously with an auto-injector, wherein the auto-injector may contain a pre-filled cartridge. Additionally, for subcutaneous delivery, pen delivery devices can be readily used to deliver the pharmaceutical compositions of the present invention. Such a pen delivery device can be reusable or disposable. A reusable pen delivery device typically utilizes a replaceable cartridge containing a pharmaceutical composition. Once all of the pharmaceutical composition within the cartridge has been administered and the cartridge is empty, the empty cartridge can be easily discarded and replaced with a new cartridge containing the pharmaceutical composition. The pen delivery device can then be used again. In a disposable pen delivery device, there is no replaceable cartridge. Instead, the disposable pen delivery device is prefilled with a pharmaceutical composition contained in a reservoir within the device. Once the reservoir is emptied of the pharmaceutical composition, the entire device is discarded.

A number of reusable syringes, pens, and autoinjector delivery devices can be used to deliver the pharmaceutical compositions of the present invention subcutaneously. Examples include, but are not limited to, AUTOPEN™ (Owen Mumford, Inc., Woodstock, UK), DISETRONIC™ Pen (Disetronic Medical Systems, Bergdorf, Switzerland), HUMALOG MIX 75/25™ Pen, HUMALOG™ Pen, HUMALIN 70/30™ Pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ Pen (Becton Dickinson, Franklin Lakes, NJ), OPTIPEN™ and OPTIPEN PRO™. Examples of disposable pen delivery devices that can be used to subcutaneously deliver the pharmaceutical compositions of the present invention include, but are not limited to, SOLOSTAR™ pen (Sanofi-Aventis), FLEXPEN™ (Novo Nordisk) and KWIKPEN™ (Eli Lilly), SURECLICK™ auto-injector (Amgen, Thousand Oaks, CA), PENLET™ (Haselmeier, Stuttgart, Germany), EPIPEN (Dey, L.P.), and HUMIRA™ pen (Abbott Labs, Abbott Park IL), to name a few.

In certain cases, the pharmaceutical composition can be delivered in a controlled release system. In one embodiment, a pump can be used. In another embodiment, polymeric materials can be used. In yet another embodiment, a controlled release system can be placed proximate to the target of the composition, so that only a portion of the systemic dose is required.

The injectable formulation may include dosage forms for intravenous, subcutaneous, intradermal and intramuscular injection, drip infusion, and the like. These injectable formulations can be prepared using the methods known in the art. For example, an injectable formulation can be prepared, for example, by dissolving, suspending or emulsifying the antibody or salt thereof described hereinbefore in a sterile aqueous medium or an oily medium conventionally used for injection. As the aqueous medium for injection, there are, for example, physiological saline, isotonic solution containing glucose and other auxiliary agents or the like, which can be used in combination with suitable solubilizers such as alcohols (for example, ethanol), polyols (for example, propylene glycol, polyethylene glycol), nonionic surfactants [for example, polysorbate 80, HCO-50 (polyoxyethylene (50mol) adduct of hydrogenated castor oil)] or the like. As the oily medium, sesame oil, soybean oil or the like can be used, which can be used in combination with a solubilizer such as benzyl benzoate, benzyl alcohol or the like. The injection solution thus prepared can be filled in suitable ampoules.

Advantageously, the aforementioned pharmaceutical compositions for oral or parenteral use can be formulated into dosage forms in a unit dose suited to fit a dose of the active ingredient. Such dosage forms in a unit dose include, for example, tablets, pills, capsules, injections (ampoules), suppositories, and the like.

In any methods or uses of the invention, the antibodies of the invention (and pharmaceutical compositions comprising the same, and any additional therapeutic agent) can be administered via any suitable administration mode, including parenteral administration, intrapulmonary administration, and intranasal administration as well as intralesional administration, if local treatment is required. Parenteral infusions include intramuscular, intravenous, intra-arterial, intraperitoneal or subcutaneous administration. Any suitable route, such as injection, e.g., intravenous or subcutaneous injection, can be adopted, partially depending on whether the administration is a short-time one or a long-term one. The present disclosure encompasses a variety of dosing courses, including, but not limited to, single dosing or multiple dosing at multiple time points, bolus dosing, and pulsed infusions.

For prevention or treatment of a disease, the appropriate dosage of an antibody of the invention, when used alone or in combination with one or more other therapeutic agents, will be selected depending on the type of disease to be treated, the type of antibody, the severity and course of the disease, whether the antibody is administered for prophylactic or therapeutic purpose, previous treatment, the patient's clinical history and response to the antibody treatment, as well as the judgment of the attending physician. The antibody is suitably administered to a patient in a single treatment or over a series of treatments.

In some preferable embodiments of the methods or uses of the invention, the anti-ANG2 antibodies of the invention are administered subcutaneously or intraperitoneally. In yet some preferable embodiments, the anti-ANG2 antibodies of the invention are administered in single or multiple dosages. Preferably, at least one dosage of the anti-ANG2 antibody of the present invention is administered before or immediately after the subject is exposed to an inflammatory agent, such as a bacterium, virus, bacterial toxin, and more preferably before the subject manifests pulmonary infiltration of inflammatory cells. In some embodiments, the antibodies are formulated as a liquid composition. In some embodiments, the antibodies are formulated for inhalation, insufflation, nebulization, or injection administration, or as pharmaceutical compositions for oral, rectal, topical, or intraperitoneal administration. In some embodiments, the injection is intravenous injection. In some preferable embodiments, the anti-ANG2 antibody of the present invention is contained in a pharmaceutical composition, and optionally the pharmaceutical composition is contained in a container selected from the group consisting of a glass vial, a syringe, a prefilled syringe, an autoinjector, and a microinfusion set.

In a further aspect, the present invention relates to a pharmaceutical combination or pharmaceutical composition for use in the above method or use comprising an anti-ANG2 antibody of the present invention and a pharmaceutically acceptable carrier. In some embodiments, the pharmaceutical combination or pharmaceutical composition further comprises a second therapeutic agent.

In a further aspect, the present invention relates to a kit for use in the method or use described above, which comprises a container comprising a pharmaceutical composition of the present invention and the instructions for use. In one embodiment, the kit further comprises a container containing a second therapeutic agent.

## X. Methods and Compositions for Diagnosis and Detection

In a further aspect, the invention relates to a method and kit for detecting ANG-2 in a sample, wherein the method comprises: (a) contacting the sample with an antibody of the invention, or an antigen-binding fragment or immunoconjugate thereof, and (b) detecting the formation of a complex between the said antibody or antigen-binding fragment or immunoconjugate thereof and an ANG-2 protein. In some embodiments, the sample is derived from a cancer patient or a patient with an ocular disease. The said sample can be a tissue biopsy specimen, a tissue section, a body fluid such as blood, plasma or serum. The said detection can be conducted in vitro or in vivo.

The term "detection", when used herein, includes quantitative or qualitative detection, and exemplary detection methods may involve immunohistochemistry, immunocytochemistry, flow cytometry (e.g., FACS), antibody molecule-conjugated magnetic beads, ELISA assays and PCR-techniques (e.g., RT-PCR). In certain embodiments, the ANG-2 to be detected is human ANG-2 or cynomolgus monkey ANG-2.

In one embodiment, an anti-ANG-2 antibody is used to select a subject suitable for treatment with an anti-ANG-2 antibody, e.g., when ANG-2 is a biomarker for selecting the subject. In one embodiment, antibodies of the invention are used to diagnose a vascular-related disease such as a tumor or an infectious disease such as sepsis, for example, to evaluate (e.g., to monitor) the treatment or progression, the diagnosis and/or staging of a disease described herein in a subject.

In certain embodiments, labeled anti-ANG-2 antibodies are provided. Labels include, but are not limited to, labels or moieties that are detected directly (such as fluorescent labels, chromophore labels, electron dense labels, chemiluminescent labels, and radiolabels), and moieties such as enzymes or ligands that are detected indirectly, for example, by enzymatic reactions or molecular interactions. Exemplary labels include, but are not limited to, radioisotopes 32P, 14C, 125I, 3H, and 131I, fluorophores such as rare earth chelates or luciferin and derivatives thereof, rhodamine and derivatives thereof, dansyl, umbelliferone, luciferases, e.g., firefly luciferase and bacterial luciferase (U.S. Patent. No. 4,737,456), luciferin, 2,3-dihydrophthalazine-1,4-dione, horseradish peroxidase (HR), alkaline phosphatase, β-galactosidase, glucoamylase, lytic enzyme, sugar oxidases, e.g., glucose oxidase, galactose oxidase, and glucose-6-phosphate dehydrogenase, heterocyclic oxidases such as uricase and xanthine oxidase, and enzymes that oxidize dye precursors using hydrogen peroxide such as HR, lactoperoxidase, or microperoxidase, biotin/avidin, spin labels, phage labels, stable free radicals, and the like.

The following Examples are described to aid the understanding of the invention. The Examples are not intended and shall not be construed in any way to limit the scope of protection of the invention.

## Examples

## Example 1. Preparation of Hybridoma Cells

In this study, mice were immunized with human angiopoietin 2 (Ang2) protein, and then the spleen cells of the mice were fused with myeloma cells to obtain hybridoma cells that express positive antibodies.

Hybridoma fusion:

| Laboratory animals and immunization methods | |
| --- | --- |
| Mouse type | Balb/c (Beijing Vital River Laboratory Animal Technology Co., Ltd.) |
| Immunizing antigen | Human hAng2 protein (Sino Biological, Cat. No. 10691-H08H) |
| Immunization method | 50 μg/animal, subcutaneous (SC) injection, 50 μl per site, 2 sites |
| Number of immunizations | 5 |

Cell fusion:

The spleen of the immunized animals was excised and the spleen cell suspension was prepared. After filtration and lysis of erythrocytes, splenocytes were suspended in 20 ml of basal medium and counted.

| Name | Composition | Preparation method |
|---|---|---|
| Basal medium | FBS(Hyclone,SH30084.03) | 10% |
| | GlutaMAX™ Supplement(Gibco, 35050-079) | 1× |
| | RPMI-1640(Hyclone, SH30809.01) | 90% |

Mouse myeloma SP2/0 cells (ATCC, CRL-1581) were resuspended in 20 ml of basal medium and counted. SP2/0 and spleen cells were mixed in a ratio of 1: 2 to 1: 1 and centrifuged at 1000 rpm for 6 min. After removal of the supernatant, the mixed cells were resuspended in 10 ml of cytofusion buffer (BTXpress, 47-0001). Another 15 ml of cytofusion buffer was added, and after centrifugation at 1000 rpm for 5 min, the supernatant was discarded. After repeating the above steps, the cells were resuspended with an appropriate amount of cytofusion buffer and the mixed cell density was adjusted to $1 \times 10^7$ cells/ml. The parameters of the electrofusion instrument were set as follows. Electrofusion was performed by adding 2 ml of the cell suspension to each electrofusion dish.

| | |
|---|---|
| Condition | Mouse (SP2/0-ECF-F) |
| Alignment: | 60v, 30 sec |
| Membrane breaking: | 1500V, 30μs, 3X |
| Post-fusion pulse: | 60V, 3 sec |

Post-electrofusion screening:

The cells were allowed to stand for 5 min at room temperature in the electrofusion dish. The cells were transferred into centrifuge tubes and diluted to $1-2 \times 10^4$ cells/ml with selection medium (see table below). A 100 μl of cell suspension was added to each well of a 96-well plate. The selection medium was replaced on Day 7 post-fusion. The screening was performed after 10 days of culture (or longer, depending on cell growth status). Hybridoma cells expressing specific anti-hAng2 antibodies were selected by Elisa assay.

| Name | Composition | Preparation method |
|---|---|---|
| Selection medium | RPMI-1640(Hyclone, SH30809.01) | 80% |
| | FBS(Hyclone,SH30084.03) | 20% |
| | HAT medium(Gibco,21060-017) | 1× |
| | GlutaMAX™ Supplement(Gibco Gibco, 35050-079) | 1× |

The antibodies expressed by the selected hybridoma cells were sequenced by PCR to determine the variable region genes of the antibodies, and the chimeric antibodies were screened, humanized, and affinity matured to obtain the antibody sequences of the invention.

The amino acid sequences of the CDR regions, light chain variable regions, heavy chain variable regions, light chain and heavy chains of six exemplary antibodies of the invention (chimeric antibodies 81H10, 86E9, 107H7; humanized antibody: hz81H10.4; affinity matured antibodies Amhz81H10.4.30, Amhz81H10.4.49), and the corresponding nucleotide sequences are shown in Table D below.

Table D: The SEQ ID NOs of the revelant sequences of each of the antibodies involved in the present Invention

| Antibody name | HCDR1 | HCDR2 | HCDR3 | VH | HC | VH DNA | LCDR1 | LCDR2 | LCDR3 | VL | L C | VL DNA |
|---|---|---|---|---|---|---|---|---|---|---|---|---|
| 81H10 | 1 | 2 | 3 | 19 | 31 | 43 | 4 | 5 | 6 | 20 | 32 | 44 |
| 86E9 | 1 | 2 | 3 | 21 | 33 | 45 | 4 | 5 | 6 | 22 | 34 | 46 |
| 107H7 | 1 | 2 | 3 | 23 | 35 | 47 | 4 | 5 | 6 | 24 | 36 | 48 |
| Hz81H10.4 | 1 | 2 | 3 | 25 | 37 | - | 4 | 5 | 6 | 26 | 38 | - |
| Amhz81H10.4.30 | 7 | 8 | 9 | 27 | 39 | - | 10 | 11 | 12 | 28 | 40 | - |
| Amhz81H10.4.49 | 13 | 14 | 15 | 29 | 41 | - | 16 | 17 | 18 | 30 | 42 | - |

(continued)

| Antibody name | HC DR1 | HCD R2 | HCD R3 | VH | HC | VH DNA | LCD R1 | LCD R2 | LCD R3 | VL | L C | VL DNA |
|---|---|---|---|---|---|---|---|---|---|---|---|---|
| Positive control antibody | | | | | | 49 | | | | | | 50 |
| Note: HC is formed by fusing the VH sequence to the IgG1 portion of SEQ ID NO: 52; LC is formed by fusing the VL sequence to the Cκ chain of SEQ ID NO: 53. | | | | | | | | | | | | |

## Example 2. Production and Purification of Antibodies

(1) Expression in GS-CHO cells and purification:

The antibody-expressing CHO-S cell lines were generated using the GS Xceed™ Gene Expression System kit (Lonza) according to the manufacturer's instructions. The DNA sequences for the heavy and light chains of an antibody molecule were first inserted into the same pCHO1.0 plasmid, where the heavy chain was positioned upstream of the light chain. Then the constructed pCHO1.0 plasmid was transferred into the CHO cell line by chemical transfection and electrotransfection method. At 48 hours after transfection, the ForteBio method was used to detect the antibody production so as to determine the transfection efficiency. After two rounds of screening under selection pressure with 10 μg/ml and 50 μg/ml puromycin, the cell pool with high antibody expression was obtained. After expanding the cell pool to express the antibody in large amounts, the cell supernatant was collected and purified with Protein A to an antibody purity > 95%.

(2) Expression in HEK293 cells and purification:

For a transient expression of an antibody in HEK293 cells, conventional plasmids such as pcDNA3.1 can be used. The cDNAs encoding the heavy and light chains of the antibody were first cloned into the pcDNA3.1 vector. The vectors carrying the heavy chain and light chain of the antibody molecule were transferred into HEK293 cells using a chemical transfection method. The chemical tranfection agent used was PEI (purchased from Polysciences) , and the cultured 293HEK (Invitrogen) cells were transiently transfected according to the procedure provided by the manufacturer. After transfection, the medium was discarded and the cells were diluted to $4 \times 10^6$/ml with fresh EXPI293 medium (Gibco). Cells were incubated in 5% $CO_2$ at 37°C for 7 days, with fresh media constantly feeding every 48 h. After 7 days, centrifugation was performed at 1300 rpm for 20 min. The supernatant was harvested and purified with Protein A to an antibody purity > 95%.

## Example 3. Determination of Binding Kinetics of Antibodies of the Invention to Antigens

Example 3.1

The equilibrium dissociation constants ($K_D$) of the above exemplary antibodies of the present invention binding to human Ang2 (h Ang2), cynomolgus monkey Ang2 (cAng2), murine Ang2 (mAng2), and human Ang1 (hAng1) were determined using the biological optical interferometry (ForteBio) assay.
The ForteBio affinity assay was performed according to the existing method (Estep, P et al., High throughput solution-based measurement of antibody-antigen affinity and epitope binning. MAbs, 2013.5 (2): p. 270-8). Briefly, the sensor was equilibrated in the assay buffer off-line for 30 min, followed by on-line detection for 60s to establish the baseline. Then the purified antibodies obtained as described above were loaded on-line onto an AHQ sensor (ForteBio) for ForteBio affinity assay. The sensors with loaded antibodies were exposed to 100 nM of hAng2, cAng2, mAng2, and hAng1 antigens for 5 min, and then were transferred into the assay buffer for dissociation for 5 min to determine the dissociation rate. Kinetic analysis was performed using a 1: 1 binding model.
The affinities of 81H10, 86E9, 107H7, hz81H10.4, Amhz81H10.4. 30, Amhz81H10.4. 49 and the control antibody Nesvacumab (Regeneron) in experiments performed as described for the above assays are shown in Table 1A.

Table 1A: Affinities of Anti-Ang2 Antibodies

| Antibody | Human Ang2 | Cynomolgus monkey Ang2 C-terminal domain (Lys274-Phe495) | Murine Ang2 C-terminal domain (Lys275-Phe496) | Human Ang1 |
|---|---|---|---|---|
| 81H10 | 6.92E-09 | 4.43E-08 | N.B | N.B |

(continued)

| Antibody | Human Ang2 | Cynomolgus monkey Ang2 C-terminal domain (Lys274-Phe495) | Murine Ang2 C-terminal domain (Lys275-Phe496) | Human Ang1 |
|---|---|---|---|---|
| 86E9 | 7.63E-09 | 4.62E-08 | N.B | N.B |
| 107H7 | 7.75E-09 | 2.52E-08 | N.B | N.B |
| hz81H10.4 | 1.25E-09 | 8.83E-08 | N.B | N.B |
| Amhz81H10.4.30 | 2.32E-08 | N.D | N.B | N.B |
| Amhz81H10.4.49 | 6.468E-10 | N.D | N.B | N.B |
| Nesvacumab | 1.42E-09 | 1.04E-09 | 1.02E-09 | N.B |
| *N.B. indicates no binding; N.D. indicates not determined. | | | | |

It can be seen from the results of the above table that all the above exemplary antibodies of the present invention exhibited a very high affinity, of which hz81H10.4 and Am81H10.4.49 had a higher affinity than the control antibody Nesvacumab.

Example 3.2

Hz81H10.4 was selected, and the binding abilities of HZ81H10.4 to ang2 proteins of different species, including human ANG2, rhesus ANG2, rat ANG2, mouse ANG2 and rabbit ANG2, were further investigated using Biacore instrument T200.

Determination of binding affinity of antibody to ANG2: Channel 2 of Protein G chip was selected to capture the antibody, and flow rate was 10 $\mu$L/min in the assay. The antibody was diluted to 1 $\mu$g/mL and was captured onto channel 2 for 30 s, and channel 1 was the blank control channel. The gradient-diluted Human ANG2, Rhesus ANG2, Rabbit ANG2, Rat ANG2 and Mouse ANG2 were sequentially injected into channels 1 and 2 of the chip, from low to high concentrations (0-32 nM), at a flow rate of 30 $\mu$L/min, with one concentration per cycle, and with 180s association time and 1200s dissociation time at 25 °C. Finally, the chip was regenerated using Glycine pH 1.5 at 30 $\mu$L/min for 30 s. The affinity assay was repeated three times.

Table 1B: Results of Anti-Ang2 Antibody Binding to Ang2 Proteins from Different Species

| immoblized antibody | Analyte | Experiment seria | $K_D$(M) | kon(1/Ms) | kdis(1/s) | $K_D$ Mean (M) $\pm$ SD |
|---|---|---|---|---|---|---|
| HZ81H10.4 | Human ANG2 | 1 | 6.94E-11 | 2.50E+05 | 1.74E-05 | 4.57E-11 $\pm$ 2.06E-11 |
| | | 2 | 3.19E-11 | 2.59E+05 | 8.25E-06 | |
| | | 3 | 3.57E-11 | 2.93E+05 | 1.05E-05 | |
| HZ81H10.4 | Rhesus ANG2 | 1 | 1.83E-10 | 2.66E+05 | 4.87E-05 | 1.46E-10 $\pm$ 3.18E-11 |
| | | 2 | 1.26E-10 | 2.91E+05 | 3.67E-05 | |
| | | 3 | 1.30E-10 | 3.11E+05 | 4.03E-05 | |
| HZ81H10.4 | Rabbit ANG2 | 1 | 5.18E-10 | 2.94E+05 | 1.53E-04 | 4.80E-10 $\pm$ 3.61E-11 |
| | | 2 | 4.77E-10 | 3.18E+05 | 1.52E-04 | |
| | | 3 | 4.46E-10 | 3.22E+05 | 1.44E-04 | |
| HZ81H10.4 | Rat ANG2 | 1 | | | | |
| | | 2 | No binding | | | |
| | | 3 | | | | |
| HZ81H10.4 | Mouse ANG2 | 1 | No binding | | | |

### Example 4. Binding of Antibodies to Human Ang2 Protein

ELISA Assay for binding of anti-hAng2 antibody of the present invention to human Ang2 protein The binding of the respective parental murine hybridoma antibodies of the above exemplary chimeric antibodies 81H10, 86E9, and 107H7 to human hAng2 was measured in an ELISA-based assay.

Human ang2 protein (Sino Biological, Cat No.: 10691-H08H) was diluted to 0.25 $\mu$g/ml with PBS, and was coated onto a microtiter plate at 100 $\mu$l/well at 4°C overnight. After three washes with PBST, the plate was blocked with 5% BSA. Gradient diluted antibodies (maximum concentration 20 nM, 1:2 serial dilution) were added to the microtiter plate and incubated for 2 h. After three washes with PBST, a 1: 10000 dilution of anti-mouse IgG, HRP-conjugated antibody (Cell Signaling, Cat. No. 7076P2) was added, and after incubation for 60 min, the plate was washed six times with PBST. Chromogenic reaction was performed with 100 ul TMB and terminated with 100 ul 2N sulfuric acid. The reading of $OD_{450}$ - $OD_{620}$ was taken by microplate reader. The concentration-dependent curve was fitted with GraphPad based on the $OD_{450}$- $OD_{620}$.

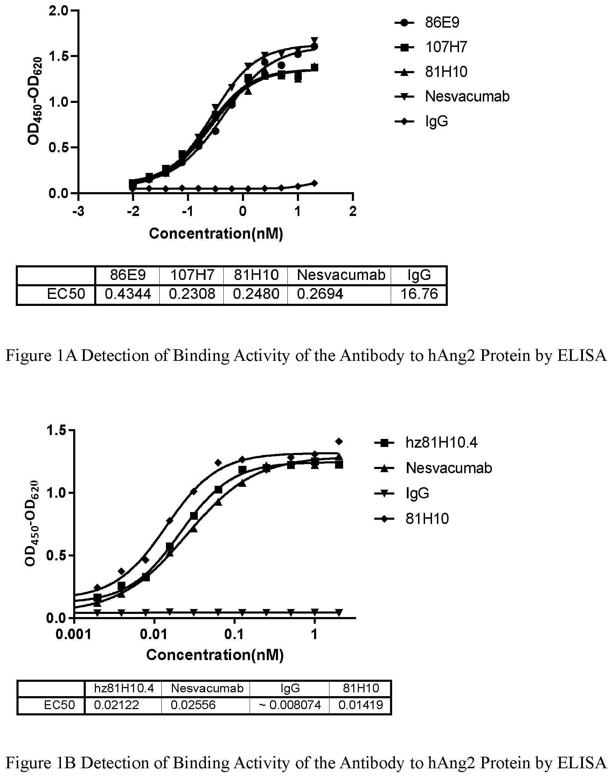

The results of the respective murine hybridoma antibodies of antibodies 81H10, 86E9 and 107H7 are shown in Figure 1A.

The binding of 81H10 and hz81H10.4 of the present invention to human biotin Ang2 protein was measured in an SA-biotin Ang2-based ELISA assay.

SA protein (Thermo Fisher) was diluted to 1 $\mu$g/ml with PBS, and was coated onto a microtiter plate at 100 $\mu$l/well at 4°C overnight. After three washes with PBST, the plate was blocked with 5% BSA for 1 h. Biotin Ang2 (R&D, Cat. No. BT623B/CF) was incubated at a concentration of 0.5 $\mu$g/ml for 1 h. Gradient diluted antibodies (maximum concentration 20 nM, 1: 3 serial dilution) were added to the microtiter plate and incubated for 2 h, and the plate was washed three times with PBST. Anti-human IgG, HRP-conjugated antibody (Bethyl, Cat. No. A80-104P) diluted at 1:10000 was added and incubated for 60 min. Then the plate was washed for 6 times with PBST. Chromogenic reaction was performed with 100 ul TMB and terminated with 100 ul 2N sulfuric acid. The reading of $OD_{450}$ - $OD_{620}$ was taken by microplate reader. The concentration-dependent curve was fitted with GraphPad based on the $OD_{450}$- $OD_{620}$.

The results of antibodies 81H10 and hz81H10.4 are shown in Figure1B.

In the experiment performed as described for the above asay, the $EC_{50}$ values of 81H10, 86E9, 107H7 and hz81H10.4 binding to hAng2 were comparable to that of the control antibody Nesvacumab (see Figure 1A and Figure 1B).

### Example 5. Antibody Blocks Binding of Human Ang2 Protein to Tie2 Protein

The abilities of 81H10, 86E9, 107H7, hz81H10.4, Amhz81H10.4.30, Amhz81H10.4.49, and the control antibodies Nesvacumab and faricimab to block the binding of human Ang2 to hTie2 were determined by ELISA. Faricimab is a bispecific antibody drug undergoing a clinical trial and developed by Roche, which targets both angiopoietin-2 (Ang-2) and VEGF-A.

The hTie2 protein (Sino Biological, Cat. No.: 10700-H03H) was resuspended and dissolved in PBS to a concentration of 2 $\mu$g/ml, and then coated onto a microtiter plate overnight. Then the plate was blocked using 5% BSA for 1 h. The biotinylated antigen, recombinant biotinylated hAngiopoietin-2 protein (R&D, Cat. No.: BT623B/CF), was diluted to 600 ng/ml, and was added into the microtiter plate at 50 $\mu$l/well. The antibodies (81H10, 86E9, 107H7, hz81H10.4, Amhz81H10.4.30 and Amhz81H10.4.49) prepared as described above were gradiently diluted with a total of 8 or 12 dilution points starting from the highest concentration, and then added to the microtiter plate at 50 $\mu$l/well, and incubated in PBS on ice for 30 min. Then, 50 ul of antigen solution was added to the microtiter plate, in which the final antigen concentration was 300 ng/ml. The plate containing the antigen-antibody mixture was incubated for 90 min. After three washes with PBS, the supernatant was discarded, and Avidin-HRP (Invitrogen) diluted at 1: 10000 was added at 100 $\mu$l/well, then incubated at room temperature for 30 min. PBS was used to wash the plate six times. Then TMB chromogen solution (solarbio) was added at 100 ul/ well to allow for chromogenic reaction for 1 min, then the reaction was terminated with the addition of stop solution (solarbio) at 100 ul/well.

The absorbance values of $OD_{450}$ and $OD_{620}$ were read using a microplate reader.

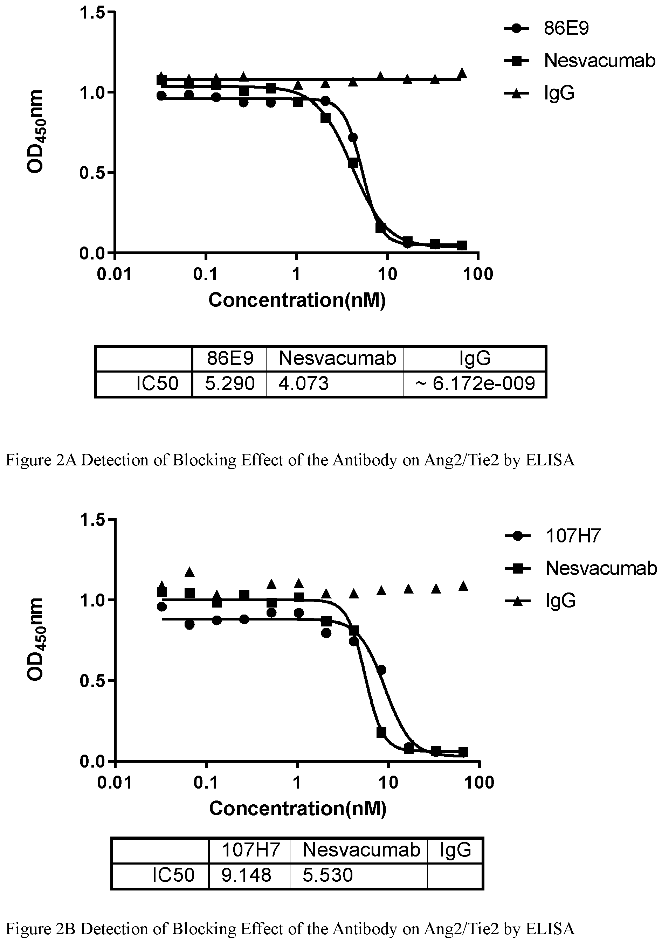

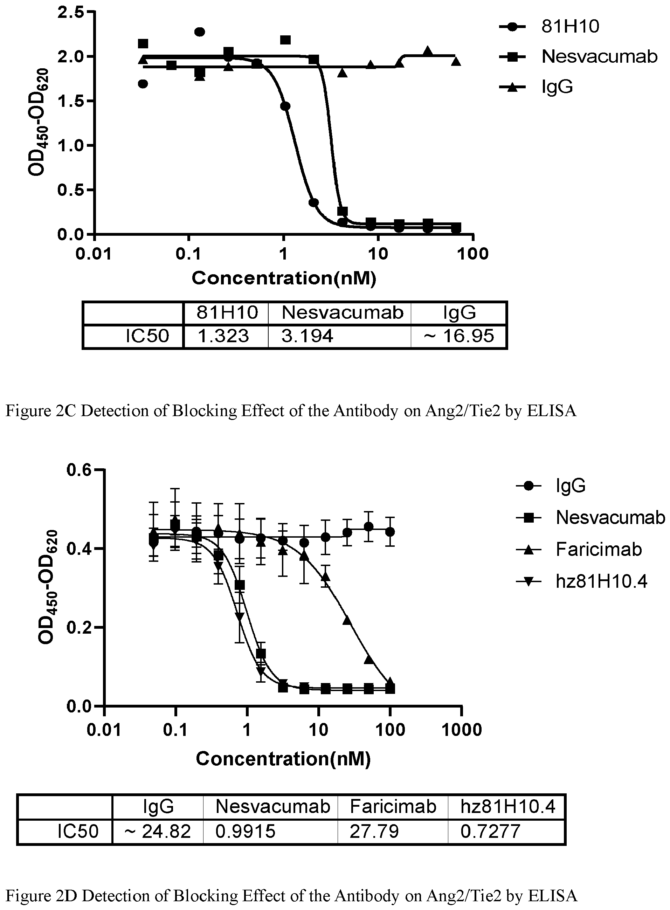

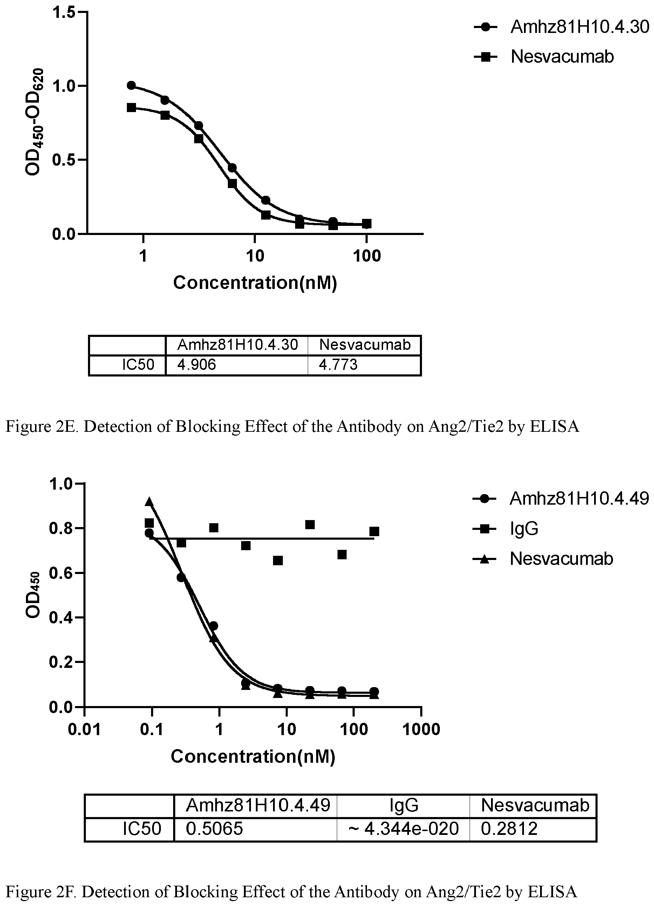

The results showed that 81H10, 86E9, 107H7, hz81H10.4, Amhz81H10.4.30, Amhz81H10.4.49 and control antibody Nesvacumab achieved complete blocking effect, and hz81H10.4 was superior to control antibody Nesvacumab and faricimab in terms of IC50 value (See Figure 2 (A)-(F)).

### Example 6. Antibody Blocks the Binding of Human Ang2-Fc Protein to 293 Cells Overexpressing Tie2

The ability of hz81H10.4 and the control antibody Nesvacumab to block the binding of human Ang2-hFc to Tie2 on the cell surface was detected by flow cytometry (FACS).

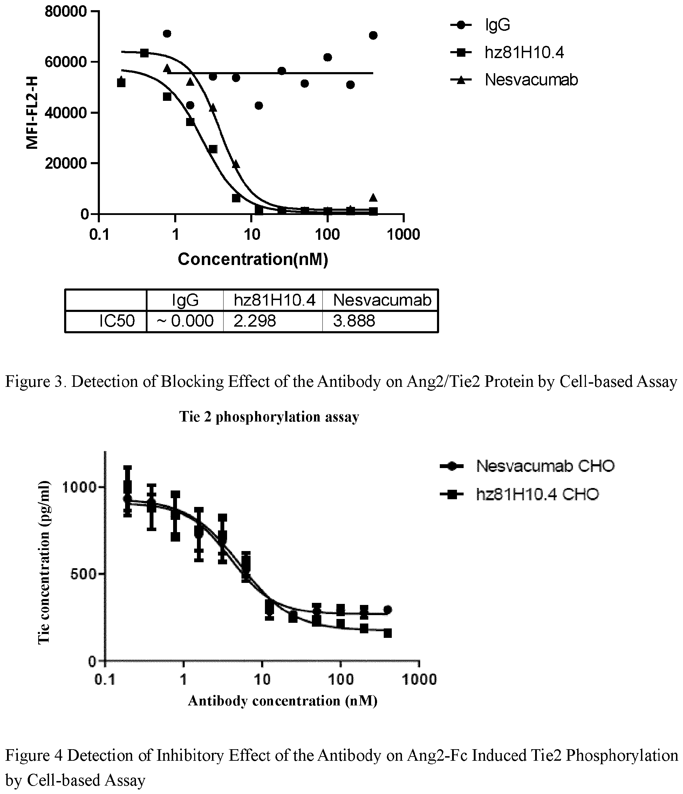

Human Tie2-overexpressing expi-293 cells (293-tie2 cells) were generated by transfection with the pCHO1.0 vector (Invitrogen) carrying the human Tie2 gene cloned into the MCS. The antigen hAng2-Fc protein (Sino Biological, Cat No.: 10691-H02H) was diluted to 4 $\mu$g/ml, and added to the plate wells at 50 $\mu$l/well. Antibodies (hz81H10.4 and control antibody Nesvacumab) prepared as described above were diluted at 2-fold with a total of 12 dilution points starting from the highest concentration of 800 nM, and added to the plate wells at 50 $\mu$l/well, and incubated in PBS on ice for 30 min, where the final concentration of the antigen was 2 $\mu$g/ml and the highest concentration of antibody was 400 nM. The 293-tie2 cells were adjusted to $2 \times 10^5$ cells/well and added to the plate wells at 100 $\mu$l/well. The cells were centrifuged at 300 g for 5 min, and after the supernatant was discarded, the cells were resuspended in the antigen-antibody mixture. The cells were incubated on ice for 30 min, and added with PBS at 100 $\mu$l/well, centrifuged at 300 g for 5 min, and washed with PBS once. A 100 $\mu$l of goat anti-human IgG-PE (Southern Biotech, Cat. No. 2040-09) diluted at 1: 200 was added to each well and placed in an ice bath for 20 min. PBS was added at 100 $\mu$l/well, and the cells were centrifuged at 300 g for 5 min, and washed once with PBS. The cells were resuspended in 100 $\mu$l PBS and the fluorescence signals of the cells were detected by a flow cytometer (BD Biosciences). Concentration-dependent curves were fitted with GraphPad according to their MFI. As shown in Figure 3, hz81H10.4 had better IC50 values compared to the control antibody Nesvacumab.

**Example 7. Antibody Blocks Human Ang2-Fc Protein-Induced Tie2 Phosphorylation in 293-tie2 Cells**

The inhibitory effect of antibody HZ81H10.4 versus the positive control antibody Nesvacumab (Regeneron, antibody HC/LC sequence SEQ ID NO: 11/12) on hAng2-Fc-induced tie2 phosphorylation was detected and compared.

Briefly, the expi293 cells overexpressing tie2, i.e., 293-tie2 cells, were incubated with the antibody and recombinant hAng2-Fc protein. The inhibitory effect of the antibody on the phosphorylation of Tie2 induced by hAng2-fc was reflected by the amount of phosphorylated Tie2 detected in the system.

Tie2-overexpressing 293-tie2 cells were pipetted and diluted to $2 \times 10^6$ cell/ml, and were added at 100 $\mu$l/well into a 96-well plate, then centrifuged at 400 g for 5 min, and the supernatant was removed. Expi293 medium (Gibco) was used to prepare the experimental medium. The test antibody was added to the plate wells (the highest final concentration at 60 $\mu$g/ml, and 1: 2 serial dilution). Then hAng2-Fc was added at a final concentration of 2.5 $\mu$g/ml. The cells were resuspended with 100 $\mu$l/well of the experimental medium and incubated at 37°C for 15 min. After centrifugation to remove the medium, 100 $\mu$l of NP-40 lysis buffer containing 1% protease and phosphatase inhibitors was added, and the lysate was placed on ice for 30 min. After centrifugation at 2000 g, the protein supernatant was harvested and stored in a freezer at -80°C.

The pTie2 concentration was determined according to the protocol of the phospho-tie2 ELISA kit (R&D, Cat. No. DYC2720E). Capture antibody was coated onto a microtiter plate at a concentration of 4 $\mu$g/ml overnight at 4°C. The plate was washed three times with PBST and blocked with 5% BSA for 1 h. A 100 $\mu$l of the test sample (which can be diluted 2-3 fold) or the control pTie2 (used to prepare the standard curve) was added and incubated at room temperature for 2 h. After three washes with PBST, 100 $\mu$l of HRP-conjugated anti-pTyr antibody was added and incubated at room temperature for 2 h. After six washing with PBST, 100 $\mu$l of TMB was added for chromogenic reaction and 100 $\mu$l of stop buffer was added after 15 minutes to stop the reaction. The $OD_{450}$-$OD_{620}$ was determined. The experimental results are shown in Figure 4.

The results showed that the antibodies of the present invention effectively inhibited hAng2-Fc-induced 293-Tie2 phosphorylation in vitro, and the inhibitory activity was comparable to that of the positive control antibody Nesvacumab.

**Example 8. Antibody Blocks Cell Adhesion Mediated by Human Ang2 Protein and Integrin**

The U87 cells (human glioma cells), which express multiple RGD integrins, were used to investigate the inhibitory effect of an antibody on hAng2/ integrin-mediated cell adhesion by measuring the number of U87 cells adhering to a microplate in the presence of the antibody by Celltiter (Promega). The results were then compared with that of the positive control antibody Nesvacumab.

Briefly, a microtiter plate was disinfected with UV irradiation, and recombinant hAng2 (Sino Biological, Cat No. 10691-H08H) was diluted to 2.5 $\mu$g/ml, and coated onto the microtiter plate at 4°C overnight. After three washes with PBS, the plate was blocked with 10% FBS in DMEM for 1 h. After discarding the blocking solution, the antibody of the present invention was added. An IgG isotype control antibody was used as a negative control, and Nesvacumab as a positive control. The highest concentration of the antibody was 100 $\mu$g/ml, and 1: 4 serial dilution was performed with a total of six concentration points. The antibody was added at 100 $\mu$l/well and incubated for 90 min; then the plate was washed three times with PBS. U87 MG cells were diluted to a concentration of $1 \times 10^5$ cells/ml in serum-free DMEM and 100 $\mu$l was added to each well. Then the cells were incubated in a $CO_2$ incubator at 37°C for 30 min. The cells were washed three times with 100 $\mu$l/well of PBS and the supernatant was discarded carefully. Then CellTiter-Glo (promega, Cat. No. # G7572) was added at 100 $\mu$l/well and shaken for 2 min. The absorbance was determined on a microplate reader according to the manufacturer's instructions for uses.

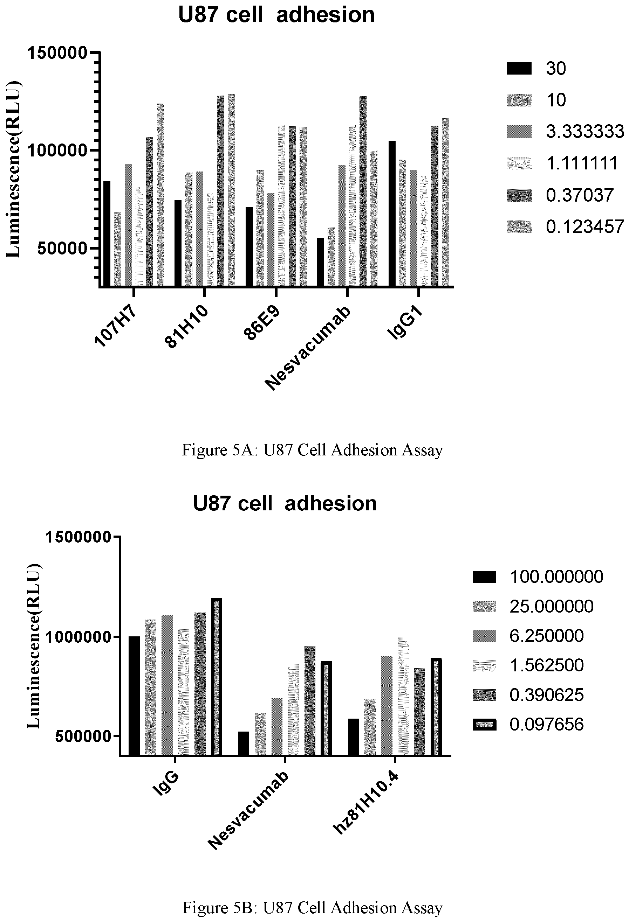

The results are shown in Figures 5A and 5B. Both antibody hz81H10.4 and Nesvacumab can effectively block the interaction of hAng2 with integrins and with comparable potency.

## Example 9. Non-specific Binding of Antibodies to Cells of Different Origins

The binding of an antibody to cells of different retinal origins was examined to identify the non-specific binding properties of the antibody to the cells.

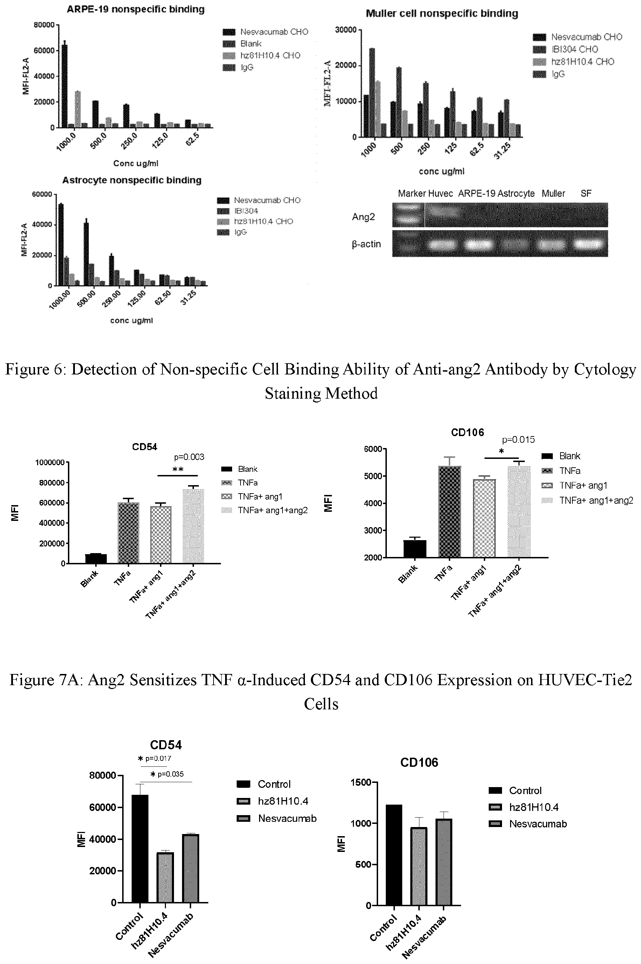

Total RNAs were extracted from $1 \times 10^6$/ml ARPE-19, Müller, astrocyte, HUVEC and SP2-0 cells (all available from ATCC), and the absence of Ang2 transcription in ARPE-19, Müller and astrocyte cells was verified by RT-PCR. The results are shown in the electropherogram of Figure 6. The upper electropherogram shows the Ang2 PCR band generated from amplification, and there was no transcription of Ang2 gene in ARPE-19, Müller and astrocytes except for the positive control cell HUVEC. The lower electropherogram shows the amplified bands of the control gene β-actin.

ARPE-19, Müller or astrocyte cells at the density of $1 \times 10^6$/ml was pipetted and added to a 96-well U-bottom plate at 100 μl/well and then centrifuged to remove the medium. Gradiently diluted Ang2 antibodies (the highest concentration 1 mg/ml, in a 1:2 serial dilution) were prepared. Gradiently diluted antibodies were added to the cells to be tested and incubated at 4°C for 1 h. PBS was added(200 μl/well), centrifugation was performed at 400 g for 5 min, and the supernatant was discarded; fluorescent secondary antibody PE-anti human IgG Fc (Southern Biotech) diluted at 1:200 was added into each well and incubated at 4°C for 30 min; then PBS was added at 200 μl/well, the cells were centrifuged at 400 g for 5 min, and after the supernatant was discarded, PBS was added at 100 μl/well to resuspend the cells, and the MFI value was measured by flow cytometry.

The experimental results are shown in Figure 6. At high concentrations, the antibody hz81H10.4 showed weaker nonspecific binding to cells not expressing Ang2, as compared to the control antibody Nesvacumab. These results indicates that the antibody hz81H10.4 has a higher selectivity, which is advantageous for the *in vivo* drug application of the antibody, especially for topical application at high concentrations, such as intravitreal injection at high concentrations for the treatment of eye diseases.

## Example 10. Assay for Inhibitory Effect of Anti-Ang2 Antibody on ICAM-1/VCAM-1 Expression on HUVEC Cells

Under physiological conditions, Ang1 is produced by vascular pericytes and Ang2 is produced by vascular endothelial cells. In addition, Ang1 and Ang2 competitively bind to TIE2 receptor expressed on endothelial cells, which triggers downstream events. In this Example, the effect of anti-ANG2 antibody on the expression of inflammatory markers in endothelial cells in the presence of both ANG1 and ANG2 was investigated.

Briefly, HUVEC cells were transfected by lentivirus to obtain the Tie2 overexpressing HUVEC-Tie2 cells. Huvec-Tie2 cells were seeded in a 24-well or 96-well plate at a concentration of $2 \times 10^5$ cells/ml.

In the first group of experiments, the medium was replaced with the following conditioned media after the Huvec-Tie2 cells were incubated for 24 h:

Blank group: EGM medium (endothelial cell culture medium, LONZA),

TNF-α group: EGM medium + 100 pg/ml TNF-α,

TNF-α + ang1 group: EGM medium + 100 pg/ml TNF-α + 50 ng/ml Ang1,

TNF-α + ang1+ang2 group: EGM medium + 100 pg/ml TNF-α + 50 ng/ml Ang1+500 ng/ml Ang2, which were used to detect the effect of Ang2 on TNF-α induced expression of CD54 and CD106 on HUVEC-Tie2 cells.

In the second group of experiments, after Huvec-Tie2 cells were incubated for 24 h, the medium was replaced with the following conditioned medium to further investigate the effect of ang2 on the expression of CD54 and CD106 in the presence of anti-Ang2 antibody:

Control group: EGM medium + 100 pg/ml TNF-α + 50 ng/ml Ang1+500 ng/ml Ang2,

Hz81H10.4 group: EGM medium + 100 pg/ml TNF-α + 50 ng/ml Ang1+500 ng/ml Ang2 + 15 μg/ml HZ81H10.4,

Nesvacumab group: EGM medium + 100 pg/ml TNF-α + 50 ng/ml Ang1+500 ng/ml Ang2 + 15 μg/ml Nesvacumab.

After 24 h of culturing in a conditioned medium, the cells were washed three times with PBS, digested with 100 μl of trypsin, and stained with the anti-CD54 antibody (Biolegend Cat No.: 353108) and anti-CD106 antibody (Biolegend Cat. No.: 305810) (diluted at 1:500, at 500 μl/well). After staining and washing, the expression of CD54 and CD106 on the cells was determined by flow cytometry.

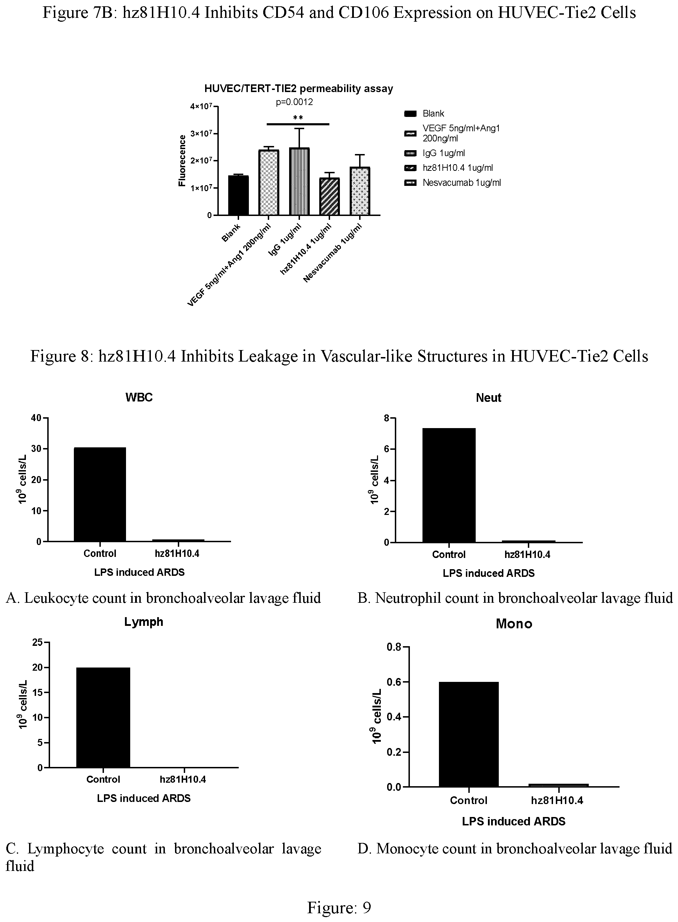

The experimental results (Figure 7) showed that hz81H10.4 can inhibit the expression of inflammatory markers CD54 (ICAM-1) and CD106 (VCAM-1) in vascular endothelial cells by blocking Ang2 binding to the relevant receptor.

As shown in Figure 7A, Ang2 can sensitize TNF-α induced expression of CD54 and CD106 on HUVEC cells in the presence of ANG1. As shown in Figure 7B, HZ81H10.4 significantly inhibits this function of Ang2, with a potency comparable to or better than the positive control antibody Nesvacumab (Figure 7B). ICAM-1 and VCAM-1 are annotated on uniport under GO Biological Process GO: 0002438 (Acute Inflammatory Response to Antigenic Stimuli) and are involved in the migration of leukocytes across vascular endothelium. The inhibitory effect of the antibody on ICAM-1/VCAM-1 expression suggests that the antibody may be beneficial for preventing leukocyte migration across vascular endothelium and leukocyte infiltration in inflammatory tissues during acute inflammatory response.

**Example 11. Permeability Assay in HUVEC-Tie2 Cells**

Ang2/Tie2 and Ang2/integrin signaling pathways are involved in the increased vascular endothelial permeability and vascular leakage under pathological conditions. hz81H10.4 molecule blocks the functions of Ang2/Tie2 and Ang2/integrin. Therefore, the permeability assay in HUVEC-Tie2 cells was used to further identify the effect of the antibody on vascular leakage.

HUVEC cells were transfected by lentivirus to obtain the Tie2 overexpressing HUVEC-Tie2 cells. A mini-well 96-well dish with insert was used, with 300 μl of culture medium in the lower chamber. Adherent HUVEC-Tie2 cells (which secretes their own Ang2) in culture were digested and resuspended to $1 \times 10^7$ cell/ml in culture medium, and 100 μl/well was plated in the upper chamber of the dish. The medium in the lower chamber was replaced every 24 h, and the medium in the upper chamber was replaced with the following experimental medium 48 hours later:

Blank group: EGM-2 medium (endothelial cell culture medium, LONZA),

Control group: EGM-2 medium+5 ng/ml VEGF+200 ng/ml Ang1,

IgG isotype control group: EGM-2 medium+5 ng/ml VEGF+200 ng/ml Ang1+1 μg/ml IgG isotype control antibody,

Hz81H10.4 group: EGM-2 medium+5 ng/ml VEGF+200 ng/ml Ang1+1 μg/ml hz81H10.4,

Nesvacumab group: EGM-2 medium+5 ng/ml VEGF+200 ng/ml Ang1+1 μg/ml Nesvacumab.

Cells were incubated at 37°C in 5% $CO_2$. After 24 h, 10 μl/well of FITC-Dextran (4 mg/ml) was added to the upper chamber, and placed at 37°C in 5% $CO_2$. After 30 min, the culture medium in the lower chamber was removed, diluted at 1:10 with PBS, and detected on a multifunctional microplate reader using an excitation wavelength of 488 nm and an emission wavelength of 535 nm.

The experiment results are shown in Figure 8. As shown in this figure, VEGF still caused significant vascular leakage on HUVEC cells in the presence of Ang1, but the VEGF-induced vascular leakage was significantly inhibited after the addition of anti-Ang2 antibody. With administration of antibody hz81H10.4, VEGF-induced increase in vascular endothelial cell permeability in the presence of ang1 was almost completely inhibited, which effect of antibody hz81H10.4 was superior to that of the positive control antibody Nesvacumab.

**Example 12. Acute Respiratory Distress syndrome Experiment**

**Example 12.1 Anti-Ang2 Antibody Inhibits Acute Respiratory Distress syndrome Induced by Different Concentrations of LPS**

Acute respiratory distress syndrome (ARDS) is an acute and critical disease in clinical practice characterized by severe respiratory distress and progressive respiratory failure. Its pathological features include: diffuse damage to alveolar epithelial barrier and pulmonary capillary endothelial barrier, leading to inflammatory cytokine infiltration and increased lung weight. A LPS-induced acute respiratory distress syndrome model in New Zealand white rabbits was used to mimic the abnormal lung function caused by acute lung inflammatory injury in human patients; and the function of anti-ANG2 antibody in helping the recovery of alveolar-capillary barrier in individual animals with pneumonia was investigated.

**Animal Modeling**

Animal species: New Zealand rabbits (2-5 months old) were purchased from Suzhou Genescience Biotechnology Co., Ltd. (laboratory animal production license No.: SCXK (Su) 2020-0002; laboratory animal quality certificate No.: 20200907214). The animals were subjected to one week of acclimation prior to the initiation of the study.

Lipopolysaccharide (LPS) was purchased from SIGMA, with Cat. No. of L2630-100MG-PW, and lot No. of 0000082412.

On the day of administering lipopolysaccharide (LPS) (the agent for modeling), an appropriate amount of LPS was accurately weighed and added with an appropriate amount of sodium chloride injection, and the mixture was well mixed by vortex oscillation. Finally, LPS was prepared at the concentrations of 0.5 mg/mL, 1 mg/mL, 2 mg/mL and 5 mg/mL. An appropriate amount of HZ81H10.4 stock solution was aseptically weighed and added with an appropriate amount of sterile PBS, then vortexed to mix well, and finally the test solution was prepared at the concentration of 7.5 mg/mL. Twelve male animals that passed the quarantine were randomly divided into LPS low-dose group, LPS medium-dose group and LPS high-dose group after weighing, with 4 animals in each group, and the average weight of animals in each group was not different. All the animals were administered with the modeling agent of lipopolysaccharide (LPS) via both intraperitoneal injection and intratracheal nebulization. See the table below for details:

Table 2 Dose of LPS Used in the Establishment of Acute Respiratory Distress Syndrome Model

| Group No. | Group | Modeling by intraperitoneal injection | | Modeling by intratracheal nebulization | | Modeling volume (mL/kg) |
|---|---|---|---|---|---|---|
| | | Dose (mg/kg) | Concentrati on (mg/ml) | Dose (mg/kg) | Concentratio n (mg/ml) | |
| 1 | LPS low-dose group | 0.5 | 1 | 0.5 | 1 | 1 |
| 2 | LPS medium-dose group | 0.2 | 1 | 1.0 | 2 | 2 |
| 3 | LPS high-dose group | 0.2 | 0.5 | 2.5 | 5 | 3 |

Method of modeling:

Three LPS dose groups were divided into three batches for modeling operation.

On Day 0, lipopolysaccharide (LPS) was injected intraperitoneally to the animals of each group at a dose calculated according to their body weight.

On Day D1, 16 h ($\pm$ 30 min) after intraperitoneal injection of LPS, the animals in each group were given LPS via intratracheal nebulization according to the modeling dose described in Table 2 above.

Intraperitoneal injection of lipopolysaccharide (LPS) was performed as follows: the amount of LPS given to each animal was drawn using a disposable syringe according to the most recently weighed body weight, and was administered intraperitoneally. The volume of LPS to be administered was round to the nearest tenth. If the LPS volume was between two volume graduation lines, LPS was drawn according to the volume value of the upper graduation line.

Intratracheal nebulization of LPS was performed as follows: animals were anesthetized by intramuscular injection of Zoletil 50 (8 mg/kg, 50 mg/mL) combined with xylazine hydrochloride (0.3 mg/kg, 20 mg/mL) and then the animals were fixed to a rabbit fixator placed at 30°. An animal anesthesia laryngoscope was used to press the animal's tongue base to expose the glottis. The needle (with a blunt tip) of a liquid micronebulizer that drew a quantitative amount of LPS solution was gently inserted into the trachea, then the piston was rapidly pushed to nebulize the LPS solution into the lungs, then the needle was quickly withdrawn, and the animal was released from the fixator with the head facing upward and rotated left and right to distribute the LPS as evenly as possible throughout the lung lobes. The volume of LPS to be administered was round to the nearest tenth. If the LPS volume was between two volume graduation lines, LPS was drawn according to the volume value of the upper graduation line.

**Antibody Dose and Dosing Regimen**

Approximately 0.5 h after the animals were intraperitoneally injected with the modeling reagent LPS, the test antibody was administered according to Table 3 below.

Table 3 Antibody Dosing Regimen for the Groups of Acute Respiratory Distress Syndrome Animal Model

| Group | | Dose (mg/kg) | Concentration (mg/ml) | Volume (mL/kg) | Animal No. |
|---|---|---|---|---|---|
| 1 | LPS low-dose group | 15 | 7.5 | 2 | 1, 2 |
| | | 0 | 0 | 2 | 3, 4 |
| 2 | LPS medium-dose group | 15 | 7.5 | 2 | 5, 6 |
| | | 0 | 0 | 2 | 7, 8 |
| 3 | LPS high-dose group | 15 | 7.5 | 2 | 9, 10 |
| | | 0 | 0 | 2 | 11,12 |
| Note: The first two animals in each group were injected with the test antibody via marginal ear vein, and the last two animals were injected with sodium chloride injection via marginal ear vein. | | | | | |

**Results**

Anti-hAng2 antibody hz81H10.4 Inhibites leukocyte infiltration in the alveoli

After euthanasia of the animals on D7, a tracheal cannula was inserted into the left bronchus and the cannula was fixed to the bronchus with sutures. A total volume of 10 mL/kg sodium chloride injection solution was drawn using a syringe and slowly injected to fill the lungs. The pulmonary alveoli were lavaged three times, with each lavage lasting about 10s, and the lavage fluids were collected in a centrifuge tube with an appropriate volume. The collected lavage fluid, 2 mL per animal, was retained and centrifuged at 4 °C at about 3000 rpm for 10 min. The supernatant was discarded, and the precipitate was resuspended in 1 mL of PBS buffer for total and differential leukocyte count.

Anti-hAng2 antibody hz81H10.4 effectively inhibited the infiltration of leukocytes and various types of inflammatory cells in the alveoli of ARDS animals. The results of the LPS low-dose group are presented in Figure 9. As shown in the figure, following the administration of the antibody drug to LPS-induced ARDS animals, the total white blood cell count, as well as the differential count (counts of inflammatory cells, neutrophils, lymphocytes and monocytes) in the pulmonary alveolar lavage fluid, were significantly decreased compared to the control group to which the sodium chloride was injected.

Anti-hAng2 Antibody hz81H10.4 Improves Lung Weight Index

All animals in each group (including those found dead, moribund or euthanized) were dissected and the tissues were preserved. The animals were placed in a refrigerator at 2-8°C after being found dead during non-working hours and dissected as soon as possible within 24 hours. The lung tissues and bronchi were dissected and preserved. During the necropsy, the lungs, trachea and bronchi of the animals were observed for abnormalities. Lungs were excised using scissors and weighed to calculate lung weight index (lung weight index= lung weight/body weight $\times$ 100%).

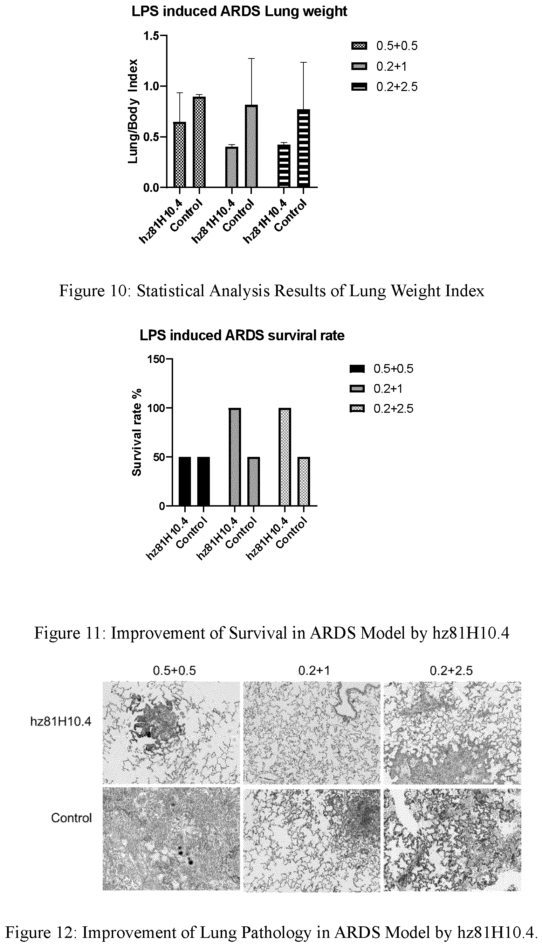

Figure 10 shows the calculated results of the lung weight index. The normal lung weight index of male New Zealand rabbits is about 0.3 ("Study on the Chief Organ Weight and Coefficients of Different Species of Experimental Rabbits", Chinese Journal of Comparative Medicine, December 2004, Volume 14, No. 6). Each of the three doses of LPS induced significant increases in lung weight index in individuals of the control group. Administration of the antibody showed a general tendency to reduce the LPS-induced increase in lung weight index.

Anti-hAng2 Antibody hz81H10.4 Improves Animal Survival Rate

After the scheduled euthanasia on Day 7, the ratio of the number of surviving animals to the total number of animals in each group during the test period was calculated. Survival rate (%) = the number of surviving animals in a group/ the total number of animals in the group $\times$ 100%. The results are shown in Figure 11. Overall, the administration of the antibody improved the survival rate in ARDS animal model.

Anti-hAng2 Antibody hz81H10.4 Improves Lung Pathology in Animals

During necropsy, the left lung was excised using scissors to collect bronchoalveolar lavage fluid. The remaining right lung tissues and bronchi were fixed in 10% neutral buffered formalin solution, embedded in paraffin, sliced, prepared into sections and stained with HE to observe the pathological morphology of the lung tissues. Pathological examination of lung tissues was performed using a 4-point scale (minimal, mild, moderate, severe) according to the Standard Terminology for Diagnosis and Classification (DOI: 10.1177/0192623318761348).

Figure 12 shows the staining results of pathological lung sections of the ARDS model.

- Low-dose LPS + hz81H10.4 group (upper left): slight inflammatory cell infiltration in alveoli/interstitium in the right lung;

- Low-dose LPS + control group (lower left): severe inflammatory cell infiltration in alveoli/interstitium/pluera, severe edema/fibrin-like material exudation, moderate thrombus, severe degeneration/necrosis in blood vessels/bronchi/alveoli/interstitium, mild abscess, mild alveolar wall thickening and slight hemorrhage in pleura in the right lung;

- Medium-dose LPS + hz81H10.4 group (upper middle): no abnormal pathological changes were observed in the right lung.

- Medium-dose LPS + control group (lower middle): mild inflammatory cell infiltration in alveoli/interstitium/bronchi/pleura, slight abscess, and mild alveolar wall thickening in the right lung;

- High-dose LPS + hz81H10.4 group (upper right): mild inflammatory cell infiltration in alveoli/interstitium/bronchi, mild alveolar wall thickening, slight edema/fibrin-like material exudation in the right lung;

- High-dose LPS +control group (lower right): severe congestion, moderate inflammatory cell infiltration in alveoli/interstitium, slight edema/fibrin-like material exudation and slight alveolar hemorrhage in the right lung;

Pathological findings revealed that hz81H10.4 treatment can ameliorate LPS-induced alveolar/interstitial/bronchial/pleural inflammatory cell infiltration, edema/fibrin-like material exudation, lung abscess, vascular/bronchial/alveolar/interstitial degeneration/necrosis, alveolar wall thickening, thrombus and pleural hemorrhage.

Mohammad Reza Mokhber Defouli et al. reported (https://doi.org/10.1186/s13054-018-2272-x) that, in order to investigate the therapeutic efficacy of drug candidates in ARDS, a rabbit model of ARDS was induced by intrapulmonary administration of LPS at 400 $\mu$g/kg. In thisExample, ARDS animal models induced by the low-, medium-, and high-dose of LPS were investigated. The three dose levels of LPS induced ADRS symptoms in the animal individuals of the control group and no significant difference in the ADRS parameters tested was observed across the three dose groups. In contrast, the administration of anti-ANG2 antibody in the three LPS dose groups not only improved inflammatory cell infiltration in the lungs of ADRS animals, but also showed a general tendency to alleviate other symptoms of ADRS animals and improve animal survival rate.

**Example 12.2. Inhibitory Effect of Anti-Ang2 Antibody on ARDS induced by Different Concentrations of LPS**

In order to investigate the therapeutic effect of different concentrations of anti-Ang2 antibody on LPS-induced ARDS model, the LPS-induced ARDS animals were treated with low dose (5 mg/kg), medium dose (15 mg/kg) and high dose (25 mg/kg) of HZ81H10.4 antibody in this Example.

The animals used in this Example were the same as in Example 12.1.

Thirty-six male animals that passed quarantine were weighed and randomly divided into model control group (9 animals, injected with sodium chloride solution), low-dose antibody test product group (7 animals), medium-dose antibody test product group (7 animals), high-dose antibody test product group (7 animals) and positive control group (6 animals, injected with dexamethasone at 2 mg/kg). The average body weights were not different across the groups. The modeling agent LPS was drawn at an amount according to the body weight of the animals, and was delivered to the animals via intraperitoneal injection at 0.2 mg/kg (0.2 mL/kg), and 16 hours ($\pm$ 30 min) after the intraperitoneal injection, delivered to the animals via intratracheal nebulization at 0.5 mg/kg (0.5 mL/kg) .

At about 0.5 h after the animals were intraperitoneally injected with LPS to establish the model, animals were treated with drugs (one dose), intravenously in Groups 1-4, and intraperitoneallyin Gourp 5. On day 7 after modeling with intraperitoneal injection, the model animals were subjected to CT examination and blood gas analysis and then euthanized; the bronchoalveolar lavage fluid was collected and analyzed, and the lung tissues were embedded for preparation of pathological sections.

Table 4 Antibody Dosing with Gradient Concentrations for the Groups of Acute Respiratory Distress Syndrome Animal Model

| | Group | Dose (mg/kg) | Concentration (mg/ml) | Volume (mL/kg) | Animal No. |
|---|---|---|---|---|---|
| 1 | Model control group | 0 | 0 | 2 | 1~9 |
| 2 | Low-dose test product group | 5 | 2.5 | 2 | 10~16 |
| 3 | Mid-dose test product group | 15 | 7.5 | 2 | 17~23 |
| 4 | High-dose test product group | 25 | 12.5 | 2 | 24~30 |
| 5 | Positive control group | 2 | 5 | 0.4 | 31~36 |

**Results**

Anti-hAng2 Antibody Inhibits Leukocyte Infiltration in the alveoli

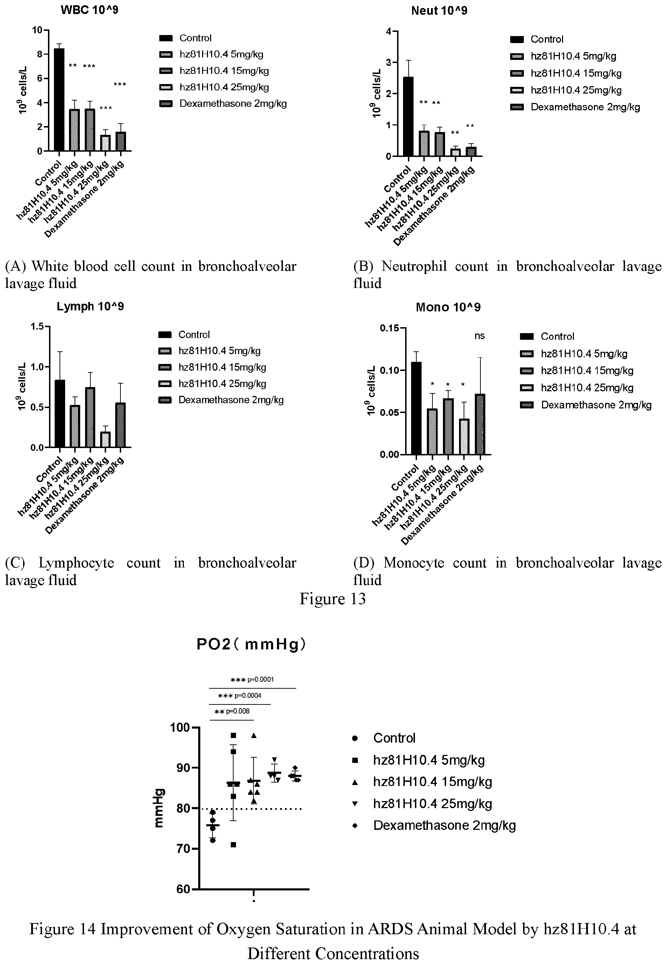

The bronchoalveolar lavage fluid was detected as in Example 12.1 to reveal leukocyte infiltration in the alveoli of ARDS animals. The results are shown in Figure 13. Consistent with the results in Example 12.1, administration of antibody hz81H10.4 significantly inhibited leukocyte infiltration in the alveoli of ARDS animals (*: $p < 0.05$; **: $p < 0.01$; ***: $p < 0.001$) ; and administration of antibody hz81H10.4 exhibited dose-dependent effects, with the greatest therapeutic efficacy at 25 mg/ml in improving the infiltration of leukocytes and various types of inflammatory cells (neutrophils, lymphocytes and monocytes) in the alveoli, and demonstrated a therapeutic efficacy that was comparable to that of dexamethasone in reducing the total leukocyte count and the neutrophil count, and superior to that of dexamethasone in reducing the infiltration of lymphocytes and monocytes.

Anti-hAng2 Antibody Enhances Oxygen Saturation in the model Animals

The effect of antibody on oxygen saturation of the model animals was investigated by blood gas detection. At the end of the CT examination, the animals were anesthetized, and a midline ventral incision was performed to isolate the abdominal aorta, and about 0.5 mL of arterial blood was collected using an arterial blood collection syringe. After arterial blood was collected, the needle was gently inserted into the blood entry port on the test card and the blood was slowly injected to fill the sample collection tube. When the blood reached the sample collection level, sample collection was stopped, the cap was closed, and the blood automatically entered the test tube. The test card was inserted into the i-STAT ® 1 Handheld Blood Gas Analyzer (300-G. Abott Point of Care Inc. USA), and the dection indexes included $PO_2$ (mmHg), $PCO_2$ (mmHg), pH and $sO_2$%.

The detection results of $PO_2$ are shown in Figure 14. In the control group, ARDS model animals showed hypoxemia, and the partial pressure of oxygen was lower than 80 mmHg. In the antibody treated group, the antibody induced improvement in oxygen saturation in ARDS model animals in a dose-dependent manner, and the improvement was statistically significant at the doses of 15 mg/ml and 25 mg/ml (*: $p < 0.05$; **: $p < 0.01$; ***: $p < 0.001$); moreover, the effect of the antibody dosed at 25 mg/ml was comparable to that of dexamethasone in terms of improving oxygen saturation in the model animals.

Anti-hAng2 Antibody Improves lung Pathology in Animals

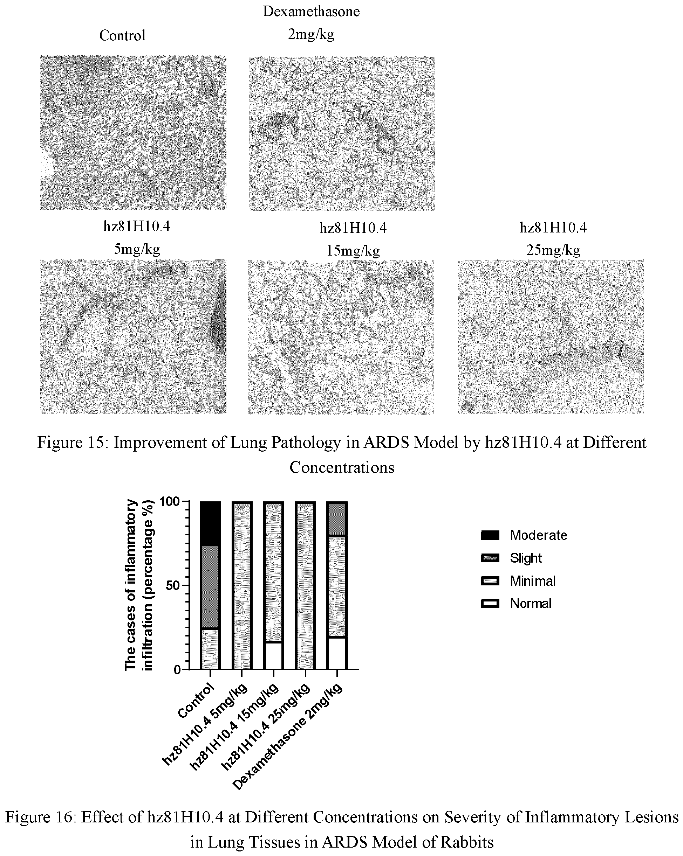

During necropsy, the left lung was excised using scissors to collect bronchoalveolar lavage fluid. The remaining right lung tissues and bronchi were fixed in 10% neutral buffered formalin solution, embedded in paraffin, sliced, prepared into sections, and stained with HE to observe the pathological morphology of the lung tissues. Pathological examination of lung tissues was performed using a 4-point scale (minimal, mild, moderate, severe) according to the Standard Terminology for Diagnosis and Classification. Figure 15 shows the staining results of lung pathological sections of the ARDS animals. Figure 16 shows the proportion of individual animals with different grades of lung tissue inflammatory lesions in different treatment groups.

In pathological examinations, lung tissues of animals in the control group were observed to have mild inflammatory cell infiltration in blood vessels/alveoli/peribronchus/bronchial epithelium/interstitium/pleura, alveolar wall thickening, mild alveolar/bronchial/interstitial degeneration/necrosis, slight pleural fibrosis, and mild exudation of fibrin like material in the alveoli. Compared with the control group, the administration of low-, medium- and high-doses of hz81H10.4 and positive control dexamethasone exhibited alleviative effect on LPS-induced alveolar/peribronchial/interstitial inflammatory cell infiltration, alveolar wall thickening, exudation of fibrin like material in the alveoli, alveolar wall/bronchial/interstitial degeneration/necrosis, pleural fibrosis, arterial thrombotic vasculitis and pleural inflammation.

Anti-hAng2 Antibody Improves CT images in Animals

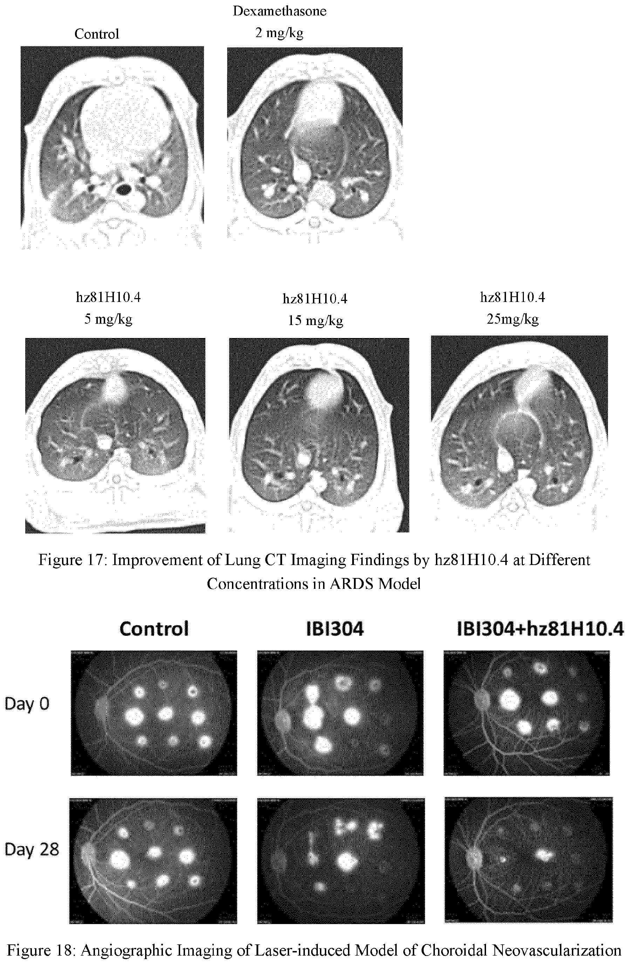

On D7, animals were intramuscularly injected with Zoletil 50 (8 mg/kg, 50 mg/mL) and xylazine hydrochloride injection (0.5 mg/kg, 20 mg/mL) for anesthesia and were laid flat on the CT scanning table for CT scanning of the lungs, as shown in Figure 17.

CT images showed that the CT symptoms of local pulmonary inflammation, increased markings in both lungs, and low transmittance in both lungs wereimproved in the hz81H10.4 group.

**Example 12.3. Anti-Ang2 Antibody Improves Survival Rate in LPS-induced ARDS Animals**

LPS was administered (0.2 mg/kg intraperitoneally + 0.5 mg/kg intratracheally) to the rabbit model animals, followed by the administration of hz81H10.4 antibody (5 mg/kg intravenously). The method of animal modeling and subsequent drug administration was the same as that in Example 12.2. In addition, sodium chloride injection as the negative control in place of the antibody was administered intravenously, and dexamethasone (2mg/kg) as the positive control was administered intraperitoneally. There were 7 animals in each group. After antibody administration, the animals were dissected on Day 7 to calculate the lung weight index and survival rate.

Anti-hAng2 Antibody Improves Lung Weight Index

After antibody drug administration, the lung weight index of the model animals was calculated to investigate the ameliorative effect of the antibody on lung weight index of the ARDS animals. Compared with the control group, a significant improvement in lung weight index was observed in the antibody group.

Anti-hAng2 Antibody Improves Survival Rate in Animals

Animal survival rate was determined as described in Example 12.1. Briefly, after the scheduled euthanasia on Day 7, the ratio of the number of surviving animals to the total number of animals in each group during the test period was calculated and the survival rate (%) was calculated using the following formula: Survival rate= the number of surviving animals in a group/the total number of animals in the group x100%. Compared with the control group, an improvement in survival rate was observed in ARDS model animals treated with antibody.

**Example 13. Anti-ang2 Antibody Ameliorates Ocular Neovascularization**

In vivo pharmacodynamic study in animals

In this experiment, the ameliorative effect of the hz81H10.4 antibody of the present invention in combination with an anti-VEGF inhibitor (IBI304, the sequence shown in SEQ ID NO: 60) on fundus neovascularization disorder accompanied with vascular leakage was investigated using a rhesus monkey model of laser-induced choroidal neovascularization.

Rhesus monkey:

Species: Macaca mulatta; grade: conventional level; body weight: 3.30-4.20 kg at purchase; body weights were within 3.35- 4.35 kg at modeling; purchased from Sichuan Greenhouse Biotechnology Co., Ltd., production license No.: SCXK (Chuan) 2014-013, test quality certificate No.:0022202.

In this study, laser photocoagulation around the central fovea of macula in ocular fundus of rhesus macaque was performed to induce choroidal neovascularization so as to establish an animal model mimiking human choroidal neovascularization. Fundus fluorescein angiography (FFA) was performed before and 20 days after photocoagulation to determine the modeling status. Twelve successfully-established model rhesus monkeys (half male and half female) were selected and divided into 3 groups: model control group, IBI304 group and IBI304 + hz81H10.4 group, with 4 monkeys in each group (half male and half female). At 21 days after photocoagulation, animals of the IBI304 group and IBI304 + 81H10.4 group were intravitreously injected in both eyes, with 0.6 mg/mL IBI304 and 0.6 mg/mL IBI304 +1.8 mg/mL hz81H10.4 at 30 μg/eye and 30 +90 μg/eye, respectively. The animals in the model control group were injected with an equal volume of 0.9% sodium chloride injection. Color fundus photography and fundus fluorescein angiography were performed on Day 7, Day14, Day 21 and Day 28 post drug administration to observe the inhibitory effect of the test products on choroidal neovascularization. The animals were euthanized 29 days after drug administration, and their two eyes were excised for histological examination with HE staining.

Experimental Design Table

| Group | Dose | Dosing volume | Concentration | Method of administration |
|---|---|---|---|---|
| IBI304 | 30 μg/eye | 50 μl/eye | 0.6mg/ml | Intravitreal administration |
| IBI304+hz81H10.4 | 30 μg + 90 μg/eye | 50 μl/eye | 0.6mg+1.8mg/ml | Intravitreal administration |

\* hz81H10.4 is also represented as code IBI335

Fundus Fluorescein Angiography

Improvement rate of fluorescein leakage area

Improvement rate of fluorescein leakage area = (fluorescein leakage area before drug administration × relative fluorescence intensity - fluorescein leakage area after drug administration× relative fluorescence intensity)/ fluorescein leakage area before drug administration× relative fluorescence intensity

Relative fluorescence intensity= mean fluorescence intensity in fluorescence spot leakage area- mean fluorescence intensity in the background area

The fundus fluorescein angiographic results of the model rhesus monkeys were shown in Figure 18-19, which revealed that the vascular leakages in the IBI304 + hz81H10.4 combination group were better inhibited compared to those obtained on Day 0, and this effect persisted from Day 7 through Day 28. At the time point of Day 28, the inhibitory effect of IBI304 + hz81H10.4 combination was significantly superior to that of IBI304 alone.

Grading of Fluorescent Spots

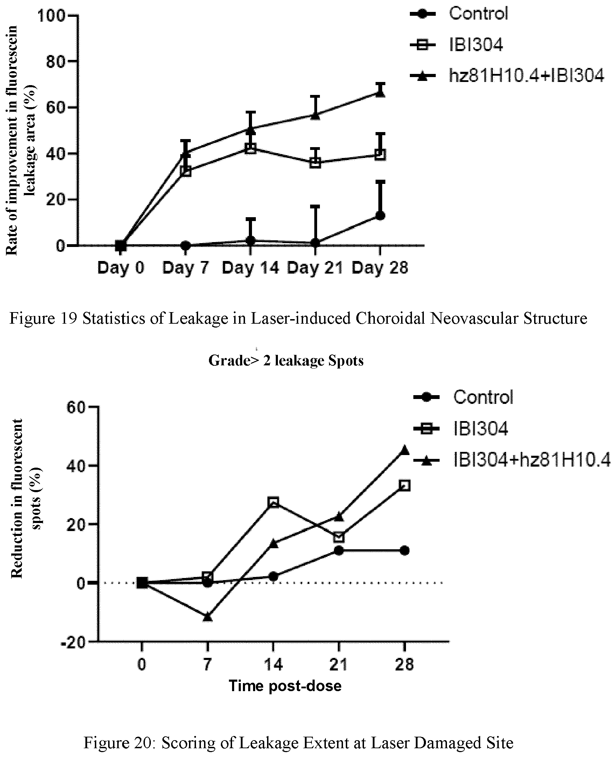

The grading scale of the light spots captured by fluorescence angiography after modeling is as follow: Grade 1: the light spot does not have high-intensity fluorescence; Grade 2: the light spot has high-intensity fluorescence but without fluorescein leakage; Grade 3: the light spot has high-intensity fluorescence with mild fluorescein leakage that does not extend beyond the edge of the light spot; Grade 4: the light spot has high-intensity fluorescence, with significant fluorescein leakage beyond the edge of the light spot. The percentage of light spots of grade 2-4 was counted. The results showed that the IBI304 + hz81H10.4 group was superior to IBI304 group and to the control group in terms of the improvement rate of grade 2-4 spots (the percentage of decrease in the number of grade 2-4 spots) at the late stage in the animal model (Figure 20).

These results showed that the antibody of the present invention exhibited a significant anti-neovascularization effect and a vascular leakage ameliorative effect 28 days after administration (Figures 18-20), demonstrating that the antibody of the present invention in combination with an anti-VEGF inhibitor has a significant inhibitory effect on laser-induced fundus neovascularization and a protective effect on vascular integrity.

Immunofluorescence Staining and Histological Examination

At 29 days after administration, rhesus monkeys were anesthetized with sodium pentobarbital according to body weight (intravenous injection of about 30 mg/kg, and the dose could be adjusted according to animal health conditions), euthanized by exsanguination via the abdominal aorta or femoral artery, macroscopic observation was performed, and bilateral eyeballs were excised.

Six eyeballs were randomly selected from 8 eyes in each group, fixed with modified Davidson's fixative, embedded in paraffin and sectioned. The laser-induced modeling areas were taken for routine HE staining, CD31-IHC staining and CD31/Desmin double-fluorescein staining for histopathological examination.

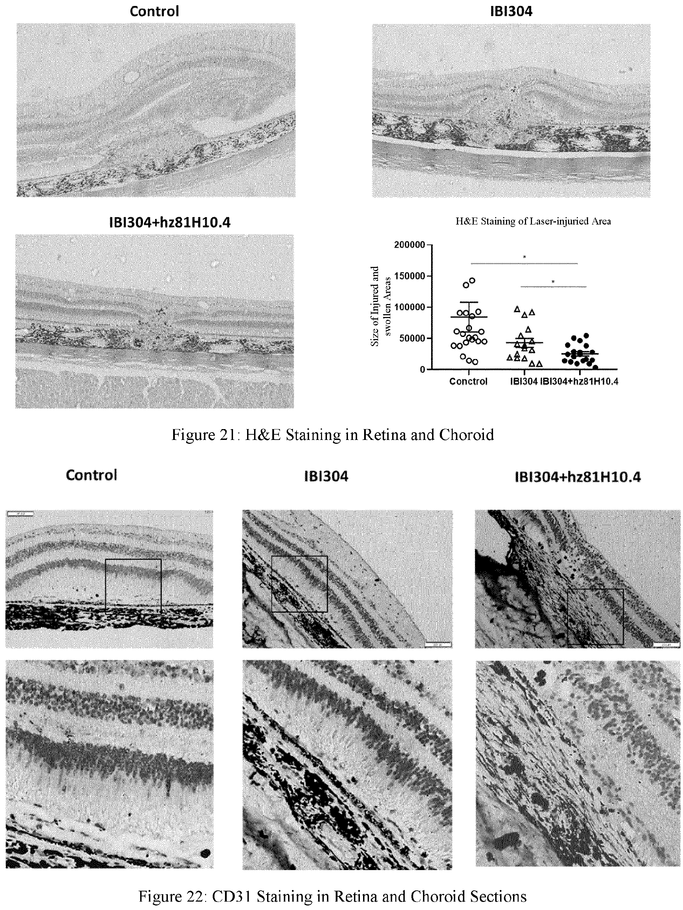

The laser-injuryed sites were histologically examined by HE staining to detect the areas with tissue hyperplasia, edema and retinal detachment and the sizes of injured and swollen areas were calculated by the Image J software. The results showed that in the control group, there was large areas of edema at the laser-induced modeling sites, accompanied with significantly irregular retina morphology and tissue proliferation; after treatment with IBI304, edema was significantly ameliorated; in addition, a further improvement in retinal morphology and tissue proliferation in the laser-induced modeling sites was observed in the IBI304 + hz81H10.4 treatment group over the IBI304 monotherapy. The results are shown in Figure 21.

As shown in Figures 22-23, in order to histologically observe the degree of neovascularization in the laser-induced modeling sites, staining for CD31 (neovascularization biomarker) was performed on the laser-induced modeling sites. The results showed that a large number of vascular cells were present in the laser modeling sites in the control group, while significantly reduced vascular cell numberafter treatment in the IBI304 group. The number of neovascular cells was further reduced in the IBI304 + hz81H10.4 treatment group over IBI304 monotherapy so that the purpose of inhibiting neovascularization was achieved in the modelingsites.

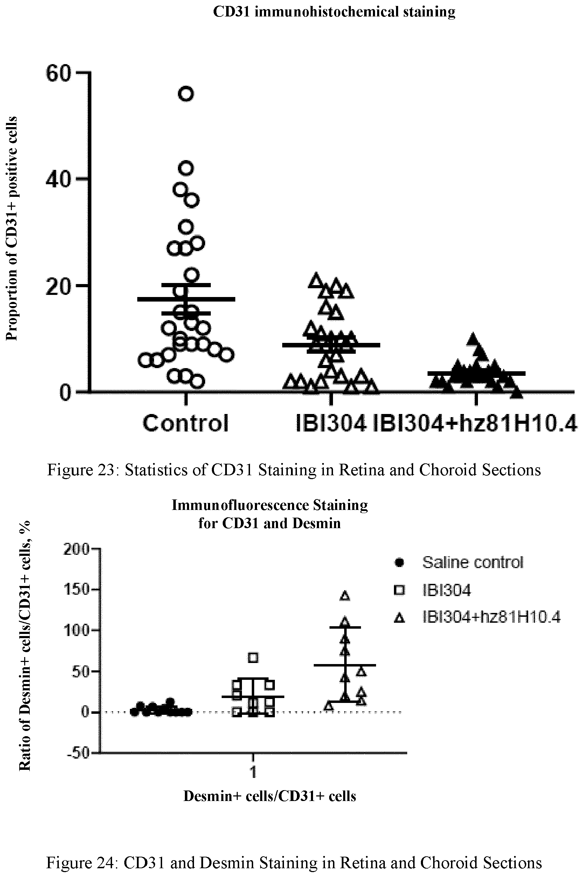

To investigate the impact of antibody on vascular integrity, the vascular status of the laser-induced modeling sites was assessed by double staining for CD31 and Desmin. The results showed that in the control group, there were a large number of CD31 positive cells (vascular endothelial cells) and a few Desmin positive cells (pericytes and smooth muscle cells) in the laser-induced modeling sites; in the IBI304 group, the number of CD31 positive cells decreased significantly, but the number of Desmin positive cells did not increase significantly. In the IBI304 + hz81H10.4 group, the number of CD31-positive cells was significantly reduced, and the number of Desmin-positive cells near CD31-positive cells was also significantly increased, indicating a significant improvement in vascular integrity. Figure 24 shows the ratio of Desmin+ cells/CD31+ cells in the laser-induced modeling sites in the control group, the IBI304 group and the IBI304 + hz81H10.4 group.

The results from the pathological sections reflect that the combination of the antibody of the present invention with anti-VEGF agent exhibited a superior effect in improving retinal morphology, inhibiting retinal choroidal neovascularization, and enhancing vascular integrity compared with anti-VEGF monotherapy.

**Description of Sequence Listing**

| SEQ ID NO: | Description |
|---|---|
| 1 | Hz81H10.4 Antibody HCDR1 |
| 2 | Hz81H10.4 Antibody HCDR2 |
| 3 | Hz81H10.4 Antibody HCDR3 |
| 4 | Hz81H10.4 Antibody LCDR1 |
| 5 | Hz81H10.4 Antibody LCDR2 |
| 6 | Hz81H10.4 Antibody LCDR3 |
| 7 | Amhz81H10.4.30 Antibody HCDR1 |
| 8 | Amhz81H10.4.30 Antibody HCDR2 |
| 9 | Amhz81H10.4.30 Antibody HCDR3 |
| 10 | Amhz81H10.4.30 Antibody LCDR1 |
| 11 | Amhz81H10.4.30 Antibody LCDR2 |
| 12 | Amhz81H10.4.30 Antibody LCDR3 |
| 13 | Amhz81H10.4.49 Antibody HCDR1 |
| 14 | Amhz81H10.4.49 Antibody HCDR2 |
| 15 | Amhz81H10.4.49 Antibody HCDR3 |
| 16 | Amhz81H10.4.49 Antibody LCDR1 |
| 17 | Amhz81H10.4.49 Antibody LCDR2 |

(continued)

| SEQ ID NO: | Description |
|---|---|
| 18 | Amhz81H10.4.49 Antibody LCDR3 |
| 19 | 81H10 Antibody VH |
| 20 | 81H10 Antibody VL |
| 21 | 86E9 Antibody VH |
| 22 | 86E9 Antibody VL |
| 23 | 107H7 Antibody VH |
| 24 | 107H7 Antibody VL |
| 25 | Hz81H10.4 Antibody VH |
| 26 | Hz81H10.4 Antibody VL |
| 27 | Amhz81H10.4.30 Antibody VH |
| 28 | Amhz81H10.4.30 Antibody VL |
| 29 | Amhz81H10.4.49 Antibody VH |
| 30 | Amhz81H10.4.49 Antibody VL |
| 31 | 81H10 Antibody HC |
| 32 | 81H10 Antibody LC |
| 33 | 86E9 Antibody HC |
| 34 | 86E9 Antibody LC |
| 35 | 107H7 Antibody HC |
| 36 | 107H7 Antibody LC |
| 37 | Hz81H10.4 Antibody HC |
| 38 | Hz81H10.4 Antibody LC |
| 39 | Amhz81H10.4.30 Antibody HC |
| 40 | Amhz81H10.4.30 Antibody LC |
| 41 | Amhz81H10.4.49 Antibody HC |
| 42 | Amhz81H10.4.49 Antibody LC |
| 43 | 81H10 Antibody VH DNA |
| 44 | 81H10 Antibody VL DNA |
| 45 | 86E9 Antibody VH DNA |
| 46 | 86E9 Antibody VL DNA |
| 47 | 107H7 Antibody VH DNA |
| 48 | 107H7 Antibody VL DNA |
| 49 | Nesvacumab HC |
| 50 | Nesvacumab LC |
| 51 | Antigen Ang2 |
| 52 | Antibody heavy chain constant region IgG: |
| 53 | Light chain constant region: (kappa light chain) |
| 54 | HCDR1 consensus sequence |
| 55 | HCDR2 consensus sequence |

(continued)

| SEQ ID NO: | Description |
| --- | --- |
| 56 | HCDR3 consensus sequence |
| 57 | LCDR1 consensus sequence |
| 58 | LCDR2 consensus sequence |
| 59 | LCDR3 consensus sequence |
| 60 | Amino acid sequence of anti-VEGF molecule used in combination |

**Some embodiments of the invention**

A1. An antibody or an antigen-binding fragment thereof that binds to ANG-2, comprising

(i) the HCDR 1, HCDR 2 and HCDR3 sequences of the heavy chain variable region as shown in SEQ ID NO: 19, 21, or 23 and the LCDR 1, LCDR 2 and LCDR 3 sequences of the light chain variable region as shown in SEQ ID NO: 20, 22, or 24; or

(ii) the HCDR 1, HCDR 2 and HCDR 3 sequences of the heavy chain variable region as shown in SEQ ID NO: 25, and the LCDR 1, LCDR 2 and LCDR 3 sequences of the light chain variable region as shown in SEQ ID NO: 26; or

(iii) the HCDR 1, HCDR 2 and HCDR 3 sequences of the heavy chain variable region as shown in SEQ ID NO: 27, and the LCDR 1, LCDR 2 and LCDR 3 sequences of the light chain variable region as shown in SEQ ID NO: 28, or

(iv) the HCDR 1, HCDR 2 and HCDR 3 sequences of the heavy chain variable region as shown in SEQ ID NO: 29 and LCDR 1, LCDR 2 and LCDR 3 sequences of the light chain variable region as shown in SEQ ID NO: 30.

A2. An antibody or an antigen-binding fragment thereof that binds to ANG-2, comprising 3 complementarity-determining regions, HCDRs, of a heavy chain variable region, and 3 complementarity-determining regions, LCDRs, of a light chain variable region, wherein,

(a) an HCDR1 comprises the amino acid sequence shown in SEQ ID NO: 1; an HCDR2 comprises the amino acid sequence shown in SEQ ID NO: 2; an HCDR3 comprises the amino acid sequence shown in SEQ ID NO: 3; an LCDR1 comprises the amino acid sequence shown in SEQ ID NO: 4; an LCDR2 comprises the amino acid sequence shown in SEQ ID NO: 5, and an LCDR3 comprises the amino acid sequence shown in SEQ ID NO: 6; or

(b) an HCDR1 comprises the amino acid sequence shown in SEQ ID NO: 7, an HCDR2 comprises the amino acid sequence shown in SEQ ID NO: 8; an HCDR3 comprises the amino acid sequence shown in SEQ ID NO: 9; an LCDR1 comprises the amino acid sequence shown in SEQ ID NO: 10; an LCDR2 comprises the amino acid sequence shown in SEQ ID NO: 11, and an LCDR3 comprises the amino acid sequence shown in SEQ ID NO: 12; or

(c) an HCDR1 comprises the amino acid sequence shown in SEQ ID NO: 13; an HCDR2 comprises the amino acid sequence shown in SEQ ID NO: 14; an HCDR3 comprises the amino acid sequence shown in SEQ ID NO: 15; an LCDR1 comprises the amino acid sequence shown in SEQ ID NO: 16; an LCDR2 comprises the amino acid sequence shown in SEQ ID NO: 17, and an LCDR3 comprises the amino acid sequence shown in SEQ ID NO: 18; or

(d) an HCDR1 comprises the amino acid sequence shown in SEQ ID NO: 54; an HCDR2 comprises the amino acid sequence shown in SEQ ID NO: 55; an HCDR3 comprises the amino acid sequence shown in SEQ ID NO: 56; an LCDR1 comprises the amino acid sequence shown in SEQ ID NO: 57; an LCDR2 comprises the amino acid sequence shown in SEQ ID NO: 58, and an LCDR3 comprises the amino acid sequence shown in SEQ ID NO: 59.

A3. The antibody or antigen-binding fragment thereof of embodiment A1 or A2, wherein the said antibody comprises a variant of the said CDR sequence combination, which collectively comprises at least one and not more than 12, 11, 10 or 5, 4, 3, 2 or 1 modification in amino acids (preferably amino acid substitution, preferably conservative substitution) in 1, 2, 3, 4, 5 or preferably 6 CDR regions, preferably wherein the heavy chain CDR3 and the light chain CDR3 remain unchanged.

A4. The antibody or antigen-binding fragment thereof of any one of the embodiments A1 to A3, comprising

(i) a heavy chain variable region comprising or consisting of an amino acid sequence having at least 80%, 85% or 90% sequence identity to an amino acid sequence shown in SEQ ID NO: 19, 21, or 23, and/or a light chain variable region comprising or consisting of an amino acid sequence having at least 80%, 85% or 90% sequence identity to an amino acid sequence shown in SEQ ID NO: 20, 22, or 24; or

(ii) a heavy chain variable region comprising or consisting of an amino acid sequence having at least 80%, 85% or 90% sequence identity to an amino acid sequence shown in SEQ ID NO: 25, 27, or 29, and/or a light chain variable region comprising or consisting of an amino acid sequence having at least 80%, 85% or 90% sequence identity to an amino acid sequence shown in SEQ ID NO: 26, 28, or 30.

A5, The antibody or antigen-binding fragment thereof of any one of embodiments A1 to A4, comprising a heavy chain variable region and a light chain variable region selected from the group consisting of

(i) a heavy chain variable region comprising or consisting of an amino acid sequence shown in SEQ ID NO: 19, 21, or 23, and a light chain variable region comprising or consisting of an amino acid sequence shown in SEQ ID NO: 20, 22, or 24; or

(ii) a heavy chain variable region comprising or consisting of an amino acid sequence shown in SEQ ID NO: 25, 27, or 29, and a light chain variable region comprising or consisting of an amino acid sequence shown in SEQ ID NO: 26, 28, or 30.

A6. The antibody or antigen-binding fragment thereof of any one of the preceding embodiments, wherein the said antibody is an antibody of IgG1, IgG2, or IgG4 type, or antigen-binding fragment thereof, preferably comprising a human IgG1 Fc region.

A7. The antibody or an antigen-binding fragment thereof of any one of the preceding embodiments, wherein the said antibody is a monoclonal antibody, a chimeric antibody, or a humanized antibody.

A8. The antibody or antigen-binding fragment thereof of any one of the preceding embodiments, wherein the said antigen-binding fragment is an antibody fragment selected from the group consisting of: Fab, Fab ', Fab'-SH, Fv, single-chain antibody such as scFv, (Fab ') 2 fragment, single-domain antibody, a diabody (dAb) or a linear antibody.

A9/ An anti-ANG-2 antibody or antigen-binding fragment thereof, wherein the said antibody has one or more of the following properties:

(i) inhibiting (e.g., competitively inhibiting) the binding of an antibody of any one of embodiments 1-8 to human ANG-2;

(ii) binding to the epitope same as or overlapping with that bound by an antibody of any one of embodiments A1-A8;

(iii) competing with an antibody of any one of embodiments A1-A8 for binding to human ANG-2, or

wherein the said antibody has one or more of the following properties:

(i) binding with high affinity to human ANG-2 but not to human ANG-1;

(ii) having immunological cross-reactivity with monkey ANG-2;

(iii) blocking the binding of human ANG-2 to human Tie2 protein;

(iv) blocking the binding of human ANG-2 to human Tie2 on a cell surface;

(v) blocking human ANG-2-induced Tie2 phosphorylation in cells;

(vi) blocking the binding of human ANG-2 to an integrin and inhibiting cell adhesion mediated by ANG2/integrin;

(vii) having a low nonspecific binding to cells not expressing ang2, such as retinal cells;

(viii) when used in combination with an anti-VEGF agent, resulting in one or more of the following effects in a vascular-related disorder, such as an ocular disease, e.g., choroidal neovascularization (CNV): reduction of vascular leakage, inhibition of neovascularization and improvement of vascular integrity.

A10. An isolated nucleic acid, which encodes an anti-ANG-2 antibody or antigen-binding fragment thereof of any one of the preceding embodiments.

A11. A vector, comprising the nucleic acid of embodiment A10, preferably wherein the vector is an expressing vector.

A12. A host cell, comprising the nucleic acid of embodiment A10 or the vector of embodiment A11, preferably wherein the host cell is a prokaryotic or eukaryotic cell, more preferably a yeast cell, a mammalian cell (e.g., A 293 cell or a CHO cell).

A13. A method of preparing an anti-ANG-2 antibody or antigen-binding fragment thereof, wherein the said method comprises culturing a host cell of embodiment A12 under conditions suitable for expressing a nucleic acid encoding the antibody or antigen-binding fragment thereof of any one of the preceding embodiments A1-9, optionally isolating the said antibody or antigen-binding fragment thereof, optionally the said method further comprising recovering the said anti-ANG-2 antibody or antigen-binding fragment thereof from the said host cell.

A14. An immunoconjugate comprising an antibody or antigen-binding fragment thereof of any one of the preceding embodiments A1-9 conjugated to a therapeutic or diagnostic agent.

A15. A multispecific antibody comprising an antibody or antigen-binding fragment thereof of any one of the preceding embodiments A1-9, preferably wherein the said multispecific antibody is a bispecific antibody that binds to ANG-2 and VEGF-A.

A16. A pharmaceutical composition, comprising an antibody or antigen-binding fragment thereof of any of the preceding embodiments A1-A9 or an immunoconjugate of embodiment A14 or a multispecific antibody of embodiment A15 and optionally a pharmaceutical excipient.

A17. The pharmaceutical composition of embodiment A16, comprising a second therapeutic agent; preferably, wherein the said second therapeutic agent is selected from the group consisting of a therapeutic drug for a vascular-related disease, such as an anti-neovascularization drug, and an anti-VEGF agent.

A18. The pharmaceutical composition of embodiment A17, wherein the said second therapeutic agent is an anti-VEGF agent, wherein the anti-VEGF agent is a fusion protein formed from the second immunoglobulin-like domain of VEGFR-1, the third immunoglobulin-like domain of VEGFR-2, and a human immunoglobulin Fc, wherein a peptide linker that increases the structural flexibility is inserted before the Fc; preferably, the anti-VEGF agent is as shown in SEQ ID NO: 60 or at least 90%, 91%, 92%, 93%, 94%, 95%, 96%, 97%, 98% or more than 99% identity thereto.

A19. A method for preventing or treating a disease or a disorder associated with an ANG2 biological activity, such as a vascular-related disease, in a subject, wherein the method comprises administering to the subject an effective amount of an anti-ANG-2 antibody or antigen-binding fragment thereof of any of the preceding embodiments A1-A10, or an immunoconjugate of embodiment A15, or a multispecific antibody of embodiment A16, or a pharmaceutical composition of embodiment A17 or A18; preferably the said disease is selected from the group consisting of an ocular neovascularization disease and a solid tumor.

A20. A method for detecting ANG-2 in a sample, comprises

(a) contacting the sample with an antibody or antigen-binding fragment thereof of any one of the preceding embodiments A1-A9; and

(b) detecting the formation of a complex between the antibody or antigen-binding fragment thereof and ANG-2; optionally, the antibody is detectably labeled.

**Some other embodiments of the invention:**

B1. A method for preventing or treating a disease accompanied with vascular leakage and/or vascular inflammation, comprises administering to a subject in need thereof an anti-Ang-2 antibody or antigenic fragment thereof, preferably wherein the said anti-Ang-2 antibody or antigenic fragment thereof comprises:

- the HCDR1, HCDR2 and HCDR3 sequences of the heavy chain variable region comprising SEQ ID NO: 7; and

- the LCDR1, LCDR2 and LCDR3 sequences of the light chain variable region comprising SEQ ID NO: 8.

B2. The method of embodiment B1, wherein the disease accompanied with vascular leakage is vascular leak syndrome, acute respiratory distress syndrome, or acute lung injury; and/or wherein the disease accompanied with vascular inflammation is a vascular infection or pulmonary inflammation,
preferably, the said disease is acute respiratory distress syndrome or acute pneumonia (e.g., viral or bacterial pneumonia).

B3. The method of embodiment B1, wherein the said disease is acute respiratory distress syndrome, wherein the administration of the said antibody ameliorates one or more of the following parameters of disease:

(1) the infiltration intensity of inflammatory cells in the lung tissues, such as the total number of infiltrating leukocytes, and/or the number of infiltrating inflammatory cells selected from one of neutrophils, lymphocytes and monocytes, or any combination thereof;

(2) the blood oxygen saturation in the subject, such as partial pressure of oxygen ($PO_2$) and/or oxygenation index ($PaO_2/FiO_2$);

(3) the pulmonary edema degree or lung weight index;

(4) the intensity of inflammatory lesions in lung tissue;

(5) the lung images (e.g., X-ray or CT images);

(5) the survival rate of the animal subject.

B4. A method for modulating vascular permeability, or for reducing vascular leakage and/or ameliorating a vascular inflammatory condition, comprising administering an anti-Ang-2 antibody or antigenic fragment thereof, wherein the said anti-Ang-2 antibody or antigenic fragment thereof comprises:

- the HCDR1, HCDR2 and HCDR3 sequences of the heavy chain variable region comprising SEQ ID NO: 7; and

- the LCDR1, LCDR2 and LCDR3 sequences of the light chain variable region comprising SEQ ID NO: 8.

B5. The method of embodiment B4, wherein the said increase in vascular permeability is induced by a pro-inflammatory cytokine such as VEGF, or a bacterial or viral infection or a bacterial endotoxin.

B6. A method of any of embodiments B1-B5, wherein the administration of the said anti-ANG2 antibody inhibits the expression of an inflammatory marker in vascular endothelial cells, preferably the said inflammatory marker is ICAM-1 and/or VCAM-1.

B7. The method of any one of embodiments B1-B6, wherein the said subject is an individual at the risk of developing ARDS; or wherein the said subject is an individual who has been diagnosed or suspected of having ARDS.

B8. The method of embodiments B1-B7, wherein the said anti-ANG2 antibody or antigen-binding fragment thereof specifically binds to human ANG2 and optionally has one or more of the following characteristics:

(i) binding with high affinity to human ANG-2 but not to human ANG-1, wherein preferably as measured by a Biacore affinity assay, e.g. as described in Example 2, the antibody binds to human ANG2 with a $K_D$ value of about 0.1-10 $\times$ 10$^{-10}$ M;

(ii) having immunological cross-reactivity with monkey and rabbit ANG-2 but not with murine ANG-2;

(iii) blocking the binding of human ANG-2 to human Tie2 proteins, such as human TIE2 on a cell surface;

(iv) having an inhibitory effect on human ANG-2-induced Tie2 phosphorylation in cells;

(v) blocking ANG-2 binding to an integrin;

(vi) having an inhibitory effect on cell adhesion mediated by ANG2/integrin;

(vii) having high binding specificity and selectivity for ANG2-expressing cells relative to cells not expressing ang2.

B9. A method or a use of embodiment B1-B7, wherein the said anti-ANG2 antibody or antigen-binding fragment thereof comprises:

- an HCDR1 sequence of SEQ ID NO: 1;

- an HCDR2 sequence of SEQ ID NO: 2;

- an HCDR3 sequence of SEQ ID NO: 3;

- an LCDR1 sequence of SEQ ID NO: 4;

- an LCDR2 sequence of SEQ ID NO: 5; and

- an LCDR3 sequence of SEQ ID NO: 6.

B10. A method of embodiment B9, wherein the said anti-ANG2 antibody or antigen-binding fragment thereof comprises:

- an VH sequence of SEQ ID NO: 7 or a sequence having at least 85%, 90%, 95%, or 99% identity thereto; and

- an VL sequence of SEQ ID NO: 8 or a sequence having at least 85%, 90%, 95%, or 99% identity thereto.

B11. The method of embodiments B1-B10, wherein the said anti-ANG2 antibody comprises a heavy chain Fc region, such as an Fc region of IgG1, IgG2 or IgG4 isotype, preferably a human IgG1 Fc region; and/or comprises a kappa light chain constant region, such as a human kappa light chain constant region.

B12. The method of embodiment B11, wherein the anti-ANG2 antibody has the heavy chain of SEQ ID NO: 9 and the light chain of SEQ ID NO: 10.

B13. The method of embodiments B1-B12, wherein the anti-ANG2 antibody is administered as a monotherapy.

B14. The method of embodiments B1-B13, wherein the anti-ANG2 antibody is administered in combination with a second therapeutic agent, preferably the second therapeutic agent is a VEGF blocker.

B15. The method of embodiments B1-B14, wherein the said anti-ANG2 antibody is administered subcutaneously or intraperitoneally.

B16. The method of embodiments B1-B15, wherein the said anti-ANG2 antibody is administered in a single-dosge or multiple dosges, preferably at least one dosage of the anti-ANG2 antibody is administered before or after the subject is exposed to an inflammatory agent such as a bacterium, a virus and a bacterial toxin, more preferably before the subject manifests pulmonary infiltration of inflammatory cells.

B17. The method of embodiments B1-B16, wherein the said anti-ANG2 antibody is contained in a pharmaceutical composition, and optionally, the pharmaceutical composition is contained in a container selected from the group consisting of a glass vial, a syringe, a prefilled syringe, an autoinjector, and a microinfusion set.

**Claims**

1. An antibody or antigen-binding fragment thereof that binds to an ANG-2, comprising three complementarity determining regions HCDRs of a heavy chain variable region, and three complementarity determining regions LCDRs of a light chain variable region, wherein:

   (a) an HCDR1 comprises the amino acid sequence shown in SEQ ID NO: 1; an HCDR2 comprises the amino acid sequence shown in SEQ ID NO: 2; an HCDR3 comprises the amino acid sequence shown in SEQ ID NO: 3; an LCDR1 comprises the amino acid sequence shown in SEQ ID NO: 4; an LCDR2 comprises the amino acid sequence shown in SEQ ID NO: 5; and an LCDR3 comprises the amino acid sequence shown in SEQ ID NO: 6; or
   (b) an HCDR1 comprises the amino acid sequence shown in SEQ ID NO: 7, an HCDR2 comprises the amino acid sequence shown in SEQ ID NO: 8; an HCDR3 comprises the amino acid sequence shown in SEQ ID NO: 9; an LCDR1 comprises the amino acid sequence shown in SEQ ID NO: 10; an LCDR2 comprises the amino acid sequence shown in SEQ ID NO: 11; and an LCDR3 comprises the amino acid sequence shown in SEQ ID NO: 12; or
   (c) an HCDR1 comprises the amino acid sequence shown in SEQ ID NO: 13; an HCDR2 comprises the amino acid sequence shown in SEQ ID NO: 14; an HCDR3 comprises the amino acid sequence shown in SEQ ID NO: 15; an LCDR1 comprises the amino acid sequence shown in SEQ ID NO: 16; an LCDR2 comprises the amino acid sequence shown in SEQ ID NO: 17; and an LCDR3 comprises the amino acid sequence shown in SEQ ID NO: 18; or
   (d) an HCDR1 comprises the amino acid sequence shown in SEQ ID NO: 54; an HCDR2 comprises the amino acid sequence shown in SEQ ID NO: 55; an HCDR3 comprises the amino acid sequence shown in SEQ ID NO: 56; an LCDR1 comprises the amino acid sequence shown in SEQ ID NO: 57; an LCDR2 comprises the amino acid sequence shown in SEQ ID NO: 58; and an LCDR3 comprises the amino acid sequence shown in SEQ ID NO: 59.

2. The antibody or antigen-binding fragment thereof of claim 1, comprising a heavy chain variable region and a light chain variable region selected from the group consisting of

   (i) a heavy chain variable region comprising or consisting of the amino acid sequence shown in SEQ ID NO: 25, and a light chain variable region comprising or consisting of the amino acid sequence shown in SEQ ID NO: 26;
   (ii) a heavy chain variable region comprising or consisting of the amino acid sequence shown in SEQ ID NO: 27, and a light chain variable region comprising or consisting of the amino acid sequence shown in SEQ ID NO: 28;
   (iii) a heavy chain variable region comprising or consisting of the amino acid sequence shown in SEQ ID NO: 29, and a light chain variable region comprising or consisting of the amino acid sequence shown in SEQ ID NO: 30;
   (iv) a heavy chain variable region comprising or consisting of the amino acid sequence shown in SEQ ID NO: 19, and a light chain variable region comprising or consisting of the amino acid sequence shown in SEQ ID NO: 20;
   (v) a heavy chain variable region comprising or consisting of the amino acid sequence shown in SEQ ID NO: 21, and a light chain variable region comprisng or consisting of the amino acid sequence shown in SEQ ID NO: 22; or
   (vi) a heavy chain variable region comprising or consisting of the amino acid sequence shown in SEQ ID NO: 23, and a light chain variable region comprising or consisting of the amino acid sequence shown in SEQ ID NO: 24.

3. The antibody or antigen-binding fragment thereof of any one of the preceding claims, wherein said antibody is an antibody of IgG1, IgG2, or IgG4 type, or antigen-binding fragment thereof, preferably comprising a human IgG1 Fc region.

4. An isolated nucleic acid encoding an anti-ANG-2 antibody or antigen-binding fragment thereof of any one of the preceding claims.

5. A vector or a host cell, comprising the nucleic acid of claim 4, preferably said vector is an expression vector, and preferably said host cell is a prokaryotic or eukaryotic cell, more preferably a yeast cell or a mammalian cell (e.g. a 293 cell or a CHO cell).

6. A method for preparing an anti-ANG-2 antibody or antigen-binding fragment thereof, comprising culturing a host cell of claim 5 under conditions suitable for expressing a nucleic acid encoding the antibody or antigen-binding fragment thereof of any one of the preceding claims 1-3, and optionally isolating said antibody or antigen-binding fragment thereof,
optionally said method further comprising recovering said anti-ANG-2 antibody or antigen-binding fragment thereof from said host cell.

7. A pharmaceutical composition comprising an antibody or antigen-binding fragment thereof of any one of the preceding claims 1-3, and optionally a pharmaceutical excipient,

optionally, said pharmaceutical composition further comprises a second therapeutic agent;
preferably, said second therapeutic agent is selected from the group consisting of therapeutic agents for vascular-related diseases, such as an anti-neovascularization agent, e.g. an anti-VEGF agent.

8. The pharmaceutical composition of claim 7, wherein said second therapeutic agent is an anti-VEGF agent, wherein said anti-VEGF agent is a fusion protein formed from the second immunoglobulin-like domain of VEGFR-1, the third immunoglobulin-like domain of VEGFR-2, and a human immunoglobulin Fc, wherein a peptide linker that increases the structural flexibility is inserted before the Fc;
preferably, the anti-VEGF agent is as shown in SEQ ID NO: 60, or at least 90%, 91%, 92%, 93%, 94%, 95%, 96%, 97%, 98%, or more than 99% identity thereto.

9. A method for preventing or treating a disease or a disorder associated with an ANG2 biological activity, such as a vascular-related disease (e.g., an ocular neovascularization disease and a solid tumor) in a subject, wherein said method comprises administering to the subject an effective amount of an anti-ANG-2 antibody or an antigen-binding fragment thereof of any of the preceding claims 1-3, or a pharmaceutical composition of claim 7.

10. A method for preventing or treating a disease accompanied with vascular leakage and/or vascular inflammation, or for modulating vascular permeability, or for reducing vascular leakage and/or ameliorating a vascular inflammatory condition, comprising administering to a subject in need thereof an anti-Ang-2 antibody or antigen-binding fragment thereof of any one of claims 1-3,
preferably, wherein the anti-Ang-2 antibody or antigen-binding fragment thereof comprises:

- an HCDR1 sequence of SEQ ID NO: 1;
- an HCDR2 sequence of SEQ ID NO: 2;
- an HCDR3 sequence of SEQ ID NO: 3;
- an LCDR1 sequence of SEQ ID NO: 4;
- an LCDR2 sequence of SEQ ID NO: 5; and
- an LCDR3 sequence of SEQ ID NO: 6.

11. The method of claim 10, wherein the disease accompanied with vascular leakage is vascular leak syndrome, acute respiratory distress syndrome, or acute lung injury; and/or wherein the disease accompanied with vascular inflammation is a vascular infection or a pulmonary inflammation,
preferably, said disease is acute respiratory distress syndrome or acute pneumonia (e.g., viral or bacterial pneumonia).

12. The method of claim 10, wherein said disease is acute respiratory distress syndrome, wherein the administration of said antibody ameliorates one or more of the following disease parameters :

(1) the infiltration intensity of inflammatory cells in lung tissues, such as the total number of infiltrating leukocytes, and/or the number of infiltrating inflammatory cells selected from one of neutrophils, lymphocytes and monocytes, or any combination thereof;

(2) the blood oxygen saturation in the subject, such as partial pressure of oxygen ($PO_2$) and/or oxygenation index ($PaO_2/FiO_2$);

(3) the pulmonary edema degree or lung weight index;

(4) the intensity of inflammatory lesions in lung tissue;

(5) the lung images (e.g., X-ray or CT images); and

(6) the survival rate of the animal subject.

13. The method of claim 10, wherein said increase in vascular permeability is induced by a pro-inflammatory cytokine, such as VEGF, or a bacterial or viral infection or a bacterial endotoxin.

14. The method of any one of claims 10-13, wherein the administration of said anti-ANG2 antibody inhibits the expression of an inflammatory marker in vascular endothelial cells, preferably said inflammatory marker is ICAM-1 and/or VCAM-1.

15. The method of any one of claims 10-14, wherein said subject is at the risk of developing ARDS; or wherein said subject has been diagnosed or suspected of having ARDS.

16. A method of claim 10, wherein said anti-ANG2 antibody or antigen-binding fragment thereof comprises:

- an VH sequence of SEQ ID NO: 25, or a sequence having at least 85%, 90%, 95%, or 99% identity thereto; and
- an VL sequence of SEQ ID NO: 26, or a sequence having at least 85%, 90%, 95%, or 99% identity thereto,

optionally, said anti-ANG2 antibody comprises a heavy chain Fc region, such as an Fc region of an IgG1, IgG2 or IgG4 isotype, preferably a human IgG1-Fc region; and/or comprises a kappa light chain constant region, such as a human kappa light chain constant region.

17. The method of claim 10, wherein said anti-ANG2 antibody has a heavy chain of SEQ ID NO: 37 and a light chain of SEQ ID NO: 38.

18. The method of claims 10-17, wherein said anti-ANG2 antibody is administered as a monotherapy agent, or wherein said anti-ANG2 antibody is administered in combination with a second therapeutic agent, preferably the second therapeutic agent is a VEGF blocker.

19. The method of claims 10-18, wherein said anti-ANG2 antibody is administered in a single dosage or multiple dosages, preferably at least one dosage of the anti-ANG2 antibody is administered before or after the subject is exposed to an inflammatory agent such as a bacterium, a virus and a bacterial toxin, more preferably before the subject manifests pulmonary infiltration of inflammatory cells.

| | 86E9 | 107H7 | 81H10 | Nesvacumab | IgG |
|---|---|---|---|---|---|
| EC50 | 0.4344 | 0.2308 | 0.2480 | 0.2694 | 16.76 |

Figure 1A Detection of Binding Activity of the Antibody to hAng2 Protein by ELISA

| | hz81H10.4 | Nesvacumab | IgG | 81H10 |
|---|---|---|---|---|
| EC50 | 0.02122 | 0.02556 | ~ 0.008074 | 0.01419 |

Figure 1B Detection of Binding Activity of the Antibody to hAng2 Protein by ELISA

| | 86E9 | Nesvacumab | IgG |
|---|---|---|---|
| IC50 | 5.290 | 4.073 | ~ 6.172e-009 |

Figure 2A Detection of Blocking Effect of the Antibody on Ang2/Tie2 by ELISA

| | 107H7 | Nesvacumab | IgG |
|---|---|---|---|
| IC50 | 9.148 | 5.530 | |

Figure 2B Detection of Blocking Effect of the Antibody on Ang2/Tie2 by ELISA

| | 81H10 | Nesvacumab | IgG |
|---|---|---|---|
| IC50 | 1.323 | 3.194 | ~ 16.95 |

Figure 2C Detection of Blocking Effect of the Antibody on Ang2/Tie2 by ELISA

| | IgG | Nesvacumab | Faricimab | hz81H10.4 |
|---|---|---|---|---|
| IC50 | ~ 24.82 | 0.9915 | 27.79 | 0.7277 |

Figure 2D Detection of Blocking Effect of the Antibody on Ang2/Tie2 by ELISA

|  | Amhz81H10.4.30 | Nesvacumab |
|---|---|---|
| IC50 | 4.906 | 4.773 |

Figure 2E. Detection of Blocking Effect of the Antibody on Ang2/Tie2 by ELISA

|  | Amhz81H10.4.49 | IgG | Nesvacumab |
|---|---|---|---|
| IC50 | 0.5065 | ~ 4.344e-020 | 0.2812 |

Figure 2F. Detection of Blocking Effect of the Antibody on Ang2/Tie2 by ELISA

| | IgG | hz81H10.4 | Nesvacumab |
|---|---|---|---|
| IC50 | ~ 0.000 | 2.298 | 3.888 |

Figure 3. Detection of Blocking Effect of the Antibody on Ang2/Tie2 Protein by Cell-based Assay

**Tie 2 phosphorylation assay**

Figure 4 Detection of Inhibitory Effect of the Antibody on Ang2-Fc Induced Tie2 Phosphorylation by Cell-based Assay

## U87 cell adhesion

Figure 5A: U87 Cell Adhesion Assay

## U87 cell adhesion

Figure 5B: U87 Cell Adhesion Assay

Figure 6: Detection of Non-specific Cell Binding Ability of Anti-ang2 Antibody by Cytology Staining Method

Figure 7A: Ang2 Sensitizes TNF α-Induced CD54 and CD106 Expression on HUVEC-Tie2 Cells

Figure 7B: hz81H10.4 Inhibits CD54 and CD106 Expression on HUVEC-Tie2 Cells

**HUVEC/TERT-TIE2 permeability assay**
p=0.0012

Legend:
- Blank
- VEGF 5ng/ml+Ang1 200ng/ml
- IgG 1ug/ml
- hz81H10.4 1ug/ml
- Nesvacumab 1ug/ml

Figure 8: hz81H10.4 Inhibits Leakage in Vascular-like Structures in HUVEC-Tie2 Cells

A. Leukocyte count in bronchoalveolar lavage fluid

B. Neutrophil count in bronchoalveolar lavage fluid

C. Lymphocyte count in bronchoalveolar lavage fluid

D. Monocyte count in bronchoalveolar lavage fluid

Figure: 9

**LPS induced ARDS Lung weight**

Figure 10: Statistical Analysis Results of Lung Weight Index

**LPS induced ARDS surviral rate**

Figure 11: Improvement of Survival in ARDS Model by hz81H10.4

Figure 12: Improvement of Lung Pathology in ARDS Model by hz81H10.4.

(A) White blood cell count in bronchoalveolar lavage fluid

(B) Neutrophil count in bronchoalveolar lavage fluid

(C) Lymphocyte count in bronchoalveolar lavage fluid

(D) Monocyte count in bronchoalveolar lavage fluid

Figure 13

Figure 14 Improvement of Oxygen Saturation in ARDS Animal Model by hz81H10.4 at Different Concentrations

Control

Dexamethasone
2mg/kg

hz81H10.4
5mg/kg

hz81H10.4
15mg/kg

hz81H10.4
25mg/kg

Figure 15: Improvement of Lung Pathology in ARDS Model by hz81H10.4 at Different Concentrations

Figure 16: Effect of hz81H10.4 at Different Concentrations on Severity of Inflammatory Lesions in Lung Tissues in ARDS Model of Rabbits

Figure 17: Improvement of Lung CT Imaging Findings by hz81H10.4 at Different Concentrations in ARDS Model

Figure 18: Angiographic Imaging of Laser-induced Model of Choroidal Neovascularization

Figure 19 Statistics of Leakage in Laser-induced Choroidal Neovascular Structure

Figure 20: Scoring of Leakage Extent at Laser Damaged Site

**Control**

**IBI304**

**IBI304+hz81H10.4**

H&E Staining of Laser-injuried Area

Figure 21: H&E Staining in Retina and Choroid

**Control**

**IBI304**

**IBI304+hz81H10.4**

Figure 22: CD31 Staining in Retina and Choroid Sections

**CD31 immunohistochemical staining**

Figure 23: Statistics of CD31 Staining in Retina and Choroid Sections

Figure 24: CD31 and Desmin Staining in Retina and Choroid Sections

Figure 25: Exemplary Antibody CDR Sequences of this Invention

| Antibody name | HCDR1 | SEQ ID NO: | HCDR2 | SEQ ID NO: | HCDR3 | SEQ ID NO: | LCDR1 | SEQ ID NO: | LCDR2 | SEQ ID NO: | LCDR3 | SEQ ID NO: |
|---|---|---|---|---|---|---|---|---|---|---|---|---|
| Kabat numbering position | H26-35 | | H50-65 | | H95-102 | | L24-34 | | L50-56 | | L89-97 | |
| 81H10 | GFTFSNYWMN | 1 | EIRLKSNNYA THYAESVKG | 2 | TAPFAY | 3 | KASQDVGT DVA | 4 | WASTR HT | 5 | QQYSSYPL T | 6 |
| 86E9 | GFTFSNYWMN | 1 | EIRLKSNNYA THYAESVKG | 2 | TAPFAY | 3 | KASQDVGT DVA | 4 | WASTR HT | 5 | QQYSSYPL T | 6 |
| 107H7 | GFTFSNYWMN | 1 | EIRLKSNNYA THYAESVKG | 2 | TAPFAY | 3 | KASQDVGT DVA | 4 | WASTR HT | 5 | QQYSSYPL T | 6 |
| Hz81H10.4 | GFTFSNYWMN | 1 | EIRLKSNNYA THYAESVKG | 2 | TAPFAY | 3 | KASQDVGT DVA | 4 | WASTR HT | 5 | QQYSSYPL T | 6 |
| Amhz81 H10.4.30 | GFTFRAAWMN | 7 | EIRLKSNHYE THYAESVKG | 8 | TQPGAY | 9 | KASQYVFT DVA | 10 | WASTR WT | 11 | QQTSSYPLT | 12 |
| Amhz81 H10.4.49 | GFTFQNYWAN | 13 | EIRLKSNNYA THYAESVKG | 14 | TTPAAY | 15 | KASQDVWT DVA | 16 | WGSTR WT | 17 | QQTNIYPLT | 18 |
| Consensus sequence | GFTFX$_1$X$_2$X$_3$WX$_4$N Where, X$_1$ can be any amino acid, preferably S, R or Q; X$_2$ can be any amino acid, preferably N or A; X$_3$ can be any amino acid, preferably Y or A; X$_4$ can be any amino acid, preferably M or A | 54 | EIRLKSNX$_1$Y X$_2$THYAESVK G Where, X$_1$ can be any amino acid, preferably N or H; X$_2$ can be any amino acid, preferably A or E; | 55 | TX$_1$PX$_2$A Y Where, X$_1$ can be any amino acid, preferably A, Q or T; X$_2$ can be any amino acid, preferably F, G or A; | 56 | KASQX$_1$VX$_2$ TDVA Where, X$_1$ can be any amino acid, preferably D or Y; X2 can be any amino acid, preferably G, F or W; | 57 | WX$_1$ST RX$_2$T Where, X$_1$ can be any amino acid, preferably A or G; X$_2$ can be any amino acid, preferably H or W; | 58 | QQX$_1$X$_2$X$_3$Y PLT Where, X$_1$ can be any amino acid, preferably Y or T; X$_2$ can be any amino acid, preferably S or N; X$_3$ can be any amino acid, preferably S or I | 59 |

EP 4 183 802 A1

Figure 26: Exemplary Antibody Variable Region VH and VL Sequences of This Invention

| Antibody name | VH | VL |
|---|---|---|
| 81H10 | EVKLEESGGGLVQPGGSMKLSCVASGFTFSNYWMNWVRQSPEKG LEWVAEIRLKSNNYATHYAESVKGRFTISRDVSKSSVYLQMNNLR TEDTGIYYCTDTAPFAYWGQGTLVTVSA (SEQ ID NO: 19) | DIVMTQSHKFMSTSVGDRVSITCKASQDVGTDVAWYQQKP GQSPKLLIYWASTRHTGVPDRFTGSGSGTDFTLTISNVQSED LSDYFCQQYSSYPLTFGGGTKLEIK (SEQ ID NO: 20) |
| 86E9 | EVMLVESGGGLVQPGGSMKLSCVASGFTFSNYWMNWVRQSPEKG LEWVAEIRLKSNNYATHYAESVKGRFTISRDVSKSGVYLQMNNLR TEDTGIYYCTDTAPFAYWGQGTLVTVSA (SEQ ID NO: 21) | DIVMTQSHKFLSTSVGARVSITCKASQDVGTDVAWYQQKP GQSPKLLIYWASTRHTGVPDRFTGSGSGTDFTLTISNVQSED LSDYFCQQYSSYPLTFGGGTKLEIK (SEQ ID NO:22 ) |
| 107H7 | EVMLVESGGGLVQPGGSMKLSCVASGFTFSNYWMNWVRQSPEKG LEWVAEIRLKSNNYATHYAESVKGRFTISRDVSKSGVYLQMNNLR TEDTGIYYCTDTAPFAYWGQGTLVTVSA (SEQ ID NO: 23) | DIVMTQTPKFLSTSVGARVSITCKASQDVGTDVAWYQQKP GQSPKLLIYWASTRHTGVPDRFTGSGSGTDFTLTISNVQSED LSDYFCQQYSSYPLTFGGGTKLEIK (SEQ ID NO: 24) |
| Hz81H10.4 | EVQLVESGGGLVKPGGSLRLSCAASGFTFSNYWMNWVRQAPGKG LEWVAEIRLKSNNYATHYAESVKGRFTISRDVSKNTVYLQMNSLK TEDTAVYYCTDTAPFAYWGQGTLVTVSS    (SEQ ID NO: 25) | DIQMTQSPSSLSASVGDRVTITCKASQDVGTDVAWYQQKPG KAPKLLIYWASTRHTGVPSRFSGSGSGTDFTLTISSLQPEDFA TYYCQQYSSYPLTFGGGTKVEIK (SEQ ID NO: 26) |
| Amhz81H10.4.30 | EVQLVESGGGLVKPGGSLRLSCAASGFTFRAAWMNWVRQAPGKG LEWVAEIRLKSNHYETHYAESVKGRFTISRDVSKNTVYLQMNSLK TEDTAVYYCTDTQPGAYWGQGTLVTVSS    (SEQ ID NO: 27) | DIQMTQSPSSLSASVGDRVTITCKASQYVFTDVAWYQQKPG KAPKLLIYWASTRWTGVPSRFSGSGSGTDFTLTISSLQPEDF ATYYCQQTSSYPLTFGGGTKVEIK (SEQ ID NO: 28) |
| Amhz81H10.4.49 | EVQLVESGGGLVKPGGSLRLSCAASGFTFQNYWANWVRQAPGKG LEWVAEIRLKSNNYATHYAESVKGRFTISRDVSKNTVYLQMNSLKT EDTAVYYCTDTTPAAYWGQGTLVTVSS (SEQ ID NO: 29) | DIQMTQSPSSLSASVGDRVTITCKASQDVWTDVAWYQQKPG KAPKLLIYWGSTRWTGVPSRFSGSGSGTDFTLTISSLQPEDFA TYYCQQTNIYPLTFGGGTKVEIK (SEQ ID NO: 30) |

Figure 27:

Human Ang2 Amino Acid Sequence (SEQ ID NO: 51)

YNNFRKSMDSIGKKQYQVQHGSCSYTFLLPEMDNCRSSSSPYVSNAVQRDAPLEYDDSVQRLQVLENIMENNTQWLMKLENYIQDNMK
KEMVEIQQNAVQNQTAVMIEIGTNLLNQTAEQTRKLTDVEAQVLNQTTRLELQLLEHSLSTNKLEKQILDQTSEINKLQDKNSFLEKKVLA
MEDKHIIQLQSIKEEKDQLQVLVSKQNSIIEELEKKIVTATVNNSVLQKQQHDLMETVNNLLTMMSTSNSAKDPTVAKEEQISFRDCAEVFK
SGHTTNGIYTLTFPNSTEEIKAYCDMEAGGGGWTIIQRREDGSVDFQRTWKEYKVGFGNPSGEYWLGNEFVSQLTNQQRYVLKIHLKDWE
GNEAYSLYEHFYLSSEELNYRIHLKGLTGTAGKISSISQPGNDFSTKDGDNDKCICKCSQMLTGGWWFDACGPSNLNGMYYPQRQNTNKF
NGIKWYYWKGSGYSLKATTMMIRPADF

Amino Acid Sequence of IgG1 Heavy Chain Constant Region: (SEQ ID NO: 52)

ASTKGPSVFPLAPSSKSTSGGTAALGCLVKDYFPEPVTVSWNSGALTSGVHTFPAVLQSSGLYSLSSVVTVPSSSLGTQTYICNVNHKPSNT
KVDKKAEPKSCDKTHTCPPCPAPELLGGPSVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPGK

Amino Acid Sequence of Light Chain Constant Region (kppa light chain) (SEQ ID NO: 53)

RTVAAPSVFIFPPSDEQLKSGTASVVCLLNNFYPREAKVQWKVDNALQSGNSQESVTEQDSKDSTYSLSSTLTLSKADYEKHKVYACEVT
HQGLSSPVTKSFNRGEC

Amino Acid Sequence of Anti-VEGF Molecules for Combinational Use (SEQ ID NO: 60)

PFVEMYSEIPEIIHMTEGRELVIPCRVTSPNITVTLKKFPLDTLIPDGKRIIWDSRKGFIISNATYKEIGLLTCEATVNGHLYKTNYLTHRQTVVL
SPSHGIELSVGEKLVLNCTARTELNVGIDFNWEYPSSKHQHKKLVNRDLKTQSGSEMKKFLSTLTIDGVTRSDQGLYTCAASSGLMTKKNS
TFVRVHEKGGGGGGGGGGGGDKTHTCPLCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVSLTCLVKGFYPSDIAVE
WESNGQPENNYKATPPVLDSDGSFFLYSKLTVDKSRWQQGNVFSCSVMHEALHNHYTQKSLSLSPGK

EP 4 183 802 A1

# EP 4 183 802 A1

| | | |
|---|---|---|
| **INTERNATIONAL SEARCH REPORT** | International application No. | |
| | **PCT/CN2021/101243** | |

**A. CLASSIFICATION OF SUBJECT MATTER**

C07K 16/22(2006.01)i; C07K 16/28(2006.01)i; C12N 15/13(2006.01)i; C12N 15/85(2006.01)i

According to International Patent Classification (IPC) or to both national classification and IPC

**B. FIELDS SEARCHED**

Minimum documentation searched (classification system followed by classification symbols)

C07K; C12N

Documentation searched other than minimum documentation to the extent that such documents are included in the fields searched

Electronic data base consulted during the international search (name of data base and, where practicable, search terms used)

CNMED; TWABS; MOABS; TWMED; HKABS; CNABS; CNTXT; WOTXT; EPTXT; CNKI; 万方; 百度学术; CNKI: 抗体, 血管生成素2, ANG-2, Angiopoietin-2, ANGPT2, ANG2, antibody, 人源化, 血管, 渗漏, 肺, 炎症, 通透性, SEQ ID NO.1-30, 54-59

**C. DOCUMENTS CONSIDERED TO BE RELEVANT**

| Category* | Citation of document, with indication, where appropriate, of the relevant passages | Relevant to claim No. |
|---|---|---|
| A | CN 102257008 A (F. HOFFMANN-LA ROCHE AG) 23 November 2011 (2011-11-23) entire document | 1-19 |
| A | CN 101128483 A (ASTRAZENECA AB) 20 February 2008 (2008-02-20) entire document | 1-19 |
| A | CN 109863171 A (MEDIMMUNE LIMITED) 07 June 2019 (2019-06-07) entire document | 1-19 |
| A | US 2018273613 A1 (SAMSUNG ELECTRONICS CO., LTD.) 27 September 2018 (2018-09-27) entire document | 1-19 |
| A | CN 105085678 A (ASTRAZENECA AB) 25 November 2015 (2015-11-25) entire document | 1-19 |
| A | CN 107001457 A (REGENERON PHARMACEUTICALS, INC.) 01 August 2017 (2017-08-01) entire document | 1-19 |
| A | US 2017029493 A1 (HOFFMANN-LA ROCHE INC.) 02 February 2017 (2017-02-02) entire document | 1-19 |

☑ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 September 2021** | **18 September 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/101243** |

**C.    DOCUMENTS CONSIDERED TO BE RELEVANT**

| Category* | Citation of document, with indication, where appropriate, of the relevant passages | Relevant to claim No. |
|---|---|---|
| A | HOLOPANIEN, T. et al. "Effects of Angiopoietin-2-Blocking Antibody on Endothelial Cell–Cell Junctions and Lung Metastasis"<br>*JNCI*, Vol. 104, 17 February 2012 (2012-02-17),<br>    pp. 461-475 | 1-19 |
| A | RENNEL, E.S. et al. "A Human Neutralizing Antibody Specific to Ang-2 Inhibits Ocular Angiogenesis"<br>*MICROCIRCULATION*, Vol. 18, No. 7, 31 October 2011 (2011-10-31),<br>    pp. 598-607 | 1-19 |

Form PCT/ISA/210 (second sheet) (January 2015)

**INTERNATIONAL SEARCH REPORT**

International application No.

**PCT/CN2021/101243**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2021/101243** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **9-19**
because they relate to subject matter not required to be searched by this Authority, namely:

[1] Claims 9-19 relate to a method for treating a disease related to ANG1 bioactivity, and to a method of treating diseases pursuant to PCT Rule 39.1(iv). Therefore, a search has been carried out on the basis of a corresponding use of the ANG2 antibody in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/101243**

| Patent document cited in search report | | | Publication date (day/month/year) | Patent family member(s) | | | Publication date (day/month/year) |
|---|---|---|---|---|---|---|---|
| CN | 102257008 | A | 23 November 2011 | KR | 20130103822 | A | 24 September 2013 |
| | | | | US | 2013156789 | A1 | 20 June 2013 |
| | | | | US | 2015284457 | A1 | 08 October 2015 |
| | | | | AR | 092487 | A2 | 22 April 2015 |
| | | | | ZA | 201307297 | B | 31 July 2019 |
| | | | | US | 2020262904 | A1 | 20 August 2020 |
| | | | | JP | 5559191 | B2 | 23 July 2014 |
| | | | | MY | 155654 | A | 13 November 2015 |
| | | | | DK | 2379592 | T3 | 02 March 2015 |
| | | | | HR | P20150439 | T1 | 22 May 2015 |
| | | | | EP | 2379592 | B1 | 11 February 2015 |
| | | | | WO | 2010069532 | A8 | 10 May 2012 |
| | | | | SG | 172216 | A1 | 28 July 2011 |
| | | | | AU | 2009328613 | B2 | 05 September 2013 |
| | | | | SI | 2379592 | T1 | 30 June 2015 |
| | | | | HU | E024545 | T2 | 28 January 2016 |
| | | | | IL | 226350 | A | 31 October 2016 |
| | | | | US | 8361747 | B2 | 29 January 2013 |
| | | | | US | 2014065707 | A1 | 06 March 2014 |
| | | | | TW | 201322996 | A | 16 June 2013 |
| | | | | US | 9109027 | B2 | 18 August 2015 |
| | | | | RU | 2011129204 | A | 27 January 2013 |
| | | | | US | 2012142091 | A1 | 07 June 2012 |
| | | | | PT | 2379592 | E | 24 March 2015 |
| | | | | RU | 2569461 | C2 | 27 November 2015 |
| | | | | UA | 103912 | C2 | 10 December 2013 |
| | | | | CR | 20130418 | A | 04 October 2013 |
| | | | | US | 9340609 | B2 | 17 May 2016 |
| | | | | US | 2014065151 | A1 | 06 March 2014 |
| | | | | US | 2018282404 | A1 | 04 October 2018 |
| | | | | MY | 158438 | A | 14 October 2016 |
| | | | | PH | 12013502192 | A1 | 12 October 2015 |
| | | | | CL | 2012003054 | A1 | 14 December 2012 |
| | | | | JP | 5814317 | B2 | 17 November 2015 |
| | | | | PE | 20140814 | A1 | 10 July 2014 |
| | | | | KR | 101381012 | B1 | 11 April 2014 |
| | | | | KR | 101445518 | B1 | 01 October 2014 |
| | | | | CN | 103739709 | A | 23 April 2014 |
| | | | | IL | 226350 | D0 | 27 June 2013 |
| | | | | IL | 213039 | D0 | 31 July 2011 |
| | | | | CA | 2744624 | A1 | 24 June 2010 |
| | | | | RU | 2569107 | C2 | 20 November 2015 |
| | | | | UA | 105151 | C2 | 10 April 2014 |
| | | | | US | 2010159587 | A1 | 24 June 2010 |
| | | | | CR | 20110321 | A | 14 July 2011 |
| | | | | CN | 103739709 | B | 24 August 2016 |
| | | | | WO | 2010069532 | A1 | 24 June 2010 |
| | | | | AU | 2009328613 | A1 | 24 June 2010 |
| | | | | PL | 2379592 | T3 | 31 July 2015 |
| | | | | KR | 20110084536 | A | 25 July 2011 |

Form PCT/ISA/210 (patent family annex) (January 2015)

**INTERNATIONAL SEARCH REPORT**
Information on patent family members

International application No.

**PCT/CN2021/101243**

| Patent document cited in search report | | | Publication date (day/month/year) | Patent family member(s) | | | Publication date (day/month/year) |
|---|---|---|---|---|---|---|---|
| CN | 101128483 | A | 20 February 2008 | HK | 1217713 | A1 | 20 January 2017 |
| | | | | US | 7973140 | B2 | 05 July 2011 |
| | | | | JP | 2008523841 | A | 10 July 2008 |
| | | | | EP | 2284194 | A1 | 16 February 2011 |
| | | | | AU | 2005319382 | B2 | 07 April 2011 |
| | | | | EP | 3699191 | A1 | 26 August 2020 |
| | | | | CN | 101128483 | B | 03 June 2015 |
| | | | | US | 8834880 | B2 | 16 September 2014 |
| | | | | AR | 052065 | A1 | 28 February 2007 |
| | | | | ES | 2371083 | T3 | 27 December 2011 |
| | | | | KR | 101017301 | B1 | 28 February 2011 |
| | | | | US | 2015125455 | A1 | 07 May 2015 |
| | | | | US | 2008267971 | A1 | 30 October 2008 |
| | | | | MX | 2007007484 | A | 20 July 2007 |
| | | | | HK | 1109409 | A1 | 06 June 2008 |
| | | | | RU | 2007127842 | A | 27 January 2009 |
| | | | | JP | 5642042 | B2 | 17 December 2014 |
| | | | | PT | 1838733 | E | 13 December 2011 |
| | | | | SI | 1838733 | T1 | 30 December 2011 |
| | | | | US | 2013171160 | A1 | 04 July 2013 |
| | | | | NO | 20073192 | L | 06 September 2007 |
| | | | | DK | 1838733 | T3 | 28 November 2011 |
| | | | | BR | PI0519596 | A2 | 24 March 2009 |
| | | | | HR | P20110859 | T1 | 31 December 2011 |
| | | | | EP | 1838733 | B1 | 24 August 2011 |
| | | | | NZ | 556029 | A | 30 April 2010 |
| | | | | CY | 1112909 | T1 | 13 April 2016 |
| | | | | US | 2006246071 | A1 | 02 November 2006 |
| | | | | US | 2012052073 | A1 | 01 March 2012 |
| | | | | ZA | 200705695 | A | 25 February 2009 |
| | | | | US | 10066011 | B2 | 04 September 2018 |
| | | | | ZA | 200705695 | B | 25 February 2009 |
| | | | | WO | 2006068953 | A3 | 02 August 2007 |
| | | | | AT | 521638 | T | 15 September 2011 |
| | | | | WO | 2006068953 | A2 | 29 June 2006 |
| | | | | RU | 2394839 | C2 | 20 July 2010 |
| | | | | PL | 1838733 | T3 | 29 February 2012 |
| | | | | AU | 2005319382 | A1 | 29 June 2006 |
| | | | | JP | 2012050455 | A | 15 March 2012 |
| | | | | CN | 105085678 | B | 07 May 2019 |
| | | | | CA | 2595610 | C | 05 March 2013 |
| | | | | RS | 52036 | B | 30 April 2012 |
| | | | | UY | 29288 | A1 | 31 July 2006 |
| | | | | SI | EP1838733 | T1 | 30 December 2011 |
| | | | | CN | 105085678 | A | 25 November 2015 |
| | | | | CA | 2595610 | A1 | 29 June 2006 |
| | | | | TW | 200634027 | A | 01 October 2006 |
| | | | | EP | 1838733 | A2 | 03 October 2007 |
| | | | | JP | 4884395 | B2 | 29 February 2012 |
| CN | 109863171 | A | 07 June 2019 | IL | 264962 | D0 | 30 May 2019 |

Form PCT/ISA/210 (patent family annex) (January 2015)

**INTERNATIONAL SEARCH REPORT**
Information on patent family members

International application No.

**PCT/CN2021/101243**

| Patent document cited in search report | | | Publication date (day/month/year) | Patent family member(s) | | | Publication date (day/month/year) |
|---|---|---|---|---|---|---|---|
| | | | | WO | 2018037000 | A1 | 01 March 2018 |
| | | | | KR | 20190039577 | A | 12 April 2019 |
| | | | | US | 2019194308 | A1 | 27 June 2019 |
| | | | | CA | 3034574 | A1 | 01 March 2018 |
| | | | | EP | 3504237 | A1 | 03 July 2019 |
| | | | | AU | 2017315075 | A1 | 04 April 2019 |
| | | | | US | 10836819 | B2 | 17 November 2020 |
| | | | | US | 2021040194 | A1 | 11 February 2021 |
| | | | | AU | 2017315075 | B2 | 01 October 2020 |
| | | | | JP | 2019528694 | A | 17 October 2019 |
| US | 2018273613 | A1 | 27 September 2018 | US | 10934350 | B2 | 02 March 2021 |
| | | | | US | 9994632 | B2 | 12 June 2018 |
| | | | | KR | 20150136031 | A | 04 December 2015 |
| | | | | US | 2015337033 | A1 | 26 November 2015 |
| CN | 105085678 | A | 25 November 2015 | CA | 2595610 | A1 | 29 June 2006 |
| | | | | TW | 200634027 | A | 01 October 2006 |
| | | | | EP | 1838733 | A2 | 03 October 2007 |
| | | | | JP | 4884395 | B2 | 29 February 2012 |
| | | | | KR | 20070116217 | A | 07 December 2007 |
| | | | | HK | 1217713 | A1 | 20 January 2017 |
| | | | | US | 7973140 | B2 | 05 July 2011 |
| | | | | JP | 2008523841 | A | 10 July 2008 |
| | | | | CN | 101128483 | A | 20 February 2008 |
| | | | | EP | 2284194 | A1 | 16 February 2011 |
| | | | | AU | 2005319382 | B2 | 07 April 2011 |
| | | | | EP | 3699191 | A1 | 26 August 2020 |
| | | | | CN | 101128483 | B | 03 June 2015 |
| | | | | US | 8834880 | B2 | 16 September 2014 |
| | | | | AR | 052065 | A1 | 28 February 2007 |
| | | | | ES | 2371083 | T3 | 27 December 2011 |
| | | | | KR | 101017301 | B1 | 28 February 2011 |
| | | | | US | 2015125455 | A1 | 07 May 2015 |
| | | | | US | 2008267971 | A1 | 30 October 2008 |
| | | | | MX | 2007007484 | A | 20 July 2007 |
| | | | | HK | 1109409 | A1 | 06 June 2008 |
| | | | | RU | 2007127842 | A | 27 January 2009 |
| | | | | JP | 5642042 | B2 | 17 December 2014 |
| | | | | PT | 1838733 | E | 13 December 2011 |
| | | | | SI | 1838733 | T1 | 30 December 2011 |
| | | | | US | 2013171160 | A1 | 04 July 2013 |
| | | | | NO | 20073192 | L | 06 September 2007 |
| | | | | DK | 1838733 | T3 | 28 November 2011 |
| | | | | BR | PI0519596 | A2 | 24 March 2009 |
| | | | | HR | P20110859 | T1 | 31 December 2011 |
| | | | | EP | 1838733 | B1 | 24 August 2011 |
| | | | | NZ | 556029 | A | 30 April 2010 |
| | | | | CY | 1112909 | T1 | 13 April 2016 |
| | | | | US | 2006246071 | A1 | 02 November 2006 |
| | | | | US | 2012052073 | A1 | 01 March 2012 |
| | | | | ZA | 200705695 | A | 25 February 2009 |

Form PCT/ISA/210 (patent family annex) (January 2015)

<table>
<tr><td colspan="2" align="center">INTERNATIONAL SEARCH REPORT<br>Information on patent family members</td><td colspan="2">International application No.<br><br>**PCT/CN2021/101243**</td></tr>
</table>

| Patent document cited in search report | | | | Publication date (day/month/year) | Patent family member(s) | | | Publication date (day/month/year) |
|---|---|---|---|---|---|---|---|---|
| | | | | | US | 10066011 | B2 | 04 September 2018 |
| | | | | | ZA | 200705695 | B | 25 February 2009 |
| | | | | | WO | 2006068953 | A3 | 02 August 2007 |
| | | | | | AT | 521638 | T | 15 September 2011 |
| | | | | | WO | 2006068953 | A2 | 29 June 2006 |
| | | | | | RU | 2394839 | C2 | 20 July 2010 |
| | | | | | PL | 1838733 | T3 | 29 February 2012 |
| | | | | | AU | 2005319382 | A1 | 29 June 2006 |
| | | | | | JP | 2012050455 | A | 15 March 2012 |
| | | | | | CN | 105085678 | B | 07 May 2019 |
| | | | | | CA | 2595610 | C | 05 March 2013 |
| | | | | | RS | 52036 | B | 30 April 2012 |
| | | | | | UY | 29288 | A1 | 31 July 2006 |
| CN | 107001457 | A | 01 August 2017 | | MA | 40306 | A1 | 29 March 2019 |
| | | | | | US | 2016144025 | A1 | 26 May 2016 |
| | | | | | EP | 3224278 | A1 | 04 October 2017 |
| | | | | | IL | 252159 | D0 | 31 July 2017 |
| | | | | | KR | 20170087950 | A | 31 July 2017 |
| | | | | | SG | CN 11201703609 U | A | 29 June 2017 |
| | | | | | MX | 2017006129 | A | 10 April 2018 |
| | | | | | US | 2017080086 | A1 | 23 March 2017 |
| | | | | | PH | 12017500834 | A1 | 30 October 2017 |
| | | | | | AU | 2015353838 | A1 | 08 June 2017 |
| | | | | | PE | 20170900 | A1 | 12 July 2017 |
| | | | | | US | 2019117767 | A1 | 25 April 2019 |
| | | | | | IL | 252159 | A | 25 March 2021 |
| | | | | | JP | 2017536414 | A | 07 December 2017 |
| | | | | | WO | 2016085750 | A1 | 02 June 2016 |
| | | | | | EA | 201791168 | A1 | 29 September 2017 |
| | | | | | BR | 112017009807 | A2 | 27 February 2018 |
| | | | | | EP | 3224278 | A4 | 11 July 2018 |
| | | | | | JP | 2021046431 | A | 25 March 2021 |
| | | | | | CA | 2968522 | A1 | 02 June 2016 |
| | | | | | CL | 2017001188 | A1 | 24 November 2017 |
| US | 2017029493 | A1 | 02 February 2017 | | KR | 20130103822 | A | 24 September 2013 |
| | | | | | US | 2013156789 | A1 | 20 June 2013 |
| | | | | | US | 2015284457 | A1 | 08 October 2015 |
| | | | | | AR | 092487 | A2 | 22 April 2015 |
| | | | | | ZA | 201307297 | B | 31 July 2019 |
| | | | | | US | 2020262904 | A1 | 20 August 2020 |
| | | | | | JP | 5559191 | B2 | 23 July 2014 |
| | | | | | MY | 155654 | A | 13 November 2015 |
| | | | | | DK | 2379592 | T3 | 02 March 2015 |
| | | | | | HR | P20150439 | T1 | 22 May 2015 |
| | | | | | EP | 2379592 | B1 | 11 February 2015 |
| | | | | | WO | 2010069532 | A8 | 10 May 2012 |
| | | | | | SG | 172216 | A1 | 28 July 2011 |
| | | | | | AU | 2009328613 | B2 | 05 September 2013 |
| | | | | | SI | 2379592 | T1 | 30 June 2015 |

Form PCT/ISA/210 (patent family annex) (January 2015)

**INTERNATIONAL SEARCH REPORT**
Information on patent family members

International application No.

**PCT/CN2021/101243**

| Patent document cited in search report | Publication date (day/month/year) | Patent family member(s) | | | Publication date (day/month/year) |
|---|---|---|---|---|---|
| | | HU | E024545 | T2 | 28 January 2016 |
| | | IL | 226350 | A | 31 October 2016 |
| | | US | 8361747 | B2 | 29 January 2013 |
| | | US | 2014065707 | A1 | 06 March 2014 |
| | | TW | 201322996 | A | 16 June 2013 |
| | | US | 9109027 | B2 | 18 August 2015 |
| | | RU | 2011129204 | A | 27 January 2013 |
| | | US | 2012142091 | A1 | 07 June 2012 |
| | | PT | 2379592 | E | 24 March 2015 |
| | | RU | 2569461 | C2 | 27 November 2015 |
| | | UA | 103912 | C2 | 10 December 2013 |
| | | CR | 20130418 | A | 04 October 2013 |
| | | US | 9340609 | B2 | 17 May 2016 |
| | | US | 2014065151 | A1 | 06 March 2014 |
| | | US | 2018282404 | A1 | 04 October 2018 |
| | | MY | 158438 | A | 14 October 2016 |
| | | PH | 12013502192 | A1 | 12 October 2015 |
| | | CL | 2012003054 | A1 | 14 December 2012 |
| | | JP | 5814317 | B2 | 17 November 2015 |
| | | PE | 20140814 | A1 | 10 July 2014 |
| | | KR | 101381012 | B1 | 11 April 2014 |
| | | KR | 101445518 | B1 | 01 October 2014 |
| | | CN | 103739709 | A | 23 April 2014 |
| | | IL | 226350 | D0 | 27 June 2013 |
| | | IL | 213039 | D0 | 31 July 2011 |
| | | CA | 2744624 | A1 | 24 June 2010 |
| | | RU | 2569107 | C2 | 20 November 2015 |
| | | UA | 105151 | C2 | 10 April 2014 |
| | | US | 2010159587 | A1 | 24 June 2010 |
| | | CR | 20110321 | A | 14 July 2011 |
| | | CN | 103739709 | B | 24 August 2016 |
| | | WO | 2010069532 | A1 | 24 June 2010 |
| | | AU | 2009328613 | A1 | 24 June 2010 |
| | | PL | 2379592 | T3 | 31 July 2015 |
| | | KR | 20110084536 | A | 25 July 2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7087411 B
- US 6194551 B
- WO 9951642 A
- WO 2016075035 A
- US 5677425 A
- EP 0154316 A
- EP 0401384 A
- CN 200510115530
- US 4737456 A
- SU 20200002
- SU 20200907214

### Non-patent literature cited in the description

- **PETERS S. et al.** Angiopoietin-2 multiplies the permeability enhancing effect of vascular endothelial growth factor on retinal endothelial cells. *Invest Ophthalmol Vis Sci.,* 2004, vol. 45, 1901-1906
- **LAURA HAKANPAA et al.** Endothelial destabilization by angiopoietin-2 via integrin β 1 activation. *Nature Communications,* 2015, vol. 6, 5962
- **REHAN M. HUSSAIN et al.** Tie-2/Angiopoietin pathway modulation as a therapeutic strategy for retinal disease. *Expert Opinion on Investigational Drugs,* 2019, https://doi.org/10.1080/13543784.2019.1667333
- Fundamental Immunology. Raven Press, 1989
- Fundamental Immunology. Raven Press, 1993
- Research and Application of Antibody-based Drugs. People's Medical Publishing House, 2013
- **HOLLINGER et al.** *PNAS USA,* 1993, vol. 90, 6444-6448
- **HUDSON et al.** *Nat. Med.,* 2003, vol. 9, 129-134
- **FLATMAN, S. et al.** *J. Chrom. B,* 2007, vol. 848, 79-87
- **HARLOW ; LANE.** Antibodies. Cold Spring Harbor Press
- **KABAT, E. A. et al.** Sequences of Proteins of Immunological Interest. Public Health Service, National Institutes of Health, 1991
- **KABAT et al.** Sequences of Proteins of Immunological Interest. National Institutes of Health, 1991
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **IDUSOGIE, E. E. et al.** *J. Immunol.,* 2000, vol. 164, 4178-4184
- Remington's Pharmaceutical Sciences. 1980
- **RACHEAL G. AKWII et al.** Role of Angiopoietin-2 in Vascular Physiology and Pathology. *Cells,* 2019, vol. 8, 471
- The ARDS Definition Task Force. *JAMA,* 2012, vol. 307 (23), 2526-2533
- Remington's Pharmaceutical Sciences. Mack Publishing Company
- **ESTEP, P et al.** High throughput solution-based measurement of antibody-antigen affinity and epitope binning. *MAbs,* 2013, vol. 5 (2), 270-8
- Study on the Chief Organ Weight and Coefficients of Different Species of Experimental Rabbits. *Chinese Journal of Comparative Medicine,* December 2004, vol. 14 (6
- *Standard Terminology for Diagnosis and Classification*